# EUROPEAN PATENT APPLICATION

(11) **EP 2 762 138 A1**
(43) Date of publication of application: **06.08.2014**
(21) Application number: 12834473.6
(22) Date of filing: 20.09.2012
(51) Int. Cl.: A61K 31/4015, C07D 201/00, C07D 201/16, C07D 201/18, A61P 25/00, A61P 9/00, A61P 39/06, A61P 3/04, A61P 7/10, A61P 37/02, A61P 43/00

(54) **PHARMACEUTICAL SUBSTANCE (VARIANTS) AND COMPOSITIONS BASED THEREON WHICH EXHIBIT MODULATORY ACTIVITY WITH A COMMENSURATE EFFECT**

(30) Priority: 22.09.2011 RU 2011138840
(71) Applicant: Akhapkina, Valentina Ivanovna, 105264 Moscow (RU); Akhapkin, Roman Vitalyevich, Moscow 119019 (RU)
(72) Inventor: Akhapkina, Valentina Ivanovna, 105264 Moscow (RU); Akhapkin, Roman Vitalyevich, Moscow 119019 (RU)
(74) Representative: Glawe, Delfs, Moll
(86) International application number: PCT/RU2012/000773
(87) International publication number: WO 2013/043085

(57) **Abstract**

The invention relates to various fields of medicine, pharmaceutics and pharmacology, chemico-pharmaceutical, pharmaceutical and para-pharmaceutical industries, and in particular to new class of agents having modulatory activity with commensurate effect. The invention consists in that provided product (RS)-2-(2-oxo-4-phenylpyrrolidin-1-yl)acetamide does not contain biologically inert substances in its composition having adverse effect on its newly discovered and considerably improved known properties and characteristics, at the same time discovering unknown before vitally important and essential ideas about the composition, widening the field of its application while increasing its efficiency and safety of use, increasing its therapeutic scope, provides for preparing the product and its products with revealed properties and characteristics.

## Description

### Prior art

Originally, the definition of **modulation** (Latin "modulatio" - dimension, dimensionality) as a **commensurate** (Latin - "commensuratur") effect was introduced by V.I.Akhapkina in 2006. Conceptualization of modulation eliminated the aberration in its understanding that existed for many years specifically in biology and medicine. In the report at the XIII Russian National Congress "Man and medications" (V.I. Akhapkina, R.V. Akhapkin, Moscow, Russia, April 06, 2006) introduction of two new classes of medicines were proposed and fundamentally rationalized: modulators (commensurate effect) and divergents (from Latin "divergerens" - divergence) with their own systematization and obligate criteria (features) for identification and evaluation of basic pharmacological activity and associated components of action, including exfoliative (from Latin. exfoliatio - exfoliation) ones.

**Modulatory activity** is a commensurate effect on processes of both stimulation and suppression, their commensurately consolidated coupling and commensurate reversibility. Stimulatory and suppressing action of a commensurate effect may manifest differently mainly depending on the type of organism status in normal condition and on the type of disorder. Commensurate effect, including trigger transmission and reversibility, is the main distinctive feature of modulators from other basic classes of drugs (stimulants, suppressants and divergents).

**Divergent activity** is a change from baseline activity with a pronounced divergence of stimulation and suppression depending on the dose. Signs of divergence in contrast to the divergence in mathematics and the definition of Darwin diverge not only from one point or one species.

Fundamentally modulation (commensurate effect) and divergence are leading in structural and functional organization of any living organism. They are interrelated and interdependent, have their own obligate criteria (features) for identification and evaluation of their biological activity and therefore have all grounds to be separately presented in the classification of medicines. In contrast to medicines, the division of substances synthesized in the body into modulators and divergents are quite relative. The same substances synthesized in the body may in some cases act as modulators (e.g., due to conformation, conjointly stereospecificity asymmetric of action potential ensuring the reversibility of processes, etc.), while in others cases - as divergents with the obvious effect of the paradigm shift due to concentration, which is especially pronounced when changing the rhythm from wake to sleep and vice versa.

Today there is no reason to deny biorhytmicity of neuromodulator systems. It is known that neurotransmitters (including GABA) exhibit both excitatory and suppressing effect depending on the site, time, conditions, age. Polymorphism of synapses (that predetermines the signal differentiation) and of neurons is known. Chaperones, co-factors and transcription factors are identified and described. It was proved that in some cases they provide reduction, while in others - increase in effect and in binding constants.

Contrary to scientific discoveries and advances, the current classification of drugs has not changed substantially and is still based on a diarchy (either only stimulation or only suppression), that is rooted in the "Dale's principle" [36]. It is fundamentally, morally and physically obsolete. Its scope may not fit not only modulators with commensurate effect, but also divergents. They cannot be included into its "atypical" or "other" category as their effect is typical and organic as predetermined by the nature of evolution.

Classes of modulators (commensurate effect) and divergents allow to assess more objectively the efficacy of drugs, their mechanisms of action and place in classification, to identify new approaches to treatment, correction and prevention of various disorders and diseases with the most rational dosing and prediction of effects of various drugs. Nevertheless, the exfoliative, other related and mediated components of action of divergents and modulators, just like those of stimulants and suppressants as well, and are not critical to their classification.

In this context, "secondary" refers to importance of the features, yet because they are, to a greater or lesser degree, either dependent or generic, are usually considered in groups and subgroups at systematizing classes, although often have in medical practice a meaningful and independent use. These characteristics include primarily nootropic and adaptogenic effects, effect on metabolism, that is present in varying degrees in almost any medication. Disorder of the adaptive regulation, cognitive processes, metabolism and their plasticity occur both during any type of disorder and pathological condition (especially in the acute stage, complications, chronic stage) and in post-fertile period. Disorder of cognitive processes, adaptive regulation, plastic organization occur not only during their suppression, and the body exhaustion phase can not be included into the stress triad (positive stress, distress, destress), since it is not stress anymore, but rather a consequence desorganisational phase of the negative stress (destress).

Divergents are presented in the nomenclature of drugs quite widely, yet they are forcefully assigned to either stimulants or to suppressants, which in fact they are not. The vast number of drugs from the "nootropics" group (T.A. Voronina, 1989; T.A. Voronina, S.B. Seredenin, 1998, 2007), except for stimulants (piracetam, etc.), some antipsychotics, anxiolytics, antidepressants, antiepileptic drugs and other neurotropic and psychotropic products are divergents with their inherent secondary features.

Attempts to link the activity of divergents and many stimulants with the nootropic concept did not succeed within forty years of research for objective reasons. Yet there is no reason to assert the fact that the stimulation of neurometabolism is exclusively the prerogative of the nootropic activity. Essentially, the nootropic activity is not primary in the known drugs, but rather a component of action and very dose-dependent, exfoliating in most drugs along with the primary activity or against the background of primary activity, which disappears when the dose is increased [23; 24]. For example, Phenibut, Pantogram and others are classic divergents (stimulants in low doses and tranquilizers or even hypnotics in higher doses). Their secondary features (nootropic, adaptogenic etc. effects) exfoliate. Based on known effects and mechanisms of action of Phenotropil - N-carbamoylmethyl-4-phenyl-2-pyrrolidone [31; 2-7; 9-21; 25; 26; 32-34], identified effects of optically pure isomers of N-carbamoylmethyl-4(R)-phenyl-2-pyrrolidinone and N-carbamoylmethyl-4(S)-phenyl-2-pyrrolidinone (WO 2007/104780 A2, publication date 20.07.2007), one can clearly say that chemically pure product (racemate) and products (optically pure isomeric substances) are divergents with associated inherent components of action, including exfoliative features. The identified features on the effect of Phenotropil on the stroke symptom complex of various etiologies suggest the synergy of mechanisms of action peculiar exactly to modulating effect [26, 32, 33].

Piracetam, having the stimulating activity, displays the antihypoxic action component markedly exfoliating (stratified) with the nootropic and adaptogenic ones. The stimulating activity of piracetam increases with increasing dose and the nootropic effect disappears herewith, but the antihypoxic one emerges. Increasing the dose of piracetam is obviously contradicting with the nootropic concept, causing impaired concentration, confusion and hallucinations, which is typical of psychostimulants. Known neuro-metabolic stimulants, neuropeptides, do not have an anti-hypoxic activity but have a pronounced antiamnestic effect. Benzodiazepine tranquilizers, having a marked anti-hypoxic effect, themselves cause amnesia, while in low threshold doses are capable of exhibiting a stimulating activity.

Secondary features mitigate to a certain extent the extensive and repressive activity in the new generation drugs, especially if they are multi-active and palliative that depends on the mechanisms of action of a certain drug in different doses. Given that the secondary characteristics depend on the basic activity and mechanisms of action of the drug, they can also be characterized differently and this, in turn, determines their place in the systematization of anatomical-therapeutic-chemical (ATC) classification of drugs.

Modulators with commensurate effect are not presented in the nomenclature of medicines. The aberrated understanding of "modulation" specifically in biology and medicine [38] as confounded with the modulating effect is placed in quotes by M.D. Mashkovsky [27]: "immunomodulators" are drugs that modulate immune processes (subgroup A - immunostimulants (levamisole, thymalin etc), subgroup B - immunosuppressants (azathioprine, batridenum etc). Subsequently, immunosuppressants were excluded from the "immunomodulators". Until now, the definition "modulating effect" was only applied to complex stimulants or stimulants with a complex stimulating mechanism of action [31]. The modulating (stimulating) synergism and mediated potentiation are displayed by cerebrolysin, semax, nooglutyl, piracetam, Phenotropil etc. Similarly, "neuromodulators" with a modulating effect synthesized in the body are defined [29]. Anything is characterized by the modulating effect, including a discrete transmission (when one is turned on, and the other at the same time is turned off) [37] and the damaging effect of risk factors (N.V. Ivanov, 2009). Definition of the modulating effect (synonym of "modulation") is inconsistent with the essence of modulation as such, in principle; its obligate criteria (features) do not meet the requirements of the true modulation and modulatory activity.

Disadvantages of the direct and/or indirect either only stimulatory for the most part action under the principle of synergism or potentiation of a complex compound or a complex mechanism of action, or only the suppressant effect or with the variable (divergent), dose-dependent activity are fairly well studied and known. The discrete transmission is virtually identical to the damaging effect of risk factors, being manifested by sensitization, reduction of reaction thresholds, development of atypical behavior, formation of atypical proteins and atypical cells, development of various forms of dependency. Many diseases either cannot be eliminated using stimulants, repressors, divergents providing or accelerating at best a temporary remission (false-norm), or one has to eliminate the side effects and aversion that emerge with their use. Correction of the side effects often has to be carried out throughout the entire course of therapy, and even longer.

Given the novelty of the modulator concept, and the absence, as stated in the prior art, of drugs that can confirm its obligate criteria, the following must be emphasized:
- intensity of the modulating activity in a dose-dependent manner may not violate the obligate criteria for identification and evaluation of a certain commensurate effect;
- divergence of effects in a dose dependent manner (when at one dose only stimulating effect appears, while in another dose - only the suppressing one) is not an indication of the presence of a commensurate effect;
- divergence of effects beyond the range of single therapeutic doses can not detract from the merits of modulators;
- number of identified actions can not be indicative of a commensurate effect, unless they meet its obligate criteria;
- exfoliativeness of various accompanying dose-dependent action components cannot detract from the merits of a certain modulatory agent, because in each case, they may not only be individual, but also can occur in varying degrees depending on the dose.

For modulation and modulators with a specific commensurate effect, general concepts such as: modeling (model), modulating (module) and regulatory effect are not synonymous. Modeling and modulating effect are present at identification and assessment of any leading activity of the drug (whether of stimulants, suppressants, modulators, divergents), any concurrent effects and any adverse reactions. The regulatory effect is inherent in the very philosophy of the drug notion. An organism, a functional system and any single cell are a combination of subsets as a whole and none of their subsets is isolated from others, neither in any particular module (for example, in a specific area of the brain) nor from a particular module to other modules nor in the whole body.

Identification and evaluation of a modulatory activity with a commensurate effect does not require application of new research models, but requires new approaches and methods that primarily include studying certain indicator(s) given their initially high and low baseline level for different types of normal condition and different types of disorders.

Modulation with a commensurate effect, compared with suppression, stimulation and divergence is universal, but its universality can be limited by the evolutionary specificity of cells and tissues of the body and then it cannot have a uni-modulatory status even if in the presence of a promote-modulatory type of action. In such cases, the modulatory activity cannot be characterized without a prefix indicating the level of functional, pathogenetic, organotropic etc. type of the commensurate effect. It has its ranges and boundaries of effect within the ontogenesis and homeostasis, within the phenotype and age peculiarities in health and in disorders. Therefore the formantive (Lat. "formans, formantis" - forming) and fermative (It. "fermata" - indefinite pause or action duration) types of plasticity, the scenario of development of organization, disorganization, degradation of functional state are not equivalent and are not permanent.

From the postnatal to the fertile age, the fermative type of organization is under normal conditions aimed to achieve the formantive one in the fertile period. In the postfertile period, the fermative type of organization has the outward direction. Rhythmicity of stimulation and suppression, their reciprocally asymmetric relations and confrontation are never in equilibrium or absolute rest condition, neither on any of the levels or between levels. Consequently, the principle of true (commensurate) modulation is associated not only with how many mechanisms of action are included in a particular state, but also what the balance total of these relations is in a certain range of rhythm in a particular state or in the transition to a different state. Homeostasis, as the constancy of the environment, is constant only in the sense that it is constantly contradictory and constantly mobile.

Discovery and development of innovative modulators with a commensurate effect and divergents, identifying these types of activity in some known drugs that do not fit in the currently existing framework of the unreasonably conservative drug classification, will only expand over time, augmenting and enhancing their systematization. The modulatory concept does not imply that every modulatory substance or complex of substances must have a wide range of promote-modulatory and even more so of unimodulatory types of action. To solve some highly specific tasks, modulators with commensurate effect primarily on specific targets are required.

Discovery of new classes of drugs is a rarer phenomenon than discovery of new substances in the known classes and improvement of known products and their related products, but each of these areas is worthy and sufficient in itself by novelty, industrial applicability, applicability in various fields of medicine and related disciplines.

**Terms and their meanings used in the present invention¹:**
**destress*** (Ds) - desorganizational stage of a negative stress; condition of the body with stable hyperfunctional, hypofunctional or mixed type, as a consequence of distress;
**divergents* -** (Lat. divergerens - divergence) new class of drugs of natural or synthetic origin, the stimulating and suppressing activity of which differ depending on the dose;
**distress*** (ds) - term originally coined by H. Selye, but in the present document is understood, in contrast to interpretation of H. Selye, as an acute and subchronic negative stress;
**immunomodulators* -** substances or complexes of substances of natural or synthetic origin, that exert a commensurate effect on the level and from the level of the immune system;
**incretomodulators* -** substances or complexes of substances of natural or synthetic origin, that exert a commensurate effect on the level and from the level of the hormonal system;
**negative stress* -** a condition characterized by impairment of training stress factor set of body systems and factors with the occurrence of acute, subchronic or chronic disorders;
**modulators* -** (Lat. modulatio - dimension, dimensionality) is a new class of drugs of natural or synthetic origin, that exert a commensurate effect on processes of both stimulation and suppression, and their consolidated coupling, their reversibility, as well as substances that have the ability to conformations and reversibility;
**neuromodulators* -** substances or complexes of substances of natural or synthetic origin, that exert a commensurate effect on the level and from the level of the nervous system; Cs-modulators modulate the level and from the level of the central nervous system and As-modulators modulate the level and from the level of autonomic or peripheral nervous system;
**operandmodulators* -** (English "operand" - object of an operation) substances or complexes of substances of natural or synthetic origin, that exert a commensurate effect on the level and from the level with regard to the static and dynamic genes and proteins, as well as substances capable of overcoming formation of dominant forms of atypical nature;
**parapharmaceutical agents -** include SBAS* (special biologically active supplements), BAS (biologically active dietary supplements), restorative, adaptogenic, some homeopathic agents, hygienic, dental, cosmeceutical, and many other agents of therapeutic, preventive and corrective purpose for humans, as well as medicines for animals;
**promoutmodulators* -** (From Lat. "promovere" - promote and "ut" - like) substances or complexes of substances of natural or synthetic origin, that exert a commensurate effect on different levels directly and/or indirectly;
**psioperandmodulators (Ψ-operandmodulators)* -** same as operandmodulators, but they act on the level and from the level of higher nervous activity;
**psychomodulators (Ψ-modulators)*** - substances or complexes of substances of natural and synthetic origin, that exert a commensurate effect on the level and from the level of higher nervous activity;
**rejuvenescent* activity -** pharmacological prevention and correction of age-related changes of both external, and functional and/or pathogenic manifestation; effect on the average and maximum life expectancy and quality;
**SBAS* -** special biologically active supplements that contain in its composition no more than a single dose of a drug or in dilution of its therapeutic doses to a defined single, course or sub-course mass or volume of parapharmaceutical agent; they are not food supplements, but may be included into a dietary supplement to improve its biological properties and characteristics and then the food supplement goes into the category of SBAS even though it is related with the food supplement;
**slendering* activity -** pharmacological correction of body weight without a significant anorexigenic effect;
**related impurities for synthetic products -** a set of individual related impurities (process related substances of the active compound; synthesis products and precursors) and residual amounts of organic solvents;
**commensurate* effect -** specific activity, indicating the nature of the action; synonym of the true modulation (modulator, modulatory activity), differentiating essentially from the modulating effect; dependence of the effect on the condition;
**stress* -** common term for a negative effect on the bod y, which does not reflect an objective understanding of stress and in this case - a general characteristic that includes both positive and negative condition of the body, except for the exhaustion phase. The exhaustion phase can not be considered within the triad of stress, as it is not stress any more, but rather a consequence of desorganizational phase of negative stress;
**training stress* (Ts) -** a normal bio-rhythmic functional body state in tension and rest, depending on the type of norm that ensures its vital functions; the ability for adaptation and adaptive regulation with transition to another, more objective level of functional and pathogenetic temporary or permanent organization;
**training stress factor* activity (Tsf-activity) -** activity of substances with a difference in severity and direction of their effects on the initially high and low baseline levels of state of the body in conditions of objective norm or with transition into an objectively more effective plastic level of organization;
**cytomodulators* -** substances or complexes of substances of natural and synthetic origin, that exert a commensurate effect on the membrane and/or intracellular levels that possess, among others, the anti-apoptotic activity through a direct and/or indirect effect;
**exfoliative* activity -** pharmacological effects that exfoliate (stratify) against the leading activity in a dose-dependent manner;
**unimodulators* -** (Lat. universalis), substances or complexes of substances of natural or synthetic origin, that exert a commensurate effect not limited to tissue- and organ-specificity.
¹ New terms or those already known but introduced with a new or significantly changed meaning are marked with an asterisk

Further, the term "modulation" in the specification and the claim herein refers to a commensurate effect, also when the nature of the effect of a certain specific modulatory activity is not indicated.

**Object of the present invention -** development of a product that has a modulatory activity with the commensurate effect. This object is achieved by obtaining chemically pure stable compound of the racemic compound (RS)-2-(2-oxo-4-phenylpyrrolidin-1-yl)acetamide with stable characteristics that were identified for the first time and with the substantially improved known ones. The novel properties and characteristics of the (RS)-2-(2-oxo-4-phenylpyrrolidin-1-yl)acetamide within the present invention revolutionize the fundamental and essential understanding thereof, actually re-discovering this compound for biology and medicine that is supported by examples of the invention presented below.

As a result of the achieved, industrially applicable inventive step, the product and the related products thereof were obtained that do not carry significant disadvantages (defects) peculiar to the known compositions of the prior art and directly related to N-carbamoylmethyl-4-phenyl-2-pyrrolidone, namely:
1). A significant disadvantage of fonturacetam (fonturacetamum) or rac-2-[(4R)-2-oxo-4-phenylpirrolidin-1-yl] acetamide, according to sources [40] and [41], is that it is a nootropic agent, that is, a psychostimulant with nootropic component of action (N06B and N06BX) and contains in its composition one biologically active substance, rather than two. In such cases it is necessary to specify the ingredients of a medicine in one half of the composition of the racemate. Introduction of the precedent exception into the historically established chemical "racetams" group (pyracetam, pramiracetam, oxiracetam, aniracetam, levetiracetam, etc.) is not only capable of misleading, but also misleading.

It should be noted that this compound (derivative of diapyrrolidine, diapyrrolidone, specifically - phepirone) is not a chemical analogue, homologue or derivative of pyracetam (derivative of pyrrolidine - pyrrolidone), and "racetams" either by chemical structure or by chemical origin.

Exception to the rule in this case underlines the disadvantages of the product. The defects listed below are not eliminated, but even aggravated.
2). Significant disadvantages of Phenylpyracetam (Carphedon) from US 2009/0306225 A1 (published December 10, 2009, priority April 21, 2009) are similar to those of fonturacetam and emphasized not only by the INN feature, "racetam" (Phenyl**pyracetam**). The name of one drug (Phenyl**pyracetam**) includes entirely name of another drug (pyracetam - INN), a monomer and in particular of psychostimulant, not having any other basic activity).

The invention claim (paragraphs 1, 3) with respect to Phenylpyracetam is misleading. Unlike others (the monomer racetams), it indicates the content by the active substance, but not by mass of the racemate that completely changes the ratio of the components. Examples to support the ability of "racetams" (total number 16) to have mediated immunostimulatory (modulating) activity through stimulation of glutamate receptors in the brain when injected into the labyrinth of the middle ear, are not presented. It is not known also, for example, if levetiracetam is capable to be metabolized in the labyrinth of the middle ear, as its active substance, as is known, is not itself, but rather its secondary metabolite possessing anticonvulsant activity, which is diametrically opposed to stimulation.
3). A significant disadvantage of the product Carphedon in the description EP 2013166 B1 (priority 16.03.2006) and WO 2007/104780 A2 (published 20.07.2007, claimer Olainfarm, Latvia) is that the composition of Carphedon is destabilized and is a racemic mixture instead of the racemic compound. Only the stimulating activity was found in Carphedon (Table 1 and 2). Which activity will be exercised by a mixture of optically pure enantiomers in all other cases, is not known.

The method used for producing a mixture of isomers (Carphedon) is not specified, the stability is not defined. Optically pure products (R)/(+)-Carphedon and (S)/(-)-Carphedon have a similar pronounced divergent activity (Table 1, 2, and 3). With increasing doses, the stimulating effect disappears and the suppressing one emerges. The analgesic effect (using the hot plate method) is exfoliated from the suppressing one and appears with the use of their stimulating doses, which is typical, for example, for incretomodulators and in conditions of psycho-emotional arousal when the pain is applied in such a condition by an external physical impact.

In this case, stimulation (modulating effect) of locomotor activity is traditionally meant by "modulation" (Table 2 and p.4 of the claim). Otherwise is not stated, and otherwise does not follow from the presented examples of the research. No commensurate effect (true modulation) was identified. The present divergence and exfoliative effect (relative to the suppressing one) indicate the absence of modulatory activity with the commensurate effect.

The sources conclusions specifically state that "These findings confirm the high therapeutic efficacy of R-Carphedon that exceeds that of the racemic Carphedon, because pharmaceutical properties of the latter are adversely affected by the presence of S-Carphedon, which is characterized by a lower activity, and in some experiments - by much weaker activity".

The process for synthesis of isomers is not substantially different from that previously known for the racemate. Using the method of synthesis described in the claim (p7), it is impossible to obtain the substance and the substance with these properties (pp 1-6 of the claim), and the method for producing optically pure products (column chromatography) is not applicable commercially, which is obvious from the description of the invention.

The melting point is given for one isomer (107.5 - 108°C), not given for the other and the racemic mixture, which should be understood in such cases to be identical. The melting point not only completely disaccords with that previously known for products N-carbamoylmethyl-4-phenyl-2-pyrrolidone (132°C) from SU No 797219, A61 K21/40 and chemically pure "compendial grade" Carphedon (139-140°C) from 4.1 MUK.234-96 (Moscow, 2000, extended in 2004), but also is flawed by the same token, which will be confirmed below.
4). Even though Entrop, N- carbamoylmethyl -4-phenyl -2-pyrrolidone (Olainfarm, Latvia, N06V), according to its registered label, copies all the effects and significant drawbacks of Phenotropil [31], it acquires even more flaws. The drug ceases to have any effect on physical work capacity, which is extremely exceptional and negative phenomenon for any drug and even more for this racemate. Its stability is 1.7 times lower than that of Phenotropil and it is very demanding for the ambient temperature during storage (not more than 15 - 25°C). Given that the temperature storage conditions as maximum are not specified in accordance with the Pharmacopoeia requirements, it suggests that the polymorphism of product is developing also in the specified range.
5). The flaw of the product from SU No 797219, A61 K21/40 - "N- carbamoylmethyl -4-phenyl-2-pyrrolidone with antihypertensive activity" (priority from 08.05.1979, the date of publication 25.07.1995) is that exactly the compound claimed therein cannot have the aggregate melting point 132°C in combination with LD₅₀ = 831 mg/kg in mice, as they are completely mutually exclusive specifically for this compound as will be shown below. The invention has not received medical and industrial applications to date. The eloquent professional chemical characteristics in the description, "given that the compound is a product of condensation of piracetam and 4-phenylpyrrolidone-2... ", eliminates everything previously stated therein [1] considering the definition and understanding of condensation reactions in organic and pharmaceutical chemistry.
6). A significant disadvantage of such a product as the Carphedon "Pharmacopoeia" ("ASLG Research Laboratories", antihypertensive) from MUK 4.1.234-96 (Moscow, 2000, effect extended in 2004) is that the product claimed therein (1-carbamoylmethyl-4-phenylpyrrolidone-2) may not itself have the yellow color and the melting point of 130-140°C. Calculating all analytical series (from - at least and until - as a maximum) by the quantitative content (1-carbamoylmethyl-4-phenylpyrrolidone-2) in the products is not difficult for a specialist, in compliance with the Pharmacopoeia, unless otherwise indicated.
7). A significant disadvantage of the composition RU 2183117 C1 - ("ASLG Research Laboratories", priority from 03.11.2000), is that to achieve the desired effect with the drug to normalize blood pressure and treatment of hypertension (in 58% of cases as a maximum), course administration of N-carbamoylmethyl-4-phenyl-2-pyrrolidone at excessively high doses is required (750 mg in three divided dosages daily "but preferably taken at once"). The recommended effective course doses are 1000 mg 1 to 4 times per day, which exceeds the known LD50 in animals by several times. The safety index is not provided, and nothing otherwise is stated. The analogues closest to N-carbamoylmethyl-4-phenyl-2-pyrrolidone products are, according to nosology and trials in humans, the composition Carphedon "Pharmacopoeia" from MUK 4.1.234-96 (Moscow, 2000) and the composition from SU No 797 219, A61 K21/40, the flaws of which are described above.
8). A significant disadvantage of the composition from the Patent RU No 2240783 C1 (priority 17.07.2003) having nootropic activity and its method of preparation, is the fact that the composition contains 0.5% of a not characteristic (not related) accompanying individual impurity, which is in full sense extraneous (2-pyrrolidone), and accordingly, the total content of accompanying impurities is increased as a maximum by several times that are not identified.
9). A significant disadvantage of the composition from the Patent RU No 2327458 C1 (priority 19.02.2007), is that the compound is unstable in the claimed composition during storage (cf. the table of stability determination); the content of impurities increases which is unpredictable in terms of efficacy; no new properties were revealed. The use is extremely limited by the method of administration, unacceptable for pediatric practice due to the presence of ethyl alcohol. Toxicity of the products is increasing.
10). A significant disadvantage of the pharmaceutical composition from RU 2414898 C1 (priority 18.09.2009), possessing nootropic activity, is that at lower amounts of active racemate during storage of the composition, the psychostimulant activity increases compared with a solution of the substance and hence the amount of unidentified impurities with stimulating activity, competing for binding sites, increases. There is no reason to exclude the stimulating synergism of the main active composition and impurities.
12). A significant disadvantage of the pharmaceutical composition from WO 2009/048352 A1 (priority 12.10.2007) with finely micronization, is the adhesion of H₂O molecules by molecules of racemate that distorts the substance properties, forming a stable suspension. The water molecules rather firmly attach to each molecule of enantiomer, locking them by the acetamide group; the pharmacological activity drops sharply, the industrial application is not practical.
13). A significant disadvantage of a pharmaceutical composition in microencapsulated multicomponent particles form of N- methyl-carbamoyl-4-phenyl-2- pyrrolidone (patent RU 2391976 with priority of 18.10.2007 (A pharmaceutical composition in form of microencapsulated multicomponent particles of N-carbamoyl-methyl-4-phenyl-2-pyrrolidone and an auxiliary neutral organic low molecular weight component and a method of microencapsulation thereof) is that the mass of the substance increases due to presence of the additional component. The composition is unstable, biological properties deteriorate, the industrial application is not practical.
14). The most widely known from the prior artis the original drug Phenotropil^{®tm} [31; 2-7; 9-21; 25; 26; 32-34]. The following inventions with industrial and clinical applications have direct relation to it: Patent RU No 2050851 C1 "Substance having nootropic activity" with priority date August 28, 1990, Patent RU No 2232578 C1 with a priority date April 10, 2003 and its foreign analogues (antidepressant activity), Patent RU No 2329804 C2 ("Substance having neurotropic-neuromodulator activity" with priority of 28 March, 2006) and its foreign analogues (treatment of hemorrhagic and ischemic stroke, WO 2007/111528). The listed inventions do not overcome the disadvantages of Phenotropil products [31], which include:
   - limited use in case of positive and negative symptoms of mental disorders associated with psychomotor agitation, delusions and hallucinations, not just during manifestations, but also if there is a history of such episodes due to possibility of provocation;
   - limited use in severe forms of hypertension;
   - immune-stimulating and hormone-stimulating effect are a priori contraindicated in hyper-conditions of the immune and endocrine systems;
   - anorexigenic activity a priori is contraindicated for use in patients with anorexia;
   - divergence of the basic activity;
   - exfoliative features and other related components of action are not able to provide a modulatory activity with a commensurate effect;
   - nootropic activity has the nature of direct stimulation, as otherwise it is not revealed;
   - no true modulatory activity (commensurate effect) has been revealed.

A disadvantage of Phenotropil is also the limited use of the maximum daily dosage (750 mg) due to the therapeutic index (ratio of the maximum daily dose to LD₅₀ = 800 mg/kg in animals) in emergency clinical cases, in view of pharmacokinetics (V.I. Akhapkina, A.S. Berlyand, 2002; V.I. Akhapkina, S.N. Potrugalov, 2002). In emergency cases, constant maintenance of high doses is often required for several days or even weeks. The compound is excreted from the body in normal conditions and high physical activity for 72 hours. This fact limits the use of high doses in clinic, since it is known that with the reduced metabolism pharmacokinetics may change considerably. On the one hand, emergencies require increased doses, and on the other hand and due to a slowdown or decline in activity of barrier and excretory functions, the emergencies prevent this; the therapeutic index should therefore ensure safety of any drug.

According to findings of Y.Y. Firstova [33], devoted actually to gerontology, judging by the body weight of animals (mice 30-35 g), we can talk about the absence of effect of Phenotropil on life expectancy in older animals and that it has no mnemotropic activity, while its nootropic activity is characterized by stimulation. It was shown that Phenotropil does not affect the expression of growth and differentiation factor, BDNF, in the cerebral cortex, and the low effective group of animals displays the negative effect direction by this indicator. By "modulation" (title of the paper), the author understands the modulating effect (description and conclusions), i.e. stimulation of the studied indicators of mechanisms of action. In the low and high effective animals, Phenotropil has the similar effect (a stimulating effect on the NMDA receptors with varying degrees of intensity). Aging processes relate to the global changes in the body that trigger the growth of neurohormonal disorders in the outwards direction with accumulation of atypical dominant forms and it is impossible to resolve them using functional agents.

It has been discovered (priority January 2006, submitted for publication in 2005) that the racemic composition of N- carbamoylmethyl -4 -phenyl -2- pyrrolidone, or with any other accurate chemical name, obtained in production by the described synthesis method, in crystalline (solid) state is a racemic compound [34]. The concepts of the racemic mixture and the racemic compound in organic chemistry are not equivalent. Results of a study of the compound X-ray analysis are deposited in the Cambridge Structural Database System, (QELNEB, 2007) with reference to the source of information [34]. Disadvantages of the method of preparation include: unstable yield of the compound in technical raw materials during the synthesis that is 50-70%, composition and quantitative content of impurities are not either established or identified; no other, unknown properties and characteristics have been identified. Obtaining the chemically pure and stable composition by synthesis and by azeotropic removal of water is not possible. The above-mentioned defects are neither identified nor eliminated.

The obvious disadvantages of the products and their related products of the prior art, inconsistency of their properties and characteristics indicate the difficulty of obtaining and reproduction of stable compositions of the compound. The developed product and its related products within the present invention are devoid of these shortcomings. Their industrial production processes are simple, significantly reducing costs of expensive reagents.

### Disclosure of invention

### Examples 1. A method for producing the compound and its highly pure chemically stable pharmaceutical compositions, detection of their characteristics.

Synthesis of the desired technical raw materials were carried out by the developed one-pot process of thermodynamic equimolar disproportionation from 4 (RS)-feniilpirrolidin-2-one (fepiron, prepared by cyclizing of the linear derivative precursor) in an alkaline medium, followed by addition of acetamide-derivative without the use of catalysts, DMSO or sodium metal. The yield of compound in the raw materials is not less than 75%.

Purification, crystallization, and stabilization of technical raw materials were performed as follows: after cooling, the technical composition is treated with demineralized (distilled) water, settled, the liquid fraction is separated and isothermal crystallization from isopropanol is conducted. Then it is dried at 40-70°C to achieve constant weight with content of the desired compound at least 99.0 % calculated on a dry basis and the melting point in the range 130 to 133°C (comparing the capillary and derivatographic methods), or at least from 128°C (typically in this case, the beginning of melting of the powder is from 129°C) with content of the active compound less than 99.0 % (but no less than 98.0 %), but then the impurity content and loss of the weight in drying in manufacture of formulated compositions will be higher, as the related impurities are more hygroscopic than Phenotropil, and some of them are highly reactionary. The melting point which is beyond the boundaries defined above directly indicates that the composition contains a large quantitative content of impurities, or the composition is distorted and then it is no longer the specified compound. The melting point diagrams of the racemic compounds and racemic mixtures have characterological differences.

For comparative studies, a compound with content of not less than 99.8% (100 ± 0.2) was prepared, by the method of chromatographic separation with impurities, followed by crystallization and stabilization of the crystalline state.

The resulting compositions are stable. The studies were conducted in accordance with regulatory requirements for determination when stored in natural conditions (in a dry, dark place at a temperature not exceeding 30°C) and using the standard method of accelerated aging. Compositions containing not less than 99.0 % of active compound, based on dry substance, are stable for at least five years (loss in weight on drying did not reach the levels of 0.5 % during long-term storage in natural conditions), and containing less than 99.0 % - for at least three years. The stable product itself does not decompose during storage and does not react.

The analytical assay methods are shown in Table 1. The precise quantification was made by the developed titrimetrical method (TMT): 0.2 g (accurately weighed) of 2-(2-oxo-4-phenylpyrrolidin-1-yl)acetamide substance was placed in a Kjeldahl flask, 20 ml of 0.1 M hydrochloric acid was added. The flask was attached to the apparatus for determination of nitrogen (National Pharmacopeia vol XII, Part 1, p. 101) and the distillation was started. After settling the steady state, 40 ml of 30 % sodium hydroxide was slowly added to the distillation flask, making sure that the solution in the flask was stirred vigorously by the steam flow. 200 ml of the distillate were collected in the receptacle with 20 ml of 4 % boric acid solution and 0.1 ml of mixed indicator solution. The distilled fraction was titrated with 0.1M hydrochloric acid to achieve the red-violet color. The control experiment was carried out. The replication error for a result of individual assay by the TMT method is ∼ 0.2% relative to the standard sample solution (SSS). The value of the systematic error is not significantly different from 0 relative to the control experiment. It has been established that 1 ml of the 0.1 M HCl solution corresponds to 0.021826 g or 21.826 mg (21.83 mg) of C₁₂H₁₄N₂O₂ (Phenotropil).

TMT is the most accurate method (standard error no more than 5%) compared with any chromatographic methods including TLC, HPLC and UPLC (standard error reaches 20 %). Individual impurities and their quantitative content were determined by HPLC (high performance liquid chromatography) or UPLC (ultra performance liquid chromatography). Comparison of the results of TMT and HPLC provides objective information regarding the quantitative content of the active compound, individual related impurities and their ratios.

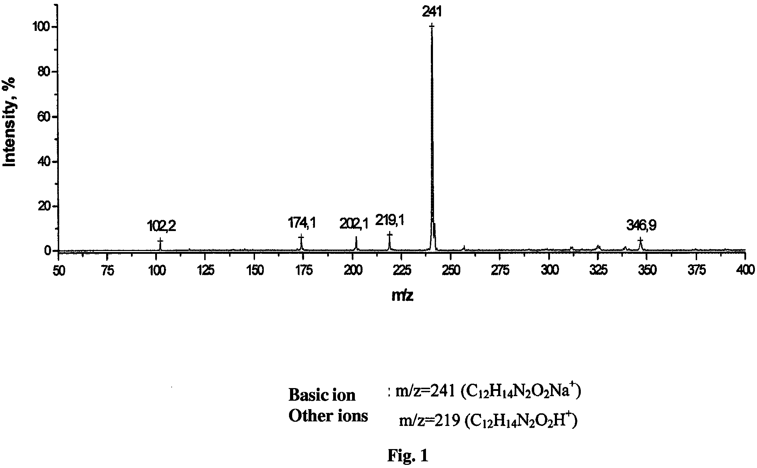

According to NMR and mass spectral analysis, it was found that traces of individual impurities are present in the desired products, but their quantitative content is unable to affect the properties and characteristics of the desired compound. The mass spectrum of positive ions of a typical sample is presented (Fig. 1). Individual impurities were identified and their quantitative content in one of the samples is shown in Table. 1.1. The list of individual impurities will depend on the method of synthesis. IR spectra of the racemic compound and racemic mixture have characteristic differences.

An analytical analysis of compositions with less than 99.0% of the active compound content in terms of dry matter and content of the related individual impurities more than 0.2% requires detailed elaboration of impurities content and their item-by-item quantitative specification in the technical documentation for the drug. The nomenclature of impurities in this case is invariable, but their quantitative composition can vary for each item in the prepared series batches.

When changing the method of synthesis, the nomenclature of individual related impurities changes, but their quantitative content in the aggregate shall comply with the specified one. The presence of a single individual impurity is an exception rather than the rule, so a determination of a single alleged impurity by TLC may be misleading.

Use in the desired raw material synthesis method of a dimer of 4-phenylpyrrolidin-2-one prepared using nitromethane would require analysis for the absence of cyanide in the chemical composition of the source and the desired product. The presence of even traces of cyanide in a series of unidentified impurities is unpredictable by effects on the body in case of course application of medicinal and pharmaceutical agents. The methods of purification, crystallization and stabilization of compounds in such cases may be much more costly than the synthesis itself. The source product prepared with use of nitromethane was never and is not being used in the synthesis of Phenotropil except for samples for comparative studies. Upon reaching the required purity of the substance, the list of the related impurities in the absence of toxic and highly toxic substances has no significance any more, as established, for the physico-chemical, organoleptic qualities, as well as for the biological ones as will be shown below.

The melting point range of each of the dried samples (from and to) was always less than 1°C (0.2-0.9°C) determined by the capillary and derivatographic methods. The statistical reliability of the indicators is high (more than 120 measurements of different samples).

Depending on the characteristics, the Compounds can be categorized as follows:

### Compound 1:

| | |
|---|---|
| (RS)-2-(2- oxo-4-phenylpyrrolidine-1-yl)acetamide | No less than 99.0 % and no more than 100.5 % on dry basis |
| Individual impurities, single or in total | no more than 0.2 % |
| Residual amounts of organic solvents | no more than 0.3 % (no more than 3000 ppm) |
| Heavy metals | no more than 0.001 % |
| Sulphated ash | no more than 0.1 |
| Loss on drying | no more than 0.1 % |

### Compound 2:

| | |
|---|---|
| (RS)-2-(2-oxo-4-phenylpyrrolidin-1-yl)acetamide | No less than 99.0 % and no more than 100.5 % on dry basis |
| Individual impurities, single or in total | no more than 0.25 % |
| Residual amounts of organic solvents | no more than 0.3 % (no more than 3000 ppm) |
| Heavy metals | no more than 0.001 % |
| Sulphated ash | no more than 0.1 |
| Loss on drying | no more than 0.1 % |

### Compound 3:

| | |
|---|---|
| (RS)-2-(2-oxo-4-phenylpyrrolidin-1-yl)acetamide | No less than 99.8 % and no more than 100.2 % |
| Loss on drying | no more than 0.02 % |

### Compound 4:

| | |
|---|---|
| (RS)-2-(2-oxo-4-phenylpyrrolidin-1-yl)acetamide | No less than 98.0 % and no more than 100.5 % on dry basis |
| Individual impurities, single or in total | no more than 0.5 % |
| Residual amounts of organic solvents | no more than 0.5 % (no more than 5000 ppm) |
| Heavy metals | no more than 0.001 % |
| Sulphated ash | no more than 0.1 % |
| Loss on drying | no more than 0.5 %. |

The microbiological purity and/or apyrogenicity met the requirements of Pharmacopoeia. Preparation of organic substances according to requirements of the Pharmacopoeia does not require the use of special methods. Stable compounds can readily handle higher levels of heat treatment, as the sublimation and decomposition of the product occur at much higher temperatures than those required for sterilization. No polymorphism was detected during storage. Polymorphism was present in the samples of the Compound 4 that contained less than 99.0% of the active compound on the verge of 3 years and above, which was easily eliminated by sieving and drying the samples with a certain degree of weight loss.

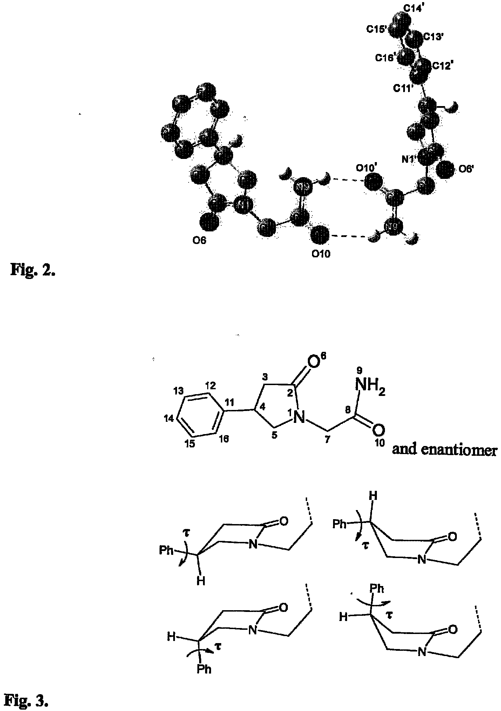

The X-ray structure analysis of the crystalline powder of all Compounds, including study during storage, confirmed the nature of the active compound as a racemic compound, which is schematically presented in the unfolded state in Figure 2. The racemic compound of each pair of chiral molecules complexon has the form of twisted ribbons oriented along the long diagonal of the central axis of the unit cell. The leading intermolecular bonds between atoms H-O (10) and H-O (10') are characterized as medium by strength.

Thus, these studies show that innovative chemical Compounds were obtained, with stable parameters of melting temperature and preservation of all established physical-chemical and structural indicators during prolonged storage, with the exception of the negative and masking effect of the related impurities, as well as distortion of the crystal structure of the compound. Chemical compositions of the compound that contains in the racemic compound, in a 50:50 ratio, 2-oxo-4(R)-phenyl-1-pyrrolidinyl acetamide and 2-oxo-4(S)-phenyl-1-pyrrolidinyl acetamide are stable, and do not contain any impurities capable to substantially mask or adversely affect the characteristics of (RS)-2-(2-oxo-4-phenylpyrrolidin-1-yl)acetamide.

The crystalline powder of the Phenotropil substances is not volatile, has white, rarely white to slightly yellowish or slightly cream color, bitter taste (bitter taste that disappears in the mouth rather quickly after dissolution, leaving no unpleasant sensations). In the related products, the bitter taste persists. The Compounds are sparingly soluble in water and saline, moderately soluble in 96.0 % alcohol and chloroform. Readily soluble in water and saline under heating, but the solutions are not stable during cooling and storage under normal conditions (not more than 2 hours, which is acceptable for the extemporaneous preparations and research), moderately soluble in 5.0 % glucose solution, dissolve well in the presence of solubilizers and emulsifiers. Tween₈₀ and cyclodextrins increase solubility in water, especially Tween₈₀, while cyclodextrins have limitations by concentration. Sterile 0.001-3 % solutions of Phenotropil in the presence of cyclodextrin or Tween (water for injection, Phenotropil, cyclodextrin (or Tween solution) were thoroughly stirred for 30 minutes, filtered, dispensed into ampoules of neutral glass, the ampoules were sealed and sterilized for 15 minutes at 120°C). Sterile solutions are storage stable for at least 12 months. The ultimate shelf life is not specified; the samples are being stored.

The lipophilic properties of the compound are expressed to a much greater degree than the hydrophilic ones, which must be considered when designing the regulations for various formulated compositions. It is established that in the presence of excipients, the composition within the therapeutically required concentration does not undergo any quantitative change in the active compound during storage, unlike the use of acids and alcohols for those purposes. Stability of solutions is sufficient. The preparation of other dosage forms is technologically simple and does not require use of complex formulations of excipients and carriers.

The obtained results of the study clearly demonstrate that the stable compound (RS)-2-(2-oxo-4-phenylpyrrolidin-1-yl)acetamide is for the first time obtained in pure form in the present invention both itself, and its chemical compounds of pharmaceutical substances, which entirely correspond by their physical-chemical and organoleptic characteristics to the pure compound itself or are as close to it so that not only they do not distort, but do not mask any significant physical-chemical and organoleptic properties and characteristics. Processes for their preparation, assay and identification of the compound and the related impurities are simple and reliable.

The method for producing the pharmaceutical and parapharmaceutical compositions based on or including (RS)-2-(2-oxo-4-phenylpyrrolidin-1-yl)acetamide in various formulations for internal and external use is different in that, after sieving, the powdered substance is further dried (if it is characterized as Compounds 2 and 4) to achieve constant weight, corresponding to at least Compound 1. The required amount of the pharmaceutical substance powder weight based on the absolute active compound was calculated, based on the actual percentage content of 2-(2-oxo-4-phenylpyrrolidin-1-yl)acetamide therein and then the stepwise procedure starts to produce acceptable formulations according to the industrial regulation or pharmacy techniques using known and suitable excipients and carriers that are authorized for use.

The substance is characterized by solubility, predominance of lipophilic properties, tendency to complementarity, ability of molecules to conformations. Achieving good solubility in water and saline solution with heating does not require high temperatures. Based on our experience, usually, based on the weight of the powder, it is enough to take no more than additional 1.01%. Production losses for this product are minimal. In the manufacture of any solid, soft formulations and patches, as well as plates, they do not exceed 0.2 % (main losses occur during unloading the substance from the packaging container), and during the manufacture of liquid and aerosol formulations - not more than 1.0%.

The following compositions can be prepared on the basis of or with the inclusion of (RS)-2-(2-oxo-4-phenylpyrrolidin-1-yl)acetamide:
Pharmaceutical and parapharmaceutical compositions for internal use, characterized for 100% of mass, mass-volume, volume by the following ratio of the components:

| | |
|---|---|
| (RS)-2-(2- oxo-4-phenylpyrrolidin-1-yl)acetamide | 0.01-75 |
| Other componets, individually or in total | 99.99-25. |

Pharmaceuticals and parapharmaceutical compositions for topical use, characterized for 100% of mass, mass-volume, volume by the following ratio of the components:

| | |
|---|---|
| ((RS)-2-(2-oxo-4-phenylpyrrolidin-1-yl)acetamide | 0.001-90 |
| Other componets, individually or in total | 99.999-10. |

"Other components" may include both excipients and carriers or special purpose additives which are approved, and additional approved biologically active components. According to its stability properties, the pharmaceutical Compound 1 is suitable for the manufacture of compositions not only industrially, but also in pharmacy conditions.

The X-ray, electron structural, electron diffraction studies of (RS)-2-(2-oxo-4-phenylpyrrolidin-1-yl)acetamide, conducted for the first time in different environments, have shown that depending on the environments and the environments changes (temperature, pH, density, electrolyte composition, electrical conductivity, electromagnetic field, etc.) the enantiomeric molecules of the compound possess the ability to form different conformations and conformers both separated in the envelope (volume) of conformational molecules (additional four, six and eight were found, but it is not excluded that their quantity may be more), and various packed (cassettes) complexons in an envelope. Complexons of more than two molecules have different shapes. An example of some variants of rotation in a gaseous medium (gas electron diffraction analysis) at two variants of the nucleus (asymmetric carbon atom in the position 4 of the nucleus of the structural formula) of enantiomeric molecules of the compound and at two variants of the six-membered ring (Ph) of the functional group is represented in figure 3.

The prognostic number of variants of potential rotation functions and stereospecific behavior of chiral molecules cannot be predicted clearly or definitively depending on changes in the environment. This requires more in-depth studies, yet they can not essentially change anything, although they can elaborate the behavior of molecules in various organs and tissues depending on their condition. This pivotal discovery for the compound indicates that it is impossible to judge about its properties and characteristics, even from the standpoint of the original optical activity of enantiomerically pure substances, since there is no reason to exclude for them the possibility of transition into each other in connection with the identified circumstances.

Molecules of the racemic compound exhibit a sufficiently high degree of complementarity at the transition from gaseous or liquid medium into the crystalline state. Saturation of the compounds with molecules of other substances and disproportionation can lead to distortion of complexons. Other substances present in the compounds are able to compete and to distort the molecule in the compounds, as well as compete for binding sites in the body. Tendency of molecules to complementarity is reason for disposition of the optically pure isomer compounds racemization, which starts pretty quickly. These properties must be considered in preparation of optically pure isomers.

Pharmacological studies of the compounds and the compositions, the statistical analysis of the findings were carried out on the standard, well-known models and methods in accordance with the laboratory and clinical-physiological placebo-controlled practice, "Guide for experimental (preclinical) study of new pharmacological substances" (Moscow, "Remedium" 2000), obligate criteria for identification and evaluation of modulatory activity as a commensurate effect.

In the above models and the methods of pharmacological studies in the examples presented below, unless otherwise indicated, it should be understood that they are well known to specialists. Omission of the specified number of the compound in the examples of studies refers to Compound 1 for Phenotropil^{®}™. The number of biological subjects in groups, unless otherwise specified, was at least 10; a placebo, conventional solvents (distilled water or saline) and compositions with other carriers and excipients were used in the control groups. Body weight, sex, age of animals, unless stated otherwise, corresponds to white outbred male rats or mice of standard sexual maturity age by body weight. When selecting subpopulations (highly effective - HEF and low effective - LEF) in animal populations, the ratio was 2:1, respectively, with permissible testing error of not more than ± 10.0%. Preparation of dosage forms was carried out based on the identified physical-chemical properties of the test compound and its pharmaceutical compositions.

### Examples 2:

Tables 2, 2.1 and 2.2 present the results of acute toxicity studies of the Compounds with active substance content in at least white outbred male mice (body weight 18-24 g) and white outbred rats 30-35 days of age, of both sexes with a single dose. Calculation of toxic doses was made using the Wilcoxon and Litchfield method. Observations were made over 24 hours and 14 days after a single dose.

When administered intraperitoneally (Table 2) to adult mice, LD50 was: for Compounds 2 and 1-1001 (959.1-1042.9) mg/kg and 1042 (982.9-1102.0), respectively, for Compound 3 - 1050 (984.6-1115.3), for Compound 4 - 904 (857.3-950.4 mg/kg). At intraperitoneal injection of Compound 1 in rats (Table 2.1), LD50 = 1029.5 mg/kg (940-1119.0). LD50 in rat pups after intragastric administration was not found. Intragastric administration to rat pups of doses of more than 1000 mg/kg is technically impossible due to the small volume of the stomach (Table 2.2).

The results show that, without the negative and masking effect of the process-related impurities, the toxic doses of the compound are substantially higher than it was previously known for all items. The enantiomeric molecules of the compound themselves without impact of related impurities have low toxicity. The resulting chemical compounds allow to address the issue of the therapeutic index, increasing the safety of high daily doses by at least 25% based on studies in adult animals and 30-35 days old rats. The closest to Compound 3 by this indicator is Compound 1. It is inappropriate to establish the standard indicator LD50 greater than 1000 mg/kg as it is established for pharmaceutical substances with a content of active substance or complex of active substances by category "as minimum" and at quantitative content of related impurities "as maximum".

Taking into account that Compounds 2 and 4 are technologically simply (sieving and drying) brought to the state of Compound 1, they are suitable for the manufacture of dosage forms with the inclusion of appropriate preparation stage and bringing to the desired characteristics by the quantitative content of the active compound and related impurities. Stability of Compounds 2 and 4 is sufficient for their transportation and storage, as well as for producing Compound 1 from them with use of simple preparation. Stable chemical compounds from - as the minimum to - as the maximum are not subject to destructive changes and disproportionation under specified terms and conditions of storage. Individual impurities are able to compete for binding sites in the body. They easily pass through the blood-brain barrier. An important role is played by proportionation of individual impurities and residual amounts of organic solvents, crystallization and stabilization mode. Compound 1 is the most approximated by its properties and characteristics to Compound 3; industrial achievement of the obtained technical result is not difficult, so further biological studies were mostly conducted with Compounds 1 and 3.

### Examples 3:

(RS)-2-(2-oxo-4-phenylpyrrolidin-1-yl)acetamide has a psychomodulatory activity with the commensurate effect on motor activity of rats. The effect is manifested commensurately depending on the initial state in the genetically HEF (highly effective) and LEF (low effective) white outbred rats 30-35 days of age (90-110 g) of both sexes by the motor activity test (Table 3). It was shown that in the doses tested (5 and 10 mg/kg intragastrically) it displays a diametrically opposite direction of the psychotropic effect and its different intensity after a single administration. In the HEF group, when treated with Phenotropil, the motor activity decreased by the total index dose-dependently by -12 % and -4.23 % compared with the control (distilled water), while in the LEF group this indicator increased by 72 % and 67 % at doses of 5 and 10 mg/kg, respectively, compared with the control group.

Using the cross-type maze model (Salimov et al., 1995), male mice of 1CR strain (18-22 g) were examined for orientation-exploratory behavior when treated with Phenotropil (100 mg/kg) - Compound 1 in unfamiliar situational conditions versus Compound 4 (100mg/kg), piracetam (200 mg/kg) and control group (distilled water) administered intraperitoneally sub-chronically (for 5 days) once a day. The animals were tested before the drug administration and one day after the last administration. Additionally, Kit was calculated (trigger transmission coefficient - the ratio of the interdependence of the numerator of one indicator individually or in total to the denominator of another indicator individually or in total in comparison with the control in arithmetic or integral terms). Kt (Table 3.1) in the LEF group was almost two times higher than in the VEF group and by 50% higher than in the norm control population. The norm of Kt for different studied effects has individual numeric expressions when calculated arithmetically or integrally.

It can be concluded that it is the trigger transmission, reversibility and contraversivity of conformations of the Phenotropil molecules themselves, which prevents development of the neurosis-like effect which was manifested after treatment with psychostimulant Pyracetam with the fermative type of action, according to the ratio of number of explorations of compartments and the number of surveillance in both groups. The psychomodulatory activity of the Compound 1 is commensurate and more pronounced than that of Compound 4. With systemic use, the effects of Compound 1 practically equalized the indicators of trigger transmission of the LEF with the HEF groups, translating them into a objectively more plastic functional state, both in the LEF, and in the HEF animals. Probably, the more "rigid" consolidation of functional and structural relationships in the HEF is not an exclusively positive feature for plastic organization, just like a highly labile in the LEF animals.

Incretomodulatory activity of the compound was found in calibration of biologically sensitive doses of the drug in Phenotropil Compound 1 when administered intraperitoneally to male rats (210-230 g) of the August strain (genetically low resistant to negative stress - Ns) and Wistar strain (genetically highly resistant to Ns) in the dose range of 0.5-750.0 mg/kg. The doses exerting a significant effect on the plasma cortisol level in rats were measured and the mean for them was calculated. The control animals received distilled water. After decapitation, blood sampling for biochemical studies of plasma cortisol was performed after 60 minutes (in 5 rats in each group) and after 3 hours (in 5 rats in each group) after the drug administration. The plasma test was conducted using chromatographic method (Schwarz, A., Roberts, W.L., Pasguali, M. Analysis of plasma amino acids by HPLC with photodiode array and fluorescence detection // Clinica Chimica Acta.-2004.-V. 312.-P. 253 -262). The study results averaged across the groups are presented in Table 3.2.

The biologically active dose of the compound was manifested reliably, starting from 1.0 mg/kg. The Tsf- activity, taking into account the known role of cortisol to the body, of (RS)-2-(2-oxo-4-phenylpyrrolidin-1-yl)acetamide manifested with commensurately pronounced effect on cortisol and was +14.1 % in the August strain group, and +2.0 % in Wistar one hour after administration. After three hours, the incretomodulatory activity manifested with diametrically opposite direction of effect. In the Wistar group it was (-7.6 %), while in the August strain group it was +6.8 %. After a single dose, the modulation factor was more pronounced in the August strain group. While in the control it was 1.13, then when treated with the compound - exactly 1.0 (- 11.5% compared to the control), that is the functional state reorganized back to normal, exceeding even the value for Wistar rats. In control Wistar it was 0.9 and 0.88 when treated with the compound. The modulation factor (Kₘ) is the numerator of the indicators difference in the study group after treatment and before treatment divided by the denominator, the square root of the sum of the squared difference of the numerator and the difference of values in the control group after and before treatment with placebo, with determining the effect of the substances on the balance value of relationships and interdependencies of these indicators. The higher the modulatory activity, the more Kₘ is approaching to norm and to the greater extent it tends to one or is equal to one (1.0).

The compound has a modulatory activity with the commensurate effect at all doses tested in all the investigated models. This indicates that the specific modulatory activity is a leading one of the compound, and does depend not as much on the dose as on the initial state. This opens up completely new perspectives for Phenotropil and shows that it is a true modulator. The psychomodulatory and incretomodulatory activity is combined with the training stress factor activity, which also manifested commensurately depending on the initial state of the organism. Therefore, we can assume that the adaptogenic activity of Phenotropil has distinguishing features both from just stimulating and just suppressing drugs in the negative stress conditions, which also opens up new opportunities for adaptive regulation with use of innovative pharmacological agents.

### Examples 4:

A unique neuroleptic (antipsychotic) activity of (RS)-2-(2-oxo-4-phenylpyrrolidin-1-yl)acetamide is identified, which does not depend on the nature of mental disorder. Studies have been conducted on white outbred male mice (18-24g), in comparison with the olanzapine (Zyprexa) with intraperitoneal injection.

In the model of positive symptoms of schizophrenia associated with hyperactivation of the dopamine systems, which allows to identify the ability of substances to block the dopaminergic transmission in the mesolimbic system of the brain, Phenotropil (100, 200, and 300 mg/kg) reduced the apomorphine-induced (2 mg/kg subcutaneously) verticalization intensity scores (Table 4) by 45 %, by 2.0 and 2.6 times, respectively. Olanzapine was significantly inferior in activity to Phenotropil in the apomorphine-induced verticalization test. Treatment with Phenotropil decreased the verticalization intensity only by 26 % at 1 mg/kg and by 66.5 % at 10 mg/kg.

In the model of negative symptoms of schizophrenia (Table 4.1) associated with hyperactivation of systems (induced by 5-oxytryptophan at 300 mg/kg intraperitoneally), olanzapine at 1 mg/kg completely eliminated the shakings caused by 5-oxytryptophan. Phenotropil in all studied doses reduced hyperkinesia (head shakings): at doses of 100 mg/kg-by 88 %, at 200 mg/kg - by 87 %, and at 300 mg/kg - by 89 %. No significant difference depending on the doses of Phenotropil studied was revealed by this test. Effect of Phenotropil on serotonergic system requires more detailed testing and studies of lower doses, since there is no reason to exclude neuroleptic activity in this test. Preventive or proactive use of lower doses may be appropriate also in dopaminergic pathology.

Due to effect on recovery of M-cholinergic transmission (considered as a link in the pathogenesis of psychosis and allows to reveal the possibility of adverse reactions during treatment with neuroleptics) after its depression by arekolin (25 mg/kg sc), olanzapine at a dose of 1 mg/kg significantly reduced the duration of tremor by 57% (see table 4.2.). Phenotropil at doses of 200 and 300 mg/kg significantly reduced the duration of tremor by 63 and 50%, respectively.

The antiparkinsonian activity of Phenotropil in the model of haloperidol-induced catalepsy in mice is shown in Table 4.3. Duration of catalepsy was measured in seconds, after 60, 120 and 180 minutes after the drug administration. Phenotropil was administered intraperitoneally at doses of 50, 100 and 300 mg/kg 15 minutes after haloperidol 0.5 mg/kg. Haloperidol induced catalepsy growing over time. Phenotropil at doses of 50 and 100 mg/kg completely prevented the development of catalepsy as compared with the control 60 min after administration, and at a dose of 100 mg/kg no catalepsy was observed. At a dose of 50 mg/kg, duration of the catalepsy after 180 min was only 4.8 seconds as compared to controls (63.4 seconds). At a dose of 300 mg/kg 60 min after the administration, it increased the severity of haloperidol catalepsy compared with the control group, but then the severity decreased markedly by 2.6 times, and after 180 minutes its severity compared with the control decreased threefold. Phenotropil markedly counteracts haloperidol-induced catalepsy, indicating its antiparkinsonian activity.

### Examples 5:

Study of the effect of Phenotropil (100, 200, 300 mg/kg) on motor activity and exploratory behavior by the open field test on mice (16-24 g) in comparison with olanzapine (1 mg/kg) is shown in Tables 5 - 5.4. Olanzapine at higher doses has sedative activity, so for comparison with Phenotropil, the drug was used only at a dose of 1 mg/kg. The drugs and distilled water to control groups were injected intraperitoneally 40 minutes prior to the study. Olanzapine significantly reduced the horizontal locomotor activity (Table 5), reducing the overall run distance by 36%. Phenotropil increased the horizontal locomotor activity during the study period at a dose of 100 mg/kg by 112.2%, at 200 mg/kg by 151.8% and at 300 mg/kg by 99.5%. This indicates the ability of the drug to reversibility, unlike olanzapine.

In terms of vertical motor activity (Table 5.1.), Olanzapine significantly reduced the number of rearings by 47%. Phenotropil at a dose of 100 mg/kg increased the total vertical locomotor activity by 237.5 %. At a dose of 200 mg/kg, it slightly inhibited the vertical motor activity in the first minute, while in the second minute it was restored to the level of the control group, and in the third minute exceeded the control values twofold. Overall for three minutes, the study indicator was slightly lower (-6.3 %) compared with the control due to the effect in the first minute. Effect of Phenotropil at 300 mg/kg showed a significant reduction in the number of rearings at the first minute (-75 %). But then the number of rearings increased and the total increase in the number of rearings for 3 minutes was +56% compared with the control group, which also demonstrates the ability of Phenotropil to reversibility and over time.

In terms of the effect of the drugs on the exploration of openings as compared to the control (Table 5.2), Phenotropil increased the total exploratory behavior index by 50% at a dose of 100 mg/kg, while at 200 mg/kg the rate decreased by 32%, and at a dose of 300 mg/kg the total index of explored holes increased by 27%. Olanzapine had no effect on the number of explorations compared to the control. By the number of washes (Table 5.3), Olanzapine had a negative effect, increasing their number by 150% during 1 and 2 minutes, and in total - by 200%. Phenotropil at a dose of 100 mg/kg reduced the total index by 25 % compared to the control group. With Phenotropil at a dose of 200 mg/kg, no washes were observed. At a dose of 300 mg/kg of Phenotropil, washes were only observed for 1 minute.

Olanzapine already at a dose of 1 mg/kg (Table 5.4) significantly reduced the number of visits of animals to the center of the open field by 39% by the total index. Phenotropil at doses of 100, 200 and 300 mg/kg significantly (by 111, 155 and 133% respectively) compared to the control increased the number of visits to the center, that indicates the presence of an anxiolytic effect without a sedative action component in all studied doses of the drug.

The pronounced neuroleptic activity of Phenotropil at different symptoms is combined with components of psychostimulant and anxiolytic action with increased orientation-exploratory behavior at all studied doses by all components without a sedative effect with manifestation of antiparkinsonian activity and it significantly distinguishes the compound from both the typical and the atypical antipsychotics, as well as from stimulating and suppressing drugs. The psychomodulatory and neuromodulatory activity of the compound prevents the development of side effects and aversivity.

### Examples 6:

Study of the effect of Phenotropil at single therapeutic doses (25-300 mg/kg, Compounds 1, 2, 3 and 4) on the components (primary positive - P1, primary negative - N1 and the secondary positive - P2, and the sum of P1 + N1) of the transcallosal stimulation induced potential (TSIP) in rabbits (n = 6) in comparison with Phenotropil (25 - 300 mg/kg), piracetam (300-500 mg/kg) and Pyritinol (50-150 mg/kg) by the standard method [22, 28] for the first time found (Table 6) significant differences in form and content from the two latter. The effects of Phenotropil during the observation period (3:00) were rhythmic. Within the first 30-40 minutes after use of Phenotropil (Compounds 1 and 3), the values of components alternately increased by an average of 35-15 % depending on the dose, and then their levels did not exceed 15%-10% compared with the baseline background values. The results can be described in relation to Phenotropil as bipolarly asymmetrical, consolidated coupling. The effect of the Compound 2 is less pronounced than that of Compounds 1 and 3, but the nature of the effect is commensurate. Compounds 1 and 3 had no differences. The effects of Compound 4 had no significant difference from the saline treated control, being initially in the range of 15-10 % above the background.

The effect of Phenotropil compared with piracetam at the doses tested is more limited, corresponding to psycho-modulatory activity, which is most promising from the therapeutical and prophylactic point of view of correction of cognitive and other disorders. TSIP characterizes not only cognitive processes, but as well any hemispheric interactions. Phenotropil, unlike Pyracetam and other psychostimulants, does not level out and does not erase the nature of asymmetry of the brain, but increases and conjugatively consolidates these relationships, predefined by nature, including the significance of hemispheric asymmetry. As a result of studies in the examples above and obtained in the present, there is every reason to talk about the advantages of modulatory effects of Phenotropil compared with uniquely targeted agents in treatment and prevention of various psychiatric and neurological disorders, not just those identified. It was revealed for the first time that nootropic activity of Phenotropil has absolutely different nature of action than that of Pyracetam. Phenotropil, unlike Pyracetam, is not a psychostimulant in the traditional sense of psychostimulation and it cannot be included in group N06B, where drugs exert only catalytic activity.

Study of the effect of Phenotropil on cerebral blood flow, systemic blood pressure (BP) and the nervous regulation of blood circulation was made on 6 anesthetized cats under general anesthesia (urethane + chloralose). Phenotropil was administered intravenously at a dose of 50 mg/kg with distilled water; the baseline state of each animal prior to drug administration served as control. Cerebral blood flow was recorded in the common carotid artery (CCA) by an electromagnetic meter. The nervous regulation of cerebral circulation was judged by the vasomotor pressor reflex (VPR). The results of the experiment show (Table 6.1) that the drug causes a moderate increase in cerebral blood flow in anesthetized animals. The cerebrovascular effect is accompanied by vascular hypotension with manifestation of oppression of the vasomotor reflex. The biphasic behaviour of SBP is consistent with the pattern of the effect on TSIP under no abnormalities with stabilization of the normotonic state. At intraperitoneal and intragastric administration of Phenotropil (25-300 mg/kg) to white outbred adult rats of both sexes, hypotensive activity (by blood method through the cannula connected to the carotid artery) was manifested one hour after administration at doses of 50-100 mg/kg and was in average 13 % ± 2.85, while with increasing doses this effect disappears. Phenotropil is not in the true sense a hypotensive or hypertensive agent, but its modulatory activity manifests in the tendency to normotonia through a regulatory effect on vasomotor reflex. This effect requires a special study in clinic. The study results suggest that the effect of Phenotropil not only has central nature, but also is reciprocal from the local effect, i.e. from the place of its presence. Such effects of the drug may be particularly useful in unstable states, as well as in vegetovascular dystonia

The stroke condition was simulated in male rats (270-320 g) at a local cerebral hemorrhage, as a hemorrhagic stroke (intracerebral post-traumatic hematoma) by the method of A.N. Makarenko et al (1990) and at cerebral ischemia with distal occlusion of the middle cerebral artery (Chen S.T., Hsu C.Y., Hogan E.L., Maricq H., Balentine J.D. A model of focal ischemic stroke in the rat reproducible extensive cortical infarction // Stroke. - 1989, V. 17, No 4, p.738 - 743) as an ischemic stroke. Phenotropil was injected intraperitoneally at hemorrhagic stroke at 100 mg/kg immediately after surgery, and one hour before and two hours after the surgery (at doses of 100, 200 and 300 mg/kg) at ischemic stroke. Control animals were treated adequately with distilled water.

Phenotropil has exerted a pronounced activity in both hemorrhagic and ischemic stroke in rats (Tables 6.2 and 6.3). Affecting the outcome of stroke, it prevented lethal outcomes (follow up for 14 days) in 100% of cases, providing curative, preventive action, accelerating the process of recovery of neurological deficit in animals compared with control groups of stroke animals and sham operated animals. It prevents development of degradation in the brain lesion foci, and thus the whole brain, restoring and maintaining the plasticity and functionality. This emphasizes again the selectivity of the compound effect depending on the initial state. Consequently, Phenotropil can be useful not only in cerebrovascular but also in other cerebral pathologies and disorders.

### Examples 7:

Phenotropil has a pronounced mnemotropic and nootropic activity. The results of the study of the antiamnestic activity (classical technique in the equipment manufactured by Lafayette Instrument Co, USA) in the retrograde amnesia of the passive avoidance conditional reflex (CRPA) induced by maximal electroshock (MES) and scopolamine in male rats (180-250 g) are shown in Table 7. Nootropic activity of the compound is manifested both in lower and higher doses tested (25-100 mg/kg) significantly eliminating amnesia after a single administration. Taking into account the studies using the open field and cross maze tests, the nootropic action component of the modulatory drug not only may be more pronounced, but is broader in manifestation depending on the type of disorder or in unfamiliar situational conditions. The pronounced nootropic activity of Phenotropil (100, 200, 300 mg/kg) also manifests in rat pups of both sexes 30-35 days of age (Table 7.1) after scopolamine-induced CRPA amnesia.

In studying immature animals, it must be considered that to generate CRPA it is required to apply higher educational incentives (0.6 mA, 8 pulses vs 5 pulses at 0.45 mA in adult rats) with re-training (after 48 hours). Development of amnesia also requires a higher dose of scopolamine (1.3 mg/kg) in rat pups. When selecting "under-trained" rat pups to assess mnemotropic activity, immature animals, which failed to achieve a positive value of the indicator of delta of the latent period (ΔLP = LP2 - LP1), were included into the group. As shown in Table 7.2, with intragastric administration of Phenotropil, its pronounced mnemotropic activity was manifested in rat pups with the initially negative (ΔLP = - 6.8) training tendency. The mnemotropic effect of Phenotropil measured by ΔLP index increased with increasing dose and was +133% at 50 mg/kg, +504% at 100 mg/kg, and +696% at 300 mg/kg versus saline treated control. Phenotropil has not only a psychomodulatory (Ψ-modulatory) activity but also a Ψ-operandmodulatory activity given that the mnemotropic effect is associated with the expression of specific proteins and genes involved in memory, attention and learning.

After a single prophylactic administration of Phenotropil (100 mg/kg) and a single administration during scopolamine-induced deprivation of the learning ability in adult male rats (Table 7.3), the compound exhibits high therapeutic efficacy on indicators of the long-term memory, attention and learning, indicating that it conjugatively consolidates them as an agent preventing the disorders and having therapeutic application when used during the disorders. These findings are of great importance. In clinic, medications have to be prescribed more often already in the midst of disorders.

Studying the mechanisms of action of the preventive and curative nootropic effect of Phenotropil (100 mg/kg) with single and subchronic administration (for 7 days) in terms of receptor binding to dopamine (D1, D2, D3 in the striatum), serotonin (5 -HT2 - frontal cortex) glutamate (NMDA in the hippocampus), the GABAA benzodiazepine (BDZ - cortex) and acetylcholine (nACh - cortex) receptors was performed on membrane preparations isolated from various competent rat brain structures (striatum, frontal cortex, hippocampus). The methods are well known and described [20, 26, 33]. Baseline studies in normal state and during the anticholinergic scopolamine induced CRPA amnesia served as the comparison groups (Table 7.4 and 7.5).

It is believed that the nootropic effect is directly related to activation of NMDA receptors. However, as shown, the scopolamine-induced amnesia itself leads to the same and significantly more than pronounced effect compared with the control (Table 7.4). Phenotropil has a coupling effect on all the indices studied. The intensity of its trigger effect and the effect direction is different in different experimental models. The results obtained show the commensurability of Phenotropil action on molecular mechanisms depending on the initial condition of an organism and during the treatment. Apparently its molecular mechanisms of action are much wider and diversifiedand and even diametrally opposite dpending on a type and extent of a certain disorder of neurological or psychic profile.

These study results also demonstrate that approximating the mechanisms of action of modulatory agents of norm as well as a proactive agent acting only on a certain disorder not only may not be unjustified, but also capable of misleading. This is confirmed by the obtained results of the research. One also must take into account the fact that a variety of molecular mechanisms of action is not a guarantee of proof of modulatory activity, if this variety is not commensurate depending on the type of norm and the type of a particular disorder. Before the discovery of the compound with the modulatory activity, it was thought that the mechanisms of action identified in the norm correspond to those under pathology conditions and it really complied with drugs with either stimulant or suppression effects, but it is not suitable for evaluation of a commensurate effect, since the nature of this effect may vary depending both on the norm and on the type of the norm disorder

### Examples 8:

Phenotropil in doses of 50 and 100 mg/kg increased indicators of orientation and exploratory behavior by 89.0 % by the vertical component and by 240.0 % by the horizontal component compared with the control group of animals (Table 8) in treadmill studies in mice with automatic measurement of vertical and horizontal components of motor activity per unit of time and increased in these doses the psychostimulant activity of Benzedrine (3 mg/kg) and after single administration of 300 mg/kg significantly counteracted the effects of Benzedrine, reducing the severity of the psychostimulant effect of the latter by 14.8 % on the vertical component of exploratory behavior and approximately by 42.9% on the horizontal component normalizing orientation exploratory behavior of mice. In regard to influencing the effects of Benzedrine, Phenotropil also can provide, depending on the dose, the diametrically opposite activity when in combination with psychostimulants. This fact must be taken into account in combined use of various pharmacological agents.

Stimulatory effect on the duration of swimming in cold (2°C) and normal (25°C) water (Table 8.1-8.1.1) of Phenotropil (25 and 50 mg/kg) compared with the control (distilled water) and Benzedrine (2,5 mg/kg) was studied in white outbred male mice (18-26 g) compared to control (distilled water) when intraperitoneally administered, 60 min after the administration and with a course administration for 4 weeks administration on day 28 (Table 8.1.2). Phenotropil markedly increased the duration of swimming and the adaptive regulation of mice after a single dose and does not lose this ability after the course administration. By the effect on the physical endurance to low ambient temperatures, Phenotropil is superior to Benzedrine by almost three times (2.5 mg/kg). A single administration at low temperatures (Table 8.1) of Phenotropil increases the duration of swimming in mice by 70.7 % and by 85 % at doses of 25 and 50 mg/kg, respectively, compared with the control group, while the size of the effect of Benzedrine was only 30% compared with the control. At normal temperature of water, the effect of Benzedrine was not modified and stayed at 34 %. When swimming in water with normal temperature (Table 8.1.1), Phenotropil at a dose of 25 mg/kg was in fact not different from the effect of Benzedrine, and at 50 mg/kg it increased the duration of swimming by 75% and was superior twofold to the effect of Benzedrine.

In case of course treatment (Table 8.1.2), the effect of Phenotropil not only does not disappear, but also is more pronounced than after single dose use, both in the cold and in a normal temperature environment, being 109-108% compared with the control group of animals. These findings indicate that, unlike psychostimulants, psychomodulators not only do not deplete the reserve capacity of the body in case of course treatment, but rather increase them. The advantage of psychomodulator Phenotropil is obvious both with the single dose and the course use, by the effect on physical performance and stability of the body, both in normal and more adverse temperature conditions.

The tranquilizing effect of Phenotropil (300 mg/kg) was studied by the method of conflict situation and conflict of two reflexes (Vogel, 1971; Sanger et al., 1991; File, 1995; Molodavkin, Voronina, 1995), associated in this case with the desire to satisfy the motivation (drinking) and application of punishment (electric shock, received after each attempt to satisfy the motivation). In the created conflict situation, the animal is experiencing a strong negative stress. Tranquilizers reduce the level of anxiety and psychoemotional stress. Phenotropil at the investigated dose reduced anxiety and emotional stress in the conflict situation by 291.0 % vs the control, increasing the time between the first and second intake of water and reducing the amount of intakes for three minutes of observations by 74.4 % (Table 8.2).

Specific effects of anticonvulsant activity in mature animals are presented in Table 8.3. Chemical convulsive agents (bicuculline, corazol, thiosemicarbazide, and picrotoxin) and the maximum electroconvulsive shock (ECS) model in the experiment the different types of seizures with their various implementation mechanisms of pathological conditions. The anticonvulsant activity of Phenotropil after a single dose for all the investigated models manifests in a pronounced positive effect on prevention of mortality in the studied doses of 100, 300, 400, 600 mg/kg. In case of bicuculline-induced seizures, mortality in the control group was 70.0 %. Phenotropil (100, 300, 600 mg/kg) was effective in 100 % of cases. The metrazole-induced mortality in the control was 60.0 % and the positive effect of Phenotropil for mortality vs control was 45-35-0%, depending on the dose, respectively. The thiosemicarbazide-induced mortality was 90.0 % in the control group, while when treated with Phenotropil (100, 300, 600 mg/kg), respectively: 60 - 0 - 0 %. In the control treated with picrotoxin, the mortality in mice was 50 %, while the use of Phenotropil 300 mg/kg completely prevented the mortality. In case of MES, mortality in the control group was 100.0 %, while in the Phenotropil groups (100, 300, 400 mg/kg) it was 20-0-0%, respectively. Thus the anticonvulsant activity also increased significantly, including as well at a dose 100.0 mg/kg, which indicates the possibility of using lower doses in order to prevent convulsive episodes, and for correction of convulsive manifestations of epilepsy. The anticonvulsant effect of Phenotropil prevents the development of seizures and affects the outcome of convulsive states, preventing mortality in all applied models of seizures in animals. The mechanisms of action of anticonvulsant activity of Phenotropil are commensurate, various and depend on the initial state.

Phenotropil in the studied doses (50, 100 and 300 mg/kg) has a pronounced antihypoxic activity, significantly increasing the survival time of mice in the pressure chamber (Table 8.4). The effect increased with increasing dose, increasing the survival time compared with the control group by 65.0, 147.8 and 591.0 %, respectively. Fepiron (25-50 mg/kg), the predecessor of Phenotropil, did not affect the survival of the animals, and at 100 mg/kg it increased the survival time by 47.8 %. Piracetam 600 mg/kg shortened the survival time in comparison with a control group of animals by 35.0 %, and only at 900-2000 mg/kg increased the survival time by 78.0 %, which is significantly inferior to the effects of Phenotropil both in terms of effective doses and therapeutic severity

The effect of Phenotropil on pain sensitivity in mice (Table 8.5) by the method of hot plate test describes the analgesic effect of the compound and is manifested at the tested doses (50, 100 and 300 mg/kg) 30, 60 and 120 minutes after intraperitoneal administration in the reduction of pain sensitivity by: 14.3 - 8.3 - 10.8% at a dose of 50 mg/kg, 12.4 - 12.5 - 31.5% at a dose of 100.0 mg/kg, and 71.4 - 61.5 - 100 0% at a dose of 300.0 mg/kg. The analgesic effect is present at all studied doses and increases with increasing dose.

### Examples 9:

Given that hypoxia is the cause of the development of various pathological conditions in the postnatal and early childhood age, results in delayed development, it was found in studies, that rats of postnatal (7-day) age of both sexes are more resistant to convulsive agents and hypoxia than mature animals. The results of the study are presented in Tables 9 (hypobaric hypoxia) and 9.1 (hypobary with hypercapnia). The drugs were administered 30 minutes prior to exposure.

In rats of postnatal age at an altitude of 11,000 m, no death was observed within 2 hours of exposure. Under the conditions of hypobaric hypoxia 100% of the control animals at an altitude of 7,000 m body twitching and strong tremor were observed (Table 9). After treatment with Phenotropil (50 and 100 mg/kg), tremor was observed only at 10,000 m. During staying at the altitude of 11,000 meters (for 2 hours) the control rat pups displayed periodic tremor (11 episodes), whereas in animals treated with Phenotropil, tremor was observed only once (only at ascension). When observing the behavior of rat pups for 2 hours, it was noted that 50% of the control group animals "freeze" (stay motionless in the chamber). The rat pups treated with Phenotropil in doses of 50 and 100 mg/kg, stayed moving over the entire exposure time. The rat pups treated with Phenotropil 300 mg/kg (Table 9.1) displayed tremor only at an altitude of 11 thousand meters, and at an altitude of 12 thousand meters the tremor disappeared. Throughout the experiment, the control rats displayed periodic tremor (8-9 episodes), whereas in animals treated with Phenotropil the tremor was observed only once (only at ascension). After the animals were removed from the pressure chamber, the rat pups treated with Phenotropil were significantly more active than the control animals, in all cases. These studies and features of animals of postnatal age indicate that in the case of mild antihypoxic effect of a particular drug in adults, their effects in pediatric practice may never emerge. Phenotropil has antihypoxic activity also in animals of postnatal age.

### Examples 10:

The antidepressant effect of Phenotropil was studied in adult rats (185-218 g) and rat pups of 28-35 days of age (85-110 g). The test drugs were injected intraperitoneally in the first case and intragastrically in the second case 40 min before the tests. The methods of behavioral despair during forced swimming (Porsolt et al., 1978) and forced swimming with freely rotating wheels (Nomura et al., 1982) were used.

Phenotropil has a statistically significant and pronounced antidepressant effect in both doses tested (100 and 200 mg/kg), as indicated by the significant decrease in the duration of immobilizing under its effect by -79.8 % and -72.2 % in comparison with the control (Table 10 and 10.1). In the study of depression by Porsolt, no significant differences between the effective doses were found (Table 10), while according to the method of Nomura this difference is statistically significant. At a dose of 100 mg/kg, the efficacy of Phenotropil as compared to the control, was +59.0 %. At a dose of 200 mg/kg it was +30.0 % in comparison with the control group (Table 10.1). When influenced by Phenotropil, the animals did not refuse to fight and continued attempting to get out of the aversive situation. They considerably less exhibit the behavioral despair. The effect of Phenotropil was investigated by the method of Nomura also in mature outbred rats (185-218 g). Phenotropil in doses of 10, 25, 50 and 100 mg/kg (Table 10.2) exhibited an antidepressant effect, increasing insistence to get out of the water by 15.4 %, 67 %, 104.2 % and 142.4 %, respectively. The antidepressant activity in adult animals manifests more pronouncedly than in immature ones and at 100 mg/kg it is superior twofold.

### Examples 11:

The antitoxic, sobering and anticraving, activity, as well as the effect of Phenotropil in abstinence was studied on white outbred male rats (weighing 180-220 g) and male mice (weighing 18 -24 g) in chronic alcoholism. It was found that after treatment with Phenotropil at doses of 25.0-200 mg/kg, the toxic effect of ethanol in mice reduced tenfold in terms of "lateral position" indicator (narcosis condition) and of lethal dose, and in case of administration of Phenotropil after ethanol, Phenotropil had awakening (sobering) action. Alcoholized rats after four months of alcoholism and treatment with Phenotropilom voluntarily give up intake of alcohol with free choice of water and alcohol (Table 11). During abstinence sate in alcoholics rats (7-month alcoholism), the daily alcohol consumption during the subsequent free choice not only restores, but significantly increases in the control group, while administration of Phenotropil leads to voluntary avoidance of alcohol (Table 11.1) in case of free choice.

Phenotropil not only possesses antitoxic effect in alcohol poisoning or prevents alcohol poisoning, but also has a marked therapeutic effect, by curing alcohol dependence in rats, showing pronounced anticraving activity, characterized by voluntary refusal of animals during free choice. The effects of Phenotropil as both an antitoxic agent and as a therapy for alcohol dependence and withdrawal are manifested starting with a single dose and increase with a course use. It appears that Phenotropil by virtue of its modulatory properties can be useful not only in alcohol dependence, but also in the treatment of other narcotic dependencies, as well as various forms of substance abuse, based on modulatory effect on neurotransmitter systems described in the examples above.

### Examples 12:

To identify and assess the anti-inflammatory action of Phenotropil, a standard technique was used; the carrageenan-induced paw edema in rats (Winter C. et al, 1962). Phenotropil at a dose of 100 mg/kg was administered intragastrically 60 minutes before carrageenan. Control animals received distilled water. Additionally, the topical application by method of formalin induced edema was investigated (subplantar administration of 0.1 ml of 2 % formalin solution). Phenotropil for topical use was prepared (1:1) on the standard ointment base carrier at 100 mg/kg of body weight for each animal (150-220 g). The preparation was rubbed in the leg and a gauze patch was applied for 2 hours and 1 hour before the induction of inflammation.

In the first test, Phenotropil shortened the inflammation by 52.3% compared with the control, and in the second test - by 64.5% (Table 12). No significant differences were found in the latter case between using the product 2 hours and 1 hour before the induction of the inflammatory agent. The revealed inflammatory activity of Phenotropil was even more pronounced when used topically. It was established that Phenotropil has anti-inflammatory activity both when administered into the body, and when used topically.

The presence of the proper anti-inflammatory effect in Phenotropil formed the basis for investigation of the effect of the drug on more complex pathological agents. The effect of different concentrations of Phenotropil on the mycobacteria (MTB) culture growth *in vitro* according to research standards (test culture - highly virulent laboratory strain H37Rv) was studied. A wide range of drug concentrations was prepared: 1 series - 100, 50, 10 µg/ml, 2 series - from 10 to 0.31 µg/ml, and 3 series - from 2 to 0.031 µg/ml. To prepare the matrix solution, the sterile distilled water was used, and the Shkolnikova medium was used to prepare the said concentrations. To assess the growth and morphological properties of the MTB culture, suspended mycobacteria at appropriate concentrations of Phenotropil solution were inoculated on the Lowenstein-Jensen medium and Shkolnikova medium (4 tubes for each point). After 2 weeks, the presence of growth was determined visually in the Shkolnikova medium and smears from the sediment were prepared. The smears were fixed and stained according to Ziehl-Neelsen method. The number of mycobacteria in smears was counted and, based on the results obtained, the concentration of the drug at which the inhibition of the growth was observed was determined. The number of the MTB colonies and their morphological characteristics were evaluated on the Lowenstein-Jensen medium after four weeks from the time of inoculation. Registration of the results was also carried out four weeks after inoculation.

The results of the study are presented in Table 12.1. In all tubes with different Phenotropil concentrations in Shkolnikova medium, the growth of the MTB culture was visually observed, as well as in the control tubes without Phenotropil. The microscopy of smears established the presence of mycobacteria in the form of characteristic bands in all fields of view in each of the preparations. In the Lowenstein-Jensen medium, the growth of colonies typical for the MTB was determined in all tubes, but their number was varying. In the control and in some test tubes, the growth of colonies was abundant, in some places confluent and poorly accountable. Such growth of colonies was designated as "contiguous growth." Less than 100 colonies were observed at Phenotropil concentrations ranging from 0.25 to 5.0 µg/ml. The lowest number of MTB colonies were observed at concentrations in the range from 0.5 to 1.0 µg/ml. No differences were found at lower and higher dilutions. Thus, Phenotropil in the *in vitro* studies in a concentration range from 0.5 to 1.0 µg/ml showed statistically significant bacteriostatic activity on the growth of colonies of mycobacteria.

### Examples 13:

The effect of intraperitoneal administration of Phenotropil at a dose of 100 mg/kg on pro- and antioxidant systems was studied in male Wistar and August rats, and white outbred rats (weighing 180-220 g, 10 rats per group) having different genetic resistance to the negative stress (Ns) that is the ds and Ds conditions. Control animals received distilled water. Acute and systemic exposure to Ns was performed using the method of N.A. Bondarenko (N.A. Bondarenko et al, 1999) with single, triple (once a day) and seven-time (for seven days) exposure to Ns of psychoemotional nature. Plasma levels of TBA (thiobarbituric acid) - MDA (malondialdehyde) were determined by spectrophotometry. NSP32 levels were determined by Western blot analysis (ng/mg of total protein). The effect of Phenotropil on plasma electrolyte balance in chronic (for 4 months intraperitoneal) administration was evaluated in outbred male white mice (weighing 20-26 g). The results are presented in Tables 13-13.8.

Baseline studies have shown (Table 13) that the MDA levels in genetically high and low genetically ds-resistant rats are different, which is consistent with the known data. The levels also have differences with the outbred animals (control). Stress-limiting system in the August rats is almost by 43.0 % lower than in the Wistar rats, as indicated by the level of TBA products as by MDA, but studies show that the reserve of these strains is as varied and the compound exhibits a selective activity within the reserve capacity of each strain of the same species. Ns also has a different effect on the MDA level in plasma of different strains of the same species.

In highly resistant animals, when exposed to Ns, the MDA concentration in blood plasma was significantly decreased by 12.0 %, compared with outbred animals, while in the low resistant animals it increased by 186.0 % compared with their baseline level in normal conditions (Table 13.1-13.2.). The acute exposure to Ns exerts a multidirectional effect in different strains of the same species and is expressed in either positive or negative values, both by sign and by intensity of the body's response by the studied parameters. The state of relationship between prooxidant and antioxidant systems in different strains of animals is not equisignificant. Consequently, the low resistance strain (August) is more reactive and more sensitive to the Ns and the response cost in low resistant animals is much higher and has the opposite sign of expression in terms of MDA.

Phenotropil at single injection into the body, without modeling stress-negative reactivity, is itself a Ts-factor, translating the functioning of the body to a higher level, both in the high- and the low-resistant strains of animals and has a commensurate effect that is reflected in increased activation of stress-limiting systems in Wistar by 66.0%, and in August by 190.0%, which is 2.9 times higher than in the Wistar strain, providing prooxidant action, which indicates the commensurability of effect of Phenotropil in genetically different strains.

During exposure to Ns, Phenotropil reduced the level of MDA by 6.0 % compared with the baseline control without stress in the Wistar strain, while in August strain, in contrast, its effect manifests in pronounced increase of plasma MDA concentration by 2.2 times compared with the baseline control and even by 17.0% higher than with Phenotropil treatment without exposure to Ns. Modulatory activity of the drug is displayed not only in Tsf-effects, but also commensurately, with diametrically opposite sign, when exposed to negative stress (Ns) in different types of genetic resistance. The excessively low baseline level of MDA probably indicates deficiency of limited systems, therefore the use of drugs aggravating the condition may have the negative rather than the positive effect.

Of particular interest are the results of a study on "intra-species" activity of LPO (lipid peroxidation) in outbred (OB) animals (Table 13.3) and other strains under 3-fold stress-negative tension. The LPO indicator, determined from the baseline level of TBA-MDA, is 1.0 (0.99) in OB. Compared to the Kt of outbred animals, the ds increased the Kt index in Wistar by 10.0%, numerically to 1.1. In the August strain, in the Ns-state, Kt was 2.1 compared to the OB. In the conditions of Ns-state, the species Kt = 1.6. After treatment with Phenotropil, Kt in both strains of animals compared to OB was 2.1 and thus the intra-species Kt was the same, which indicates a very interesting fact of effect of Phenotropil within the species reserve of stability which in the acute experimental Ns-state during treatment by the drug increased by 12.5 %, which is almost similar to the Wistar exposed to Ns, but with the opposite sign (-12.0%). Considering it accidental is impossible due to the statistically verified consistency. Phenotropil commensurately affects different strains of animals, not only within genetically predetermined conditionally phenotypic homeostasis, but it also spreads the frames of the species and phenotypic ontogenesis on the specific gene level.

However, the studies of the course (for 4 weeks) administration of Phenotropil (evaluation was carried out one hour after drug administration on days 1, 3, 7, 14, 21, and 28) showed that the drug does not exert any appreciable effect in normal conditions on the content of the plasma electrolyte balance in animals in terms of Ca, K and Na levels (Table 13.5), thus confirming the absence of adverse effects of Phenotropil on balance of pro- and antioxidant systems of animals, without causing Na⁺/K⁺-Ca²⁺-ATPase and not leading to energy shortages, as well as to electrolyte imbalance by hyper- or hypotype. Therefore, the consolidating modulatory effect of the drug in the presence of the pronounced training stress factor influence is extended by a multilevel promotemodulatory type of action with direct, reverse and cross-dependencies, including the cellular regulation with consolidation of coupling the critical systems and increased regulatory control over their condition.

The effect of Phenotropil in the low and high resistant animals in terms of the heat shock protein levels demonstrate (table 13.6-13.8) the training stress factor (Tsf) effect in brain structures with the pronounced operandmodulatory activity in different strains of animals as compared to outbred (OB). Phenotropil in the tested dose decreases gene expression in Wistar rats by 17.7 % and increases the expression by 16.9 % in the August strain, i.e. has opposite effects depending on the baseline genetic condition. Under exposure to the Ns-tension, application of Phenotropil increases the gene expression in terms of NSP32 respectively by 82% and 99 % after a single dose. Stress-tension as a negative influencing factor, when without Phenotropil, reduces NSP32 by 9.1% in Wistar and increases by 95% in August rats. In the OB group, the training stress-factor effect of a single Phenotropil dose was equivalent to the single stress-emotional impact of the methodic influence and was +38.5 % compared with the control. The effect of Phenotropil and stress-negative factor increased the Kt value by 86.0% compared to the no-treatment control.

Transition of distress into destress as well as pronounced adaptogenic modulatory activity of Phenotropil is clearly demonstrated by study results in Table 13.8, compared with the results summarized in table 13.4, indicating a high biological activity of the compound, as now can be judged based on of the results obtained. The Ds, just like the ds, develops in different strains of the same species by its own scenario and not generically, and therefore the concept of "general adaptation syndrome" cannot have a single scenario depending on a genetic predisposition; it is much more diverse in its implementation even in different subtypes of one species, not to mention different subtypes of the same disorder and disease. In the outbred group and the Wistar group, the level of TBA products compared with the control was high and reached, respectively, +270 % and +193.7%, while the transmission factor is more than three times greater than the control intra-strain one. Distress in these strains of animals develops in hyperfunctional direction, and, in the August strain, the Ds-state switches to hypofunctional and is (-30.5%) compared with the self-control group. After treatment with Phenotropil, the modulatory state of the body restored, and the modulatory activity of the compound manifests by diametrically opposite action with a pronounced adaptive-modulatory effect by diametrically opposite scenarios, respectively (-73.0, - 65.6 and +60.0%) and a better Kt indicator, than both in Wistar and August control, restoring Kt to the completely normal value of outbred rats.

The obtained results allow to characterize the effect of Phenotropil not only as that of a training stress factor modulator, but also as that of a modulator commensurately inducing and commensurately consolidating stress-limited systems at the level of cellular metabolism of pro-and antioxidant systems, as well as a cellular operandmodulator at the level of gene expression in terms of the heat shock protein. Consequently, the promote modulatory type of activity in this compound extends also onto the intracellular level as operandmodulatory as well as membrane-stabilizing and cito-modulatory with pronounced transmission. The effects of Phenotropil on metabolism have no stimulating or suppressing nature inherent in the two traditional types of drugs (stimulants and pressors or depressors), which action is based on just one particular leading effect, but they do have rather modulatory nature, which is expressed both as a pro- and antioxidant activity, while antioxidant activity and effect on heat shock proteins are also modulatory depending on the starting condition and genetic predisposition. In case of pronounced effect depending on the starting condition on the pro- and antioxidant systems, as well as on the heat shock proteins HSP32 while maintaining the electrolyte balance parameters and at chronic administration, uniqueness of the commensurately consolidating effect of Phenotropil is evident from the results of the study.

### Examples 14:

Study of the pharmacokinetic parameters of Phenotropil distribution (100 mg/kg - 1% aqueous solution orally) in outbred white rats of both sexes weighing 220-260 g (Table 14) showed no significant changes in organ homogenates (measured by GLC method) compared to that previously known in Phenotropil that indicates the ability of the related impurities to compete for binding sites in organs and tissues, but it does not affect the processes of absorption, distribution and excretion of the active compound. The level of the compound and distribution in the kidney and liver at various time intervals is close to the plasma level, while in the heart and in particular in the brain its level is significantly lower. The degree of tropism of the compound in various organs by quantitative content varies and is arranged in descending order as follows: liver, kidneys, heart, and brain. Rapid penetration into the brain confirms the good kinetic ability to penetrate through the blood-brain barrier. In 15 minutes, its presence is detected in the brain, heart, liver and kidneys. In the brain, liver and kidneys, the maximum concentration is achieved in an hour, and 6 hours after oral administration the compound is not detectable any more. In the heart, the maximum concentration is detected in 1 hour 50 minutes and it is eliminated completely after 8 hours. The maximum concentration of Phenotropil in the myocardium is detected later than in the brain, but it is eliminated from the myocardium also two hours later. In the liver, the maximum concentration is found after an hour and then the concentration is gradually decreasing. One hour after administration, the concentration in the kidneys reaches the maximum, with a gradual decrease further on.

In the male Wistar rats weighing 195-250 g, pronounced activity of Phenotropil (25, 50, 100, 300 mg/kg) with intraperitoneal injection was revealed by HSP70 expression in the myocardium 60 min after administration in normal conditions and after stress-negative exposure by the method of N.A. Bondarenko (N.A. Bondarenko et al, 1999) compared with the control groups of animals (distilled water). Determination of NSP70 (ng/mg of total protein) was studied in myocardial preparations by Western blot analysis; the results are presented in Table 14.1.

The NSP70 levels in the myocardium in control tests without infuence of the negative stress were 0.11 ± 0.007 ng/g (Table 14.1). The intraperitoneal administration of Phenotropil in doses of 25, 50, 100 and 300 mg/kg increased the content of NSP70 in the heart by 65, 82, 73 and 273%, respectively. The stress negative state in the control group of animals resulted in an increase in NSP70 by 164 % (0.29 ± 0.007 ng/g of total protein) compared with the baseline control. After treatment with Phenotropil in doses of 25, 50, 100 and 300 mg/kg, in negative stress state the NSP70 level increased in the myocardium compared with the control group by 38, 41.4, 62 and 89.7 %, respectively. Pathological and microstructural studies of myocardium revealed no destructive effect of Phenotropil on myocardium, which was clearly pronounced in rats in the control group in the case of negative stress without Phenotropil.

Phenotropil in low and high single doses under normal conditions has pronounced Tsf-activity, and in the ds-conditions provides greater protection to the body at the cellular and organ level. Given the role of cytoprotective proteins HSP70 and their prevalence in the tissues of various organs, we can talk about the role of Phenotropil in the organization of antiapoptotic mechanisms and expression of the mobilization gene not only of the brain cells, but also in the myocardium, which indicates the possibility of absence of tissue specificity of the compound. The anti-inflammatory activity of Phenotropil when applied topically and some effect on colony growth of mycobacteria indicate an extremely wide range of its possible effects. Phenotropil may find application, in view of the results, not only in neurology and psychiatry, but also in cardiology, as well as in other fields, since it is kinetically allocated to different organs and tissues, and is able to exert local effects.

### Example 15:

The adaptogenic effect of Phenotropil, its enantiomers and agents of different classes and groups was studied in a model of 14-day immobilization (from 5 pm to 10 am) in antiorthostatic position (antiorthostatic hypokinesia -45°) in male Wistar rats (187-260 g) in special box cells for each animal. From 10 am to 5 pm, the animals were in normal conditions and had free access to food and water as at normal maintenance. In preliminary studies it was found that the waiting Ns is not less aggressive by its effects than the monotonous effect of despair. One must take into account the fact that rats are species with active behavior at night. Phenotropil in doses of 100, 50, 25 mg/kg, and is isomers at doses of 25 mg/kg with the comparison drugs (Seduxen - 2 mg/kg, piracetam -200, 400, 600 mg/kg, phenibut - 100 and 50 mg/kg, isomers of phenibut - 50 mg/kg, Fepiron - 200, 100 and 50 mg/kg, isomers of Fepiron - 200 mg/kg) were administered once a day intraperitoneally with saline solution in the morning. The control group of animals was injected with saline. The second control group all this time was under normal conditions in a separate room and did not witness the experiment. At the end of the study, the thymus (T), spleen (S), and adrenal glands (A) were collected, weighed to determine their mass per 100 grams of baseline body weight of each animal and mass ratio of the spleen to the thymus and the adrenal glands were calculated, as well as the ratio of the thymus to the adrenals in terms of Kt. In the first case, the transmission rate of relationship between the immune and hematopoietic systems were obtained, in the second - between the immune and hormonal systems, and in the third - the immune-hormonal ratio were obtained. The results are presented in Table 15.

Considering that in the above-described study results, the ability of the enantiomers to form conformations is first established, the presence of (+) and (-) signs in the table 15 after the Phenotropil names characterizes only their initial state prior to administration.

In the control no-treatment group, the ratios (Kt) for S/T, S/A and T/A, respectively, were 1.1, 9.9 and 7.7. Destress changed these relations, respectively, to: 6.2 - 7.3 - 2.5. Ds leads not only to the exhaustion of stress-limiting systems, but also to a disalignment, disorganization of their relationship. All study drugs except in groups of animals treated with Phenotropil and its enantiomers did not have a consolidating effect and exacerbate in varying degrees instability of humoral systems parameters with exhaustion of the immune and endocrine segments with the irrational use of immune system by the exhaustion type of the anti-inflammatory orientation at insufficiency of the hormonal level at a certain stage due to increasing load on the spleen compartment, which led to a significant increase in the S/T balance, decrease in the S/A balance and tendency to knockout functioning of the T/A ratio. Phenotropil and both of its optically active isomers in the tested doses not just prevented the tendency to knockout exhaustion of the hormonal and immune segments, but did not allow an overtension of hematopoietic, immune and hormonal systems, keeping their plastic consolidaton and setting them to recovery of training stress state. Effects of Phenotropil enantiomers did not differ in the investigated doses, but were inferior in efficacy to the 50 mg/kg dose of the racemic Phenotropil compound, as well as markedly inferior to other doses of the racemic compound by relation to the immune - endocrine systems. Phenotropil and its enantiomers in this ratio, both in the test compound, and in monotherapy with enantiomers also retained the detected mathematical balance of the hematopoietic, immune and hormonal status indicators, tending to the indicators of normal ratios. Phenotropil is highly effective at chronic depression of the stress-limited systems state or at change in their balance due to age peculiarities, with proper selection of appropriate doses of the drug. Enantiomers of Phenotropil have a pronounced adaptogenic biological activity and likely by modulatory type, showing a trigger transmission with the commensurate coupling of functional systems.
Separately, the antiulcerogenic action of Phenotropil was investigated by this method (Table 15.1). The drugs were used as therapeutic agents after 14 years of daily exposure. The antiulcer activity of Phenotropil under Ds-conditions at investigated doses is, compared with the control (5 % glucose), and depending on the dose (25, 50 and 100 mg/kg): -45, -63, and -80%, respectively. The antiulcerogenic activity of Phenotropil is manifested by reparative and regenerative effects in case of the stomach lesions under the negative stress conditions, showing a relatively high therapeutic efficacy.

The studies results also show for the first time, that in the case of a pronounced destress (Ds), neither phenibut with its enantiomers nor Fepiron with its enantiomers, nor Piracetam nor Seduxen are not able to provide consolidated optimum plasticity of the stress-limited systems and it not only changes the understanding of adaptogenic action of nootropics, but also questions its presence in these drugs under the Ds-conditions. In fact, all tested agents except Phenotropil and its enantiomers demonstrated markedly worse results than those with the use of saline in the experimental control group, exacerbating the situation of the stress disorganization by 2-7 times by different referential indicators. Previously it was thought by the authors of the present study that phenibut was most active in terms of adaptogenic action, but these findings were based on a set of indicators of functional systems under the distress conditions, as firstly the very concept of destress did not exist, and secondly, no drugs that provided adaption by modulatory type of action, i.e., having the adaptive modulatory activity and pronounced training-stress factor activity with the commensurate effect, besides possessing both promoutmodulatory and unimodulatory activity. It was previously believed that distress immediately transits into the direction of the out state (according to the classical methodology of H. Selye), but it is far from true, which was quite clearly demonstrated by the results of the present study.

It was shown that modulators differ significantly by the adaptogenic activity from both anxiolytics, and from the nootropics properly, as well as from anxiolytics with the nootropic action component. The obtained results of the study indicate that presence of previously known effects for provision of acute adaptation and anti-destressory (Ds) action by adaptogenic-modulatory type is insufficient. Piracetam, phenibut and its enantiomers, Fepiron and its enantiomers having, as known, different spectra of neurotropic and psychotropic activity with the presence of a nootropic effect in doses determined for them, deteriorate the general functional status under Ds conditions by the ratio of the hematopoietic segment to the immune and the hormonal segments and by the ratio of the immune to the hormonal segment, despite the fact that some of them relate to the set of stimulants, while the others to the set of tranquilizers with a nootropic component of action. Seduxen, as distinct from nootropics, was even more effective, but its unidirectional anxiolytic activity exacerbates the Ds. The ratio of the immune and hormonal segments also has a positive status only after treatment with Phenotropil and its enantiomers. Probably for provision of a consolidated balance of coupling between direct and inverse, cross interrelations, just presence of neurotrophic activity of any orientation, except for the modulatory one, including psychomodulation, is not sufficient. Phenotropil also has a pronounced antiulcerogenic activity under destress conditions.

Consequently, psychostimulants and depressors or pressors, even those having nootropic component of action are not able to provide a long-term adaptation, as well as to translate the short-term into long-term one. Their effects can be characterized by fermative type of action. Thereby, it is necessary to investigate the adaptogenic effect also during the acute adaptation period for different strains of the same species. Imbalance of the ratios of all functional systems at the cellular, tissue and organ levels requires the presence not only of neuromodulatory, but also the psychomodulatory, incretomodulatory and immunomodulatory, as well as, and in all probability - the operandmodulatory activity as both the ds and Ds states can not be favorably or negatively resolved without involvement of gene expression. Also for the first time it was shown that the optically active enantiomers of Phenotropil have a pronounced biological activity of the modulatory action type in the Ds condition, and their combined presence does not deteriorate, but improves the studied parameters. In addition, this balance (R: S) between Phenotropil enantiomers is more effective in their modulatory activity than that of its individual enantiomers. Optically pure compounds are susceptible to racemization, therefore their studying and determining their reliable properties and characteristics require a special procedure to stabilize the compounds, which is much more complicated than that for the racemate.

Based upon the ratios of the investigated parameters, Phenotropil has modulatory activity both on the neurohumoral levels and on the level of higher mental functions, as this model includes elements not only of acute mental situation stress, but also of the mental pressure - stress-negative waiting state where nootropics and the tranquilizer were not effective. The promoutmodulatory type of Phenotropil activity on the mental, neuronal, immune, hormonal, hematopoietic and probably genetic levels provides a positive outcome in chronic disease state, not only preventing the development of a negative state, but also preventing exhaustion by consolidating neurohumoral stress-limited systems and higher mental functions, unlike actual nootropics and drugs containing a nootropic component of action.

Taking into account that also actual nootropics show adaptogenic and nootropic effects only at lower doses than for their proper leading activity, there is reason to say that they are not nootropics as such, but rather their nootropic effect is only one of components. Efficacy of the promoutmodulatory activity of Phenotropil is dose-dependent.

In view of these results, we can conclude that judging about the presence of adaptogenic activity in the absence of correlation between components of the stress-limited systems is impossible, even in the presence of aggregate set of indicators, and the lack of modulatory activity of the promoutmodulatory type in the drug is not able to fully ensure the adaptogenic effect under the Ds conditions. Phenotropil and its enantiomers possess an adaptogenic activity of the promoutmodulatory type of action, including different levels and from different levels of the commensurate effect with their consolidated coupling. It was found that the leading activity of Phenotropil in this case is modulatory with commensurate effect, which distinguishes it from drugs of only stimulating or only suppressing type of action, including those that are able to exercise a stimulating effect only at low doses (phenibut, fepiron, seduxen). All the drugs, including divergents except Phenotropil and its enantiomers have not formantive but fermative nature of the action, and therefore of the organization of plasticity of functional systems and their relations, which ultimately leads to desorganization and distortion of balance ratios.

### Example 16:

Taking into account that Phenotropil chemically is a derivative of diapyrrolidin - diapyrrolidon and a compound related to fepiron, as well as has in its structure formal similarities with "racetam", an achiral derivative of pyrrolidine - pyrrolidon (Piracetam), comparative studies of these drugs on adult outbred white male mice weighing 18-24 g (20 mice per group) were carried out by the method of electric extremities pain stimulation in single or pairwise grouped animals on a conductive ground. In the test studies, the reaction thresholds (in volts) of each animal were determined, and a day later - in pairwise grouped animals, placed on a conductive ground. The following parameters were registered: the sensitivity threshold without sound manifestation (freezing - the threshold of attention) TA, threshold of the first cheep (threshold of emotional response) TFC and the threshold of aggression (fight) directed towards each other (TF).

The drugs (Phenotropil and Fepiron in doses 25, 50 and 100 mg/kg, piracetam in doses of 100, 200 and 300 mg/kg) were administered intraperitoneally with saline one hour prior to the test. The control group received saline. For the purity of the experiment, all study drugs were used as chemically pure with active substance content of not less than 99.8%. The results are shown in Table 16.

Treatment with Pyracetam led to marked reduction of the pain threshold by 43.6 % at a dose of 100 mg/kg, by 48.1 % - at a dose of 200 mg/kg, and by 19.9 % - at a dose of 300 mg/k by the test of anxiety state (TA) and the growth of emotional response with the lowered threshold of emotional reactivity (TFC) by 23.5 and 47.0 %, respectively, at doses of 100 and 200 mg/kg, which indicates a direct psychoactive activity, leading to an unstable psycho-emotional state with the lower response thresholds and reduced sensitivity to pain impact. The decrease in the aggression threshold (TF) was 11.3 % at a dose of 100mg/kg, increase by 25.7 % - at a dose of 200 mg/kg and by 7.4 % - at a dose of 300 mg/kg. Piracetam reduces the stability of the anxiety reactions and of emotional response, increasing at low doses also the threshold of aggression, as well as causing instability in the psycho-emotional state lowering their thresholds, which is a negative manifestation of its psychotropic action.

The Fepiron racemate has a significant advantage by the aggression threshold which is increased twofold in comparison with controls in a dose of 100 mg/kg and by 48-84%, respectively, at 25 and 50 mg/kg. The anxiety and emotional responses thresholds actually do not change compared to the control.

Phenotropil increased the pain threshold (TA) by 74%, 65 % and 57 %, respectively, at doses of 25, 50 and 100 mg/kg. The emotional response threshold was increased by 40 %, 76 % and 82 % respectively, and the aggression threshold was increased by 8.7%, 4.0 % and 7.6 %, respectively, at doses of 25, 50 and 100 mg/kg. In comparison with piracetam, the threshold of aggression in Phenotropil treated group was stable and Phenotropil did not reduce it, but even slightly increases while maintaining an objective response in a conflict situation. Unlike Pyracetam (psychostimulant), Phenotropil improved consistently in all studied doses the emotional state and provided commensurately consolidating effect on all components of psycho-emotional reactions, showing also an analgesic effect.

Given the high neuroleptic, nootropic and mnemotropic, adaptogenic activity, the specific effect on TSIP and the results of this experiment, we can say that the effects arising from the modulatory activity are more organic, than the effects of stimulants and suppressing agents. The pronounced distinctive effects of Fepiron (tranquilizer) and Pyracetam (psychostimulant) indicate that the drug is able to be effective in bipolar mental disorders, as well as to exert an analgesic effect for pain of various etiologies and genesis, as well as to show the true adaptogenic properties.

### Example 17:

The results of studies of effect on the average life duration (ALD) of outbred white mice of both sexes at separate mode of maintenance (males and females) are presented in Table 17. The groups (30 mice, 15 females and 15 males) included mice aged 610 days without external signs of acute and chronic diseases. According to the long-term statistical observations, the average life duration of mice is 1.5-2.0 years, which corresponds to 547-730 calendar days. Phenotropil in doses of 50, 100, 200 and 300 mg/kg with distilled water was orally administered once a day in the morning throughout the life period of mice. The control group received distilled water and the second control was not subjected to any experimental intervention to exclude or to assess the effect of experimental stress under the influence of introduction of substances into the body.

The first series of studies was not successful due to respiratory viral infection of mice in the control groups (influenza, the source was found) ten days after the start of the experiment, but revealed an antiviral and anti-inflammatory activity of Phenotropil. In the control group, all animals died. In groups treated with Phenotropil, totally 15% of the mice died. Of the surviving mice, 46% did not get sick, and 54% had been ill, but survived. No gender differences in effect were found.

According to the studies of effect of Phenotropil on the average life duration of mice (Table 17) in the control groups without any treatment and receiving daily oral distilled water, no significant differences in life duration have been identified (the difference was within 3.0%), indicating the absence of experimentally stressful factors throughout the study. Administration of Phenotropil at doses below 50.0 mg/kg resulted in an increase in life duration by 8.0-10.0% and these data were not included in the table due to the insignificance of the result for the purposes. It is known that herbal adaptogens have effect within 10.0% on the life duration of animals (V.E. Chernilevsky, V.N. Krutko. History of studying agents for prolonging life. Journal Prevention of aging, No.3, 2000) which are actually psychostimulants with a cumulative effect (the effect is manifested in the use with long-term courses).

Phenotropil exerted a significant effect on life duration of mice of both sexes when administered at doses of 50, 100, 200 and 300 mg/kg. ALD was significantly higher than in the placebo control and no-treatment control groups. With respect to placebo-treated control mice, the ALD increased by 15.3 %, 29.3 %, 12.5 % and 19.6 %, respectively, which corresponded to 12, 20.6, 6.24 and 9.3 % in comparison with no-treatment control animals. The greatest effect in terms of life duration was obtained in the series of tests wren treated with of Phenotropil in doses of 100 mg/kg. It should be noted that during the course of prolonged use of Phenotropil at a dose of 300 mg/kg, the mice visually looked somewhat "untidy" and somewhat less active. No statistically significant difference between the ALD of males and females were found, indicating no selectivity in rejuvenescent activity of the compound also with regard to sex of animals in terms of ALD.

### Examples 18:

The maximum life span (MLS) of mice according to our long-term statistical observations is 2.7-3, 2 years, i.e. in average 2.95 years or 1077 days. For this study we selected mice (10 individuals per group), males with dominant behavior in mixed groups, that safely survived to 985 days without signs of pronounced senility and with satisfactory orientation-exploratory behavior, determined by the "open field test" method. Phenotropil in doses of 25, 50 and 100 mg/kg was administered orally with 5.0% glucose solution. Glucose solution was used solely to improve the solubility of the drug. One control group received the glucose solution and the second one - distilled water. The third control group was not treated.

The results of the study are presented in Table 18. Control animals of all three groups, despite a balanced diet and good conditions and care, did not survive the previously identified limit of MLS. Macroscopic examination at autopsy revealed in 95.0% males of control groups spontaneous tumors of various organs, i.e. even the specially selected "centenarians" actually were doomed to die, not without the involvement of the damaging effect of carcinogenesis (Table 18.1). Treatment with Phenotropil has significantly increased life span in all experimental groups, in average by 22.0%. The greatest effect (Table 18) was obtained after treatment with Phenotropil in doses of 25 mg/kg (23.3 %) and 100 mg/kg (24.0%). Incidence and severity of spontaneous tumors in Phenotropil mice was significantly lower (by 2.37 times) at a dose of 100 mg/kg (Table 18.1). With a sample of 30 individuals in total in the three groups, we can talk about a high reliability of the findings.

Phenotropil affects the maximum duration and quality of life of the animals, involving not only the functional elements of different levels, but also the genetic element, prevents development of carcinogenesis. Prevention of phenoptosis indicates that it prevents the development of apoptosis and organoptosis, because without all three components together it is impossible to get a positive result on the MLS. Cell or tissue apoptosis would result in death of functional organs or organ that in its turn would result in death of the whole organism.

Investigation of the effect of Phenotropil on body weight at course intragastric administration for 1 month was performed at doses of 25, 50, 100, 200 and 300 mg/kg/day in white outbred rats of both sexes with obvious signs of obesity (380-430 g) without changes in diet and behavior. Each animal was placed in a standard single cell. The body weight, the amount of water and food consumed per day were recorded.

Treatment with Phenotropil does not change the amount of food and water consumed, but the body weight of animals changed significantly depending on the dose (Table 18.2). The behavior of animals also became more active. Phenotropil exerted no anorexigenic effect as previously thought. Its effect in obesity is manifested by the slendering rather than by anorexigenic activity. The slendering effect by reducing the body weight was 14, 25, 33.3, 35 and 32%, respectively, relative to the control group of animals treated with distilled water.

### Example 19:

Investigation of the effect of Phenotropil on the fertility of mice was performed in the spring period in females aged 510 days (30 animals per group). Termination of the reproductive age was established by placing every three females to a fertile male. Mating was determined by the presence of vaginal plugs. Females not mated during one month were included in the study. Throughout the entire study period (32 days), Phenotropil was administered one time per day intragastrically to the experimental group in a dose of 100 mg/kg with distilled water; the control animals received distilled water. On the eighth day of the experiment, the females were introduced to males in the following combination: two experimental and one control animal or one control and two experimental animals. The procedure was repeated for females not covered at each stage. The same fertile males were not used for mating. The results of the study are presented in Table 19.

In the control group at the end of the first phase, no females were covered; by the end of the second stage of the research the pattern was repeated and two females died, by the end of the third - five females died and three were covered (10.0% of the initial amount). After 32 days no changes in the control group were observed. Taking into account that 10.0% of females in the control group were covered, we can assume either the fallacy in the sample selection within a ten percent or the experimental conditions provoked the result. Of 30 females in the experimental group, 9 females were covered by the morning of the day 9 of the experiment, which represents 30.0 % of the total number in the group, 11 females - by the morning of the day 17, and 4 females - by the morning of the day 25 days. The remaining females of the experimental group (6 animals) were left uncovered after 32 days, but two females from this group also died by the morning of the day 33.

Postmortem examination of dead females established the cause of death as natural due to insurmountable age related changes. Administration of Phenotropil restored not only the fertile attractiveness of the females for males, but also reproductive functions of the females. Phenotropil has incretomodulatory effect on the reproduction of biological substances that are attractive to males in the fertile period of females associated with hormonal reproductive status, exhibiting a pronounced rejuvenation effect when administered to animals.

### Examples 20:

Investigation of the effect of Phenotropil in amenorrhea and functional dysmenorrhea was conducted on groups of patients aged 14-27 years. The first group (20 -27 years) diagnosed with schizophrenia, was on the maintenance neuroleptic therapy and had menstrual cycle irregularities in the form of amenorrhea (for more than 6 months). Of those, 4 had amenorrhea of not established nature, observed also prior to the pronounced primary manifestation of mental disorder, 6 patients had amenorrhea that developed during treatment with neuroleptics. Phenotropil, tablets was administered in doses of 100 mg and 200 mg per day as part of a complex antipsychotic therapy as a corrector of psychogenic side effects of neuroleptics. The dose was selected based on the individual and clinical features of the patients. The duration of Phenotropil therapy was 3 months. Two groups (14-18 years) with functional dysmenorrhea and manifestations of pronounced spastic symptoms were studied. Duration of the Phenotropil monotherapy (50 and 100 mg per day) was one month followed by administration of the drug at one-week courses as a preventive agent. Patients with functional dysmenorrhea were investigated for cAMP level in comparison with the norm control group. The cAMP level was determined using the standard kits, by the method based on the competition with the labeled cyclic 3',5-adenosine monophosphate in the level of binding with thiobarbituric acid.

In the first group after the end of the study, all patients had improved state: increased motivation, increased range of interests, increased number of daily activities, increased intensity and range of emotional reactions. Seven out of ten patients (70 %, of which 40.0% - with amenorrhea treated with antipsychotic therapy and 30.0 % with amenorrhea of unknown nature) restored as a result of therapy with Phenotropil their menstruations and menstrual cycle. A delayed (relatively distant) and a very peculiar divergent activity of the drug was noted. It reduced the night sleep at the same time deepened it.

Phenotropil affects human reproductive functions and is able to restore their cycle in disorders, exerting modulatory activity both in amenorrhea associated with proper functional impairment of reproductive functions, and induced by neuroleptics. The results indicate that Phenotropil has not only a neuromodulatory and psychomodulatory, but also incretomodulatory activity, providing cyclicity in sex hormones in disorders of various etiologies. There is reason to assume that Phenotropil can be also effective in conditions of severe forms of the toxic phase both as an antipsychotic and as a corrector of humoral status in psychiatric patients.

The serum cAMP levels in two second groups at the peak of clinical manifestations of primary functional dysmenorrhoea was by 50-70 % higher as compared to their age-matched peers without functional disorder (control group, Table. 20.). Treatment with Phenotropil as monotherapy (for 30 days) eliminated the psycho-emotional instability. It improved mood, increased efficacy, reduced or eliminated pain and spasticity. The course use restored the functional norm of cAMP indicators compared with baseline studies, regulating the hormonal, neurological and mental status, exerting a positive effect on the processes of intracellular metabolism involving the endocrine system.

A simultaneous experiment carried out on outbred white mice of both sexes (20-24 g) showed that Phenotropil in doses of 10, 50 and 100 mg/kg without modeling of negative stress or pathology significantly stimulates intracellular metabolism (Table 20.1), increasing cAMP levels after a single intraperitoneal administration 60 minutes before the test, by 21, 65.5 and 96.3%, respectively.

### Examples 21:

It is known that neurotransmitters such as interleukin 1 (IL-1), interleukin 2 (IL-2), interferon (IFN), thymosin, tumor necrosis factor (TNF) have the ability to regulate the function of the central nervous system (CNS). Peptide ligands that perform neuroimmune interactions have receptors common to both systems. Immunocompetent cells can synthesize neuropeptides and respond to most, if not all, compounds of this group. Cells of the neuroendocrine system produce some lymphokines and monokines and respond to them after administration. The structural relatedness of receptors was shown, for example, for ACTH, endorphins, IL-1 and IL-2.

The study of immunocompetent activity of Phenotropil included patients with neurasthenia and organic asthenic disorder (20-47 years) of both sexes (men 37%, women 63%). Control group included practically healthy people (20 people). The study groups were similar in age and sex ratio. Phenotropil was administered at a dose of 100 mg/day, one time in the morning for 30 days. Study of the immune and mental status (level of IL- 1, IL- 2, IL- 3, IL- 6, α-TNF, α-INF in supernatants of cultures of peripheral mononuclears from whole blood) was carried out before treatment and at days 7, 14, 21 after the therapy course (30 days) and after 3 months of therapy. Blood sampling for analysis was carried out at the same time of day in the control group and in the group of patients. Peripheral mononuclear cells were isolated from the whole blood; interleukin level was determined by ELISA kits ("Immunotech", France).

Analysis of the results of quantitative enzyme immunoassay research of the spectrum of cytokines in supernatants preparations of peripheral mononuclear culture in asthenoneurotic disorders (neurasthenia, organic asthenic disorders) shows a steady change in the cytokine profile with a decrease of IL- 2, IL- 3 and IL-6 levels from control values in a population of healthy individuals, and increased α-TNF and α-INF levels, which indicates the uniformity of the vulnerability of immune responses in disorders of the mental state with asthenic nature of various etiologies. This was the basis for aggregation of patients with different root cause of neurotic states that belonged to the category of borderline mental disorders into a single group. The IL-1 level has a tendency to increase within 4.0% (Table 21).

The course treatment with Phenotropil restored the mental status of patients and the nature of the cytokine profile to the norm, showing the immunomodulatory activity, expressed in the correction of all the studied parameters. Intensity of the immunomodulatory effect corresponded to the intensity of changes in the parameters, but with the opposite sign, bringing into agreement activity of interleukins and restoring the balance of their ratios. If the IL- 1 level had only a tendency to increase (+4.3 % relative to control values), then after treatment with Phenotropil, the nature of the parameter completely restored relative to the control, decreasing with respect to baseline values (-3.5%). Changes in IL-2, IL-3, IL-6, α-TNF and α-INF levels, in the group of patients relative to control were: -12.7 %, -14.9, -15.8 %, +22, 0 %, +16.5 %, respectively, and treated with a course of Phenotropil (Table 27) with respect to the baseline values were +8.9 %, +12.7 %, +11.3 % -11.6 %, -10.3 %, respectively. Three months after the therapy, the interleukin profile remained at the normal level and was compared to baseline: +15.1 %, +18.0 %, + 19.0%, -18.2 %, -11.8 % with a stable IL-1 value (- 4.6 % compared with the baseline) and stable mental status. After a 3-month break, a consolidating therapy with Phenotropil at the same dosage was administered.

Phenotropil has a pronounced modulatory activity of the specific, comprehensive and universal types of actions, including psychomodulatory, neuromodulatory and immunomodulatory activity, restoring the mental and immune status in disorders of various etiologies, exhibits anti-inflammatory and anti-necrotizing effect, with a positive effect on the competent systems responsible for the growth and differentiation of activated lymphocytes. It restores, conjugatevely consolidates and stabilizes the balance of the neurohormonal functional systems.

### Example 22:

Investigations of the anti-seasick (anti-kinetosis) activity of agents with creation of visual-vestibular effects modeling motion sickness were performed on male subjects (23-35 years). Test with continuous exposure to the Coriolis acceleration was performed by rotating vestibulometric chair with angular velocity 180°/s. The eyes of the subject were open, while the optokinetic drum rotated in one direction with the chair with a speed of 90°/s. The time of visual-vestibular effect, vestibulo-vegetative reactions, optokinetic nystagmus before and after kinesia was determined when the drum rotated alternately for two minutes in the R- and S- directions at a speed of 90°s, with a break for 1 minute between rotations, as well as the severity of vestibular effects was measured and the Km was calculated. The studies were conducted 60 minutes after administration of Phenotropil in doses of 25, 50, 100 and 300 mg. Baseline state values and placebo values served as control. Alternate approaches for each participant were performed a week after each previous test. The modulation factor under normal conditions without exposure was 0.97.

It was found (Table 22), that after treatment with placebo, tolerability of the visual-vestibular effects (VVE) was 4.27 ± 0.7 min and the severity of the vestibulo-vegetative reactions (VVR) was 7.2 ± 1.1 points, the condition of the negative effects of exposure (NEE) lasted for 4-6 hours. Phenotropil has a pronounced anti-seasick (anti-kinetosis) activity, with efficacy of the dose from a higher to a lower result as 300>100>25>50 respectively. The average results compared with placebo were as follows: improvement of visual-vestibular tolerability was +80 %, reduction in the severity of the vestibulo-vegetative reactions was 68 % and the duration of negative consequences condition decreased to 15-30 minutes. Phenotropil at all doses increased the velocity of the slow phase of optokinetic nystagmus, which can be viewed as an improvement of visual functions in terms of tracking moving stimuli.

### Examples 23:

Studies of the effects of Phenotropil on condition of paradontium, enamel and dentin with topic application involved men and women (45-54 years) diagnosed with periodontitis, parodontosis and caries. Enrollment of groups by separate indicators in this age failed. In the vast majority of subjects, the diseases were combined. The gender ratio in groups was: 30.0% men and 70.0% women, the ratio of smokers to non-smokers 60:40, the number of female smokers was higher than male smokers by 35.0 %. All patients received every three days the required amount of 0.1 %, 0.2 % or 0.3 % solution of Phenotropil or placebo. The solutions were applied daily as dental trays (for 30 sec.) in the morning and evening at 30 ml per application for 30 days. Food and drinks intake, including water, were not recommended for 2 hours after the procedure. All patients underwent a dental check-up and standard hygienic treatment with removing plaque and dental scale and lavaging the gingival pockets with 1% Phenotropil solution. For each subject, the studied parameters were recorded; the aggregate integral indicator of the general periodontal condition of enamel and dentin was calculated, in the negative scores. The examinations were conducted once a week throughout the period of monotherapy. Simultaneous studies were conducted for evaluation of lipid peroxidation state in samples of saliva, at the baseline examination and after the course of therapy. The results are shown in Table 23.

Compared with the placebo-treated control group, subjects in all experimental groups displayed a pronounced positive effect after topical treatment with Phenotropil solutions. The tooth mobility significantly reduced, looseness, swelling, degradation and periodontal inflammation manifestations disappeared, caries lesions of enamel and dentin reduced. During therapy with Phenotropil, no formation of soft plaque and hard formations on teeth were noted. Recovery of the cell and tissue homeostasis was also manifested in the disappearance of cervical gingival pockets and in restoring integrity of the gingival cervical sulcus and, the most amazing thing, we observed recovery of dentin by visual inspection.

The positive effect of treatment with Phenotropil was observed after about 14 days and intensified in the course of therapy. Efficacy after one month of treatment with the drug was the highest and most evident at the initial stages of disease and moderate severity of periodontitis and caries. Effect on periodontitis was apparent, but less pronounced. Treatment of periodontitis probably requires a longer course and administration of Phenotropil tablets according to the usual regime to modulate the general functional state of the body. The ranking evaluation of efficacy (0 - no effect, 1- slightly pronounced effect below 30.0 % in the group, 2 - average degree of efficacy from 30.0 to 50.0%, 3 - high efficacy, above 50.0 %) was established by an integral indicator of the aggregate of all the parameters tested. The results are shown in Table 23.

The greatest complex effect was observed as a result of instrumental and visual examination after treatment with 0.2 % and 0.3 % drug solution. Subjective evaluations of patients in all Phenotropil-treated groups were similar and all patients reported good effect while the intensity of the effect created a positive psychological background in the doctor-patient relationship. Lack of effect of treatment with Phenotropil was not observed. No allergic reactions were observed; and the use of different doses of the drug would be likely reasonable, depending on the severity of the diseases, which requires special clinical studies.

Compared with the placebo-treated control group, one can speak of a high degree of therapeutic modulatory action of Phenotropil in various dental diseases and its modulatory and rejuvenescent activity against periodont and dentin, as well as of its anti-inflammatory and antimicrobial activity. It should be noted that 25.0 % of patients of the total number of smokers at the third week of therapy quit smoking, as some of them reported that Phenotropil provoked "aversion to the smell of cigarettes and cigarette smoke" while in the others "the need in the habit of smoking just disappeared" i.e. the drug overcame cravings and dependence, showing an anti-craving activity. Ratio inside this spontaneous group was distributed almost evenly, 43:47. Of those, 43% developed aversion to smoking that is the anti-craving effect was accompanied with a negative attitude towards the dependence, while in 47% the anti-craving activity of Phenotropil was not accompanied with an additional irritant effect. Aversion to smoking was most characteristic of women, and they constituted the vast majority in their subgroup by this indicator, more than 80.0%. All patients also reported a subjective fact of "clearing the lungs, coughing sputum" for about first two weeks 30-60 minutes after washing the mouth with Phenotropil solution. This observation was reported by both smokers and non-smokers. This indicates that the drug affects the dynamic and functional state of lungs.

Behaviour of the peroxidation indicator is shown in Table 23.1. Treatment with Phenotropil in all cases was associated with normalization of the condition in terms of lipid peroxidation as measured by MDA.

### Examples 24:

Example 24 presents the results of research of Phenotropil as parapharmaceutic - cosmeceutical agents in women (age 53-60 years) and as a standard solution. The 0.1% water solution (distilled water) of Phenotropil and placebo was prepared; for convenience of preparing therapeutic cream formulation, the standard composition of cream base was used, or actual creams "Vecher", "Lux" and "Gerantol" manufactured by "Svoboda" (Russia) were used as the base, with 100, 200, 300 mg of Phenotropil per 100 g of finished compositions to prepare a cosmeceutical agent. The cosmeceutical formulations were prepared with the powder of Compound 1 sifted and well ground in a mortar.

At baseline examination, main indicators of the skin condition of the hands and face in the groups were recorded, integral index for aggregate characteristics was calculated (dryness, wrinkling, superficial vascular fascicles, the intensity of subcutaneous vascular pattern, pigmentation - the intensity of color, the number of pigment spots and their total area). Preliminarily, each subject was tested for allergic skin reaction with the most concentrated formulation. No allergic reactions have been identified. In one group, only solution of Phenotropil was used in the morning and evening, applying a small amount (like a regular lotion) of the solution on cosmetic cotton pads and wiping the face and hands. In the other groups, this procedure was carried out before applying the Phenotropil-containing cream. The results of the baseline evaluation in each group were taken for 100.0%. The findings are presented in Tables 24-24.5.

Analysis of the studies has shown that Phenotropil has a pronounced anti-inflammatory and rejuvenescent activity with external use both as monotherapy and in combination with biologically active substances of compositions of organic and inorganic origin. Its efficacy in all cases was significantly higher also in comparison with the standard composition of the creams "Lux ", "Gerantol", "Vecher", which themselves did not no show any significant effect in the studies, even though they contain biologically active substances. The degree of positive action of Phenotropil ranged from 140 to 280% depending on its concentration in composition.

Effects of Phenotropil manifested starting from the first week of treatment, and intensified by the end of the study. The skin became soft and supple, wrinkles and intensity of cell pattern smoothed, age spots paled and in most cases disappeared, appearance of surface and subcutaneous vascular pattern normalized, vascular bundles disappeared from the surface of the skin, the mobility of the finger joints significantly improved, manifestations of joint stiffness, swelling and pain disappeared in cases where they were present, hands and face grew thin, the skin was tightened. It was noted that, notwithstanding the more pronounced rejuvenescent effect at 300 mg per 100 g of cream base or composition, a softer effect was still present at Phenotropil dilution in doses of 100 and 200 mg. No allergic reactions or side effects during the course were noted.

In this series of studies, it was also noted that the speed of onset of the therapeutic effect was more pronounced at the use of the investigated course in the patients of younger age. It was also noted, that the use of cosmeceutical formulations with Phenotropil for the face in the evening time is more appropriate 2-4 hours before bedtime, as the application just before bedtime, despite the recommendations, resulted in some (18.0 % of total) subjects in the morning to the presence of a mild (by sensations of the subjects themselves) facial swelling associated with the use of creams immediately or less than two hours before bedtime, which swelling resolved in about 2-3 hours after sleep. For skin of the hands, this peculiarity of the evening application did not have any significance, but in the case of Phenotropil application the face skin this feature should be considered, though it was not established whether Phenotropil itself or the compositions used for preparation of the cream base caused this effect.

The study results show that the use of Phenotropil with therapeutic anti-inflammatory, rejuvenescent, slendering, preventive or protective purposes in medicine and cosmeceuticals can be useful. Taking into account that Phenotropil had no irritating effect on the skin of hands and face, its topical application also for other body parts will not be contraindicated as well except individual intolerances. The anti-inflammatory effect of Phenotropil when applied topically is no less important than its rejuvenescent and slendering activity of cosmeceutical orientation. The identified anti-inflammatory and rejuvenescent effects when used topically may be promising in the treatment of eye and ENT diseases of different etiologies.

### Examples 25:

A study of effect of Phenotropil on the scalp and face skin in seborrhea oleosa and on the scalp skin in dry seborrhea, as well in the mixed form, was performed on mixed groups of both sexes (15-37 years). The 0.1% Phenotropil solution was used. In the group with mixed form, additional Phenotropil tablets were administered at a dose of 100 mg per day (in the morning). The baseline examinations were used as control. The prepared samples were rubbed into the scalp once a day 1 or 2 hours before bedtime. In the case of acneiform rash, the skin was wiped in the morning and evening by cotton pads soaked (as usual lotion) by 0.1 % aqueous Phenotropil. Washing the hair using shampoo was not recommended. A neutral baby soap "Alice" by OAO Svoboda and the rinse prepared with 25 ml of lemon juice and 100 mg Phenotropil powder per 1000.0 ml water were used. Studies were conducted for 28 calendar days with monitoring examinations every 14 days. In the dry seborrhea group, three patients withdrew from the study after two weeks due to reluctance to undergo further examination; they found it difficult to comply with the regime of the detergents use. The skin state was assessed by a 4-point system, where 0 - absent, 1 - the effect intensity less than 50.0 %, 2 - the effect intensity more than 50.0 %, 3 - the effect intensity more than 80 % but less than 100.0 % of the cases.

Phenotropil (Tables 25 and 25.1) was effective for the dry (DF), oily (OF) and mixed (MF) forms of seborrhea of the scalp, as well as for acneiform rash on oily and mixed face skin types. Effects of the agent were manifested by normalization of condition of oily and dry skin, a marked reduction in rash in adolescents, a relatively rapid cleansing of sebaceous channels and their regeneration without scarring, removal of pigmentation, redness, decreased formation of keratinized scales (dandruff) of the scalp. Compared with the control group, the therapeutic effect in seborrhoea was 200.0 %. The pronounced effect of the drug was observed after two weeks and its efficacy was not reduced till the end of the study. The most pronounced effect (300.0 %) compared to the control group was determined with simultaneous use of Phenotropil tablets. Effect on acne rash was 79-84 % at one month of application of the Phenotropil solution.

The findings suggest efficacy of Phenotropil that manifests in complex modulatory activity in various forms and etiology of adverse skin conditions including hormonal age-related restructuring associated with the transition to the fertile period, as well as in the secondary form of seborrhea. The combination method of application of the agent is the most effective. The observations also showed that the duration of treatment should be adjusted individually in each case, since the intensity of the effect in the subjects was not uniformly identical. Phenotropil also possesses antimicrobial activity; its use markedly reduces the microbial activity on the skin surface, promotes the protective skin properties against external influences.

### Examples 26:

A study of the slendering effect of Phenotropil (100, 200 and 300 mg once a day for 30 days) in women (37-65 years) with alimentary constitutional obesity and non-obese groups (males and females 23-47 years) are presented in Table 26. The control groups received placebo tablets. The nutrition regimen and ration were not regulated; no recommendations on calories or regimen were given. The purpose of the study was to identify the effect of the drug without the influence of the diet. To assess the condition before and after treatment, in addition to the measurement of body weight, the dynamics of the general clinical condition was studied: asthenia scale MFI- 20, Hospital Anxiety and Depression Scale, the quality of life scale SF- 36. The questionnaires were completed twice: one day before treatment and the next day after the end of treatment.

The body weight of subjects at baseline exceeded the constitutive norm on average by 35 ± 15%. After course treatment by placebo, body weight increased by an average of 8% compared to the baseline. When treated with Phenotropil, the body weight decreased depending on the dose (Table 26) by 12, 15 and 18 %, respectively at 100, 200 and 300 mg. In the absence of a controlled diet and nutrition regimen, the following was noted: increased sensitivity of gustatory and olfactory receptors, which contributed to a voluntary refusal from previously attractive food with artificial flavors, voluntarily replacing fat meat with lean meat and fresh fish. Patients reported a decrease in demand for sugar and salt, aversion to not-fresh food, change of preferences in perfumery ("from strong odors to more subtle one"), increased need for vigorous activity, increased sexual potency (noted by both men and women). Analysis of the rating scales showed a positive trend for all indicators. Indicators of fatigue decreased by 92%, anxiety and depression decreased on average by more than 60%. Quality of life before and after Phenotropil treatment showed improvement on all subscales, including social, role and physical functioning, as well as due to the psycho-emotional state and mental health. At the same time, elimination of comorbidities was noted: prostatitis in 3 patients (positive effect in 3 of 3), and cramps when urinating in 5 (positive effect in 5 of 5), chronic sinusitis in 8 patients (positive trend in 8 of 8), 18 patients had lost ability to sense and discriminate odors before the therapy (positive effect in 11 of 18, and in 7 - positive trend of recovering sense of smell).

The effect of Phenotropil (25, 50 and 100 mg in the morning) on diuresis was investigated in non-obese groups (males and females 23-47 years) for 10 days. The daily volume of urine when treated with placebo and Phenotropil was recorded.

In the diuresis study groups (Table 26.1), after three days of treatment with placebo, the average daily volume of urine at three and ten days did not change significantly. Phenotropil increased the daily urine output by 30-37% based on the average index in the first three days. After stabilizing, fluctuations in urine output in the next 7 days were 5-10%, which corresponds to normal fluctuations.

Phenotropil has not an anorexigenic as previously thought, but rather slendering activity. By regulating metabolism, it reduces the body weight, increases qualitatively the productive control, while inducing an increased activity. Phenotropil normalizes psycho-emotional status and self-esteem, restores the activity of gustatory and olfactory receptors, exhibits anti-inflammatory activity in chronic comorbidities, and normalizes sexual potency. It has a pronounced diuretic effect. By normalizing the amount of fluid in the body, it maintains its normal functional level. Phenotropil has no dehydrating effect and thus prevents washing-out of electrolytes and other useful substances synthesized in the body, maintaining their balance ratios, that advantageously distinguishe the product from known diuretics.

### Example 27:

Table 27 presents the results of studies in women (22-35 years) diagnosed with migraine without aura (simple migraine) - 5 subjects, and migraine with aura (classic migraine) - 5 subjects. Phenotropil as used as nasal drops (0.1 % sterile solution), 10 drops three times a day for 7 days for the purpose of relief and prevention of migraine attacks at onset of prodromal phenomena and aura episodes. 5 drops of the solution were administered into each nostril. One drop of the 0.1 % standard solution contained 0.05 mg of Phenotropil.

Treatment with Phenotropil on the stage of manifestation of the chronic disease episode completely stopped prodrome phenomena and aura, without development to headache attacks in 5 of 10 patients in the group, in 3 patients, headaches and their duration decreased significantly, and in 2 - no effect was noted. Lack of effect was not dependent on the type of migraine. Phenotropil is effective for the prevention and relief of chronic migraine status, its use may be advantageous in such cases both with systemic injection into the body, and intranasal administration. The analgesic and antispasmodic effect of Phenotropil in conjunction with psychomodulatory and neuromodulatory action may be useful not only for migraines.

Summing up, in the present invention, it was shown for the first time that all previously known sources of information concern not the product itself (compound, composition), but rather some of its related products, burdened by various faults. The accomplished technical result, the method for obtaining thereof and the examples of the invention studies for the first time identified the properties and characteristics of the product itself. It was established that enantiomers of the racemic compound (RS)-2-(2-oxo-4-phenylpyrrolidin-1-yl)acetamide possess a significant biological activity, the ability of the molecules to form different enantiomeric conformations in different environments and depending on the environment that does not preclude their ability to transform into each other and reverse.

It was established that if the compound is not burdened with content of the related impurities, and especially not distorted for some reason, then enantiomers do not degrade, but rather improve the properties and characteristics of the compound. For the first time the flawless product was obtained, its properties and characteristics were ensured in its related products, which are substantially different from everything previously known, including also the nature of the effect of its nootropic activity, which is characterized not just by stimulating effect, but by bipolarly coupled asymmetry.

Unique and important new biological activities of (RS)-2-(2-oxo-4-phenylpyrrolidin-1-yl)acetamide, as well as more pronounced efficacy of the known ones were discovered, while significant flaws were eliminated. Its safety and efficacy were significantly increased, the therapeutic range increased, providing possibility to significantly expand the scope of the use of the compound when administered also topically. The ability of molecules to enantiomeric conformations, to overcoming of specialization of cells and tissues prevents the formation of abnormal dominant forms, provides an outstandingly broad spectrum of biological activity of the compound. Achieving the sustainable technical result does not require complicated methods of preparation.

The identified modulatory types of activity meet all obligate criteria of commensurate effect and are accompanied by a variety of additional components of action. (RS)-2-(2-oxo-4-phenylpyrrolidin-1-yl)acetamide has a modulatory activity with the commensurate effect at different levels and from different levels. The commensurate action of the stereospecific effects of 2-[(4RS)-2-oxo-4-phenylpyrrolidine-1-yl]-acetamide has virtually no limits what was shown by examples of studies. It can be characterized by both stimulation, and suppression, and their supportivity with simultaneous relevance (relevance as a relation between the stimulus and the response), restriction and reversibility. Relativity of coupling the 3S ↔ 3R processes and their balance relation after treatment with the compound are accompanied by pronounced side effects and aversivity at both single and course application of the compound under different types of norm and different types of disorders and pathological conditions, which is also a significant advantage. The modulatory effects of the compound manifest commensurately at different types of norm and in various disorders and pathological conditions on functional and pathogenetic, systemically important, and structural levels.

As exemplified by the chemically pure stable compound (RS)-2-(2-oxo-4-phenylpyrrolidin-1-yl)acetamide and the compound as close to it so that it does not have any significant distinguishing properties and characteristics, it is shown that modulators with commensurate effect have fundamental differences from the stimulating and suppressing agents, as well as from divergents. (RS)-2-(2-oxo-4-phenylpyrrolidin-1-yl)acetamide has substantial advantages over them. The use of the acting compound is not limited by age and gender; the compound is highly effective both at systemic administration and at the topical application.
**Fig. 1****. Mass spectral analysis of a sample of the pharmaceutical substance Phenotropil.**

**Table 1. Parameters of quality standards of pharmaceutical substances of Phenotropil and methods of investigation; compliance shown on example one of the samples *.**

| **Parameters** | **Methods** | **Established standards and compliance** |
|---|---|---|
| Description | Organoleptic | White, with bitter taste, odorless (standard - crystalline powder, white or white with a slightly yellowish or creamy tone, odorless, bitter taste. |
| Solubility | State Pharmacopoeia XII | Complies - sparingly soluble in water, moderately soluble in 96% alcohol, in and chloroform. |
| Identification | IR spectroscopy | Complies. Infrared spectrum of the compound predried at a temperature 100 to 105°C for 30 minutes, registered in mineral oil in the range 4000 to 400 cm⁻¹, has a full match in the absorption bands corresponding to the characteristic spectrum pattern of the standard chemically pure (C₁₂H₁₄N₂O₂). |
| | | |
| | UV spectrophotometry | Complies. Ultraviolet spectrum of a 0.05% solution in a mixture of 96% alcohol and 1 M hydrochloric acid (9:1) in the range from 200 to 380 nm has an absorption maximum at 258 nm ± 2 nm; a minimum at 240 nm ± 2 nm and shoulder in the range from 251 to 257 nm. A mixture of 96% alcohol and a 1M of hydrochloric acid solution (9:1) was used as a comparison solution. |
| | | |
| | Qualitative reaction with sodium hydroxide solution | Confirmed. 0.1 g of the compound was heated on a water bath with 5 mL of water and 5 ml of 1M sodium hydroxide solution. Ammonia was produced, detectable by odor and blue coloration of wet red litmus paper. |
| Melting temperature | State Pharmacopoeia XII part 1, p. 29. | 131.0-131.5 °C (standard value 130°C to 133 °C) |
| Transparency | State Pharmacopoeia XII | Complies. A solution of 0.05 g of the compound in 10 ml of water by turbidity is comparable to Standard L |
| Colour | State Pharmacopoeia XII | A solution of 0.05 g of the compound in 10 ml of water by turbidity is comparable to Standard Y7. |
| Individual impurities | HPLC, any individual impurity or the sum not more than 0.2% | 0.1% found in the sum and in comparison with the reference standard solutions (RSS). |
| Sulphated ash | State Pharmacopoeia XII, Not more than 0.1 % | Less than 0.1 % |
| Heavy metals | State Pharmacopoeia XII, Not more than 0.001 % | Less than 0.001 % |
| Loss on drying | State Pharmacopoeia XI, not more than 0.1 % or not more than 0.5 % | 0.02 % |
| Quantitative assay | Titrimetrically | 99.43 % of C₁₂H₁₄N₂O₂ (standard value: not less than 99.0% and not more than 100.5% on dry basis). |
| | | |
| based on dry matter | HPLC | 99.87 % |
| Residual amounts of organic solvents | GLC - Not more than 3000 ppm (no more than 0.3%) individually or in total. | 750 ppm (0.075%) of isopropyl alcohol found. |

| | | |
|---|---|---|
| * - Microbiological purity or apyrogenicity meets the standard requirements of the Pharmacopoeia for non-sterile and sterile kinds of pharmaceutical drug substances. | | |

**Table 1.1. HPLC analysis of a sample of Phenotropil substance by the basic parameters.**

| **Substance** | **Solution of the substance in the mobile phase, 1.36 mg/ml, volume 10 µl** | | | |
|---|---|---|---|---|
| | **Time (min)** | **Area (%)** | **Theoretical plates** | **Asymm.** |
| 1 | 4.106 | 0.00 | 6075 | 2.81 |
| 2 | 4.512 | 0.03 | 6664 | 1.32 |
| 3 | 5.586 | 0.01 | 3650 | 0.39 |
| Phenotropil | 6.271 | 99.87 | 13795 | 1.17 |
| 4 | 10.38 | 0.01 | 15043 | 2.65 |
| 5 | 11.92 | 0.04 | 17516 | 1.32 |
| 6 | 12.75 | 0.05 | 16904 | 1.46 |

**Table 2. Investigation of acute toxicity of Compounds of Phenotropil substance in male mice (18-24g) with single intraperitoneal injection.**

| **Groups (n = 30 per group)** | **LD₅₀, mg/kg** |
|---|---|
| Control (distilled water) | - |
| Compound 1 | 1042 (982.9 - 1102.0) |
| Compound 2 | 1001 (959.1 - 1042.9) |
| Compound 3 | 1050 (984.6 - 1115.3) |
| Compound 4 | 904 (857.3 - 950.4) |

**Table 2.1. Evaluation of acute toxicity of Compound 1 of Phenotropil with single intraperitoneal injection in rats of both sexes aged 30-35 days (90-110 g).**

| **Dose (mg/kg)** | **Dead/total number** | **% of dead rat pups** | **LD₁₆** |
|---|---|---|---|
| | | | **LD₅₀** |
| | | | **LD₈₄** |
| 800 | 0/10 | 0 | LD₁₆ = 906.7 |
| 1000 | 4/10 | 40 | (902.4 - 911.0) |
| 1100 | 9/10 | 90 | LD₅₀ = 1029.5 |
| | | | (940.0 - 1119.0) |
| 1200 | 9/10 | 90 | LD₈₄ = 1159.0 |
| 1500 | 10/10 | 100 | (1154.0 - 1164.0) |

**Table 2.2. Evaluation of acute toxicity of Compound 1 and Compound 3 of Phenotropil with single intragastric administration in immature outbred white rats of both sexes aged 30-35 days (90-110 g).**

| **Dose (mg/kg)** | **Dead/total number** | **% of dead rat pups** | **LD₁₆** |
|---|---|---|---|
| | | | **LD₅₀** |
| | | | **LD₈₄** |
| Compound 1 - 800 | 0/10 | 0 | Not determined due to inability of administration of higher doses |
| Compound 3 - 800 | 0/10 | 0 | |
| Compound 1 - 1000 | 0/10 | 0 | |
| Compound 3 - 1000 | 0/10 | 0 | |

**Table 3. Study of effect of Phenotropil in doses of 5 and 10 mg/kg (intragastrically) on locomotor activity in the Opto-varimex installation in the population in genetically high and low active rats (n = 10 per group, age 30-35 days, body weight 90-110 g).**

| **Groups of rats/Intervals of registration** | **0-5 min** | **5-10 min** | **10-15 min** | **15-20 min** | **20-25 min** | **25-30 min** | **Accumulated** |
|---|---|---|---|---|---|---|---|
| Control (normal) | 1447.6±39 | 1216.2±342 | 785.6±262 | 465.6±213 | 92.2±48 | 92.6±37 | 4099.8±519 |
| Phenotropil 5 mg/kg | + 18.06 % * | - 5.95 % | - 13.03 % * | - 22.55 % * | + 50.76 % * | + 50.97 % * | + 4.28 % * |
| Phenotropil 10 mg/kg | + 38.8 %* | - 5.78 % | + 5.56 % | + 42.91 * | + 95.6 % * | - 91.47 % * | + 18.0 % * |
| Control (HEF) | 1743.5±52 | 1332.0±423 | 1001.8±275 | 72.0±23 | 57.3±37 | 42.4±45 | 4763.9± 643 |
| Phenotropil 5 mg/kg | - 0.06 % | + 7.36 % * | - 7.36 % | - 96.53 % * | - 103.15 % * | + 207.1 % * | - 12.0 % * |
| Phenotropil 10 mg/kg | - 4.2 % | - 8.4 % | - 3.73 % | + 7.44 % | - 4.72 % | 0 | - 4.23 % |
| Control (LEF) | 1201.6±34 | 1094.6±332 | 709.4±211 | 323.9±135 | 20.0±18 | 1.01±0.3 | 3349.5±409 |
| Phenotropil 5 mg/kg | + 15.0 % * | + 6.18 % | + 9.74 % | + 29.1 % * | + 2.1 % | + 10.0 % * | + 72.12 % * |
| Phenotropil 10 mg/kg | + 4.31 % | + 11.31 % * | + 27.85 % | + 7.23 * | + 14.39 % * | + 3.75 % | + 68.82 % * |

| | | | | | | | |
|---|---|---|---|---|---|---|---|
| * - significant differences compared to the control groups (p < 0.05). | | | | | | | |

**Table 3.1. Effect of substances of Phenotropil (100 mg/kg) at subchronic use (once a day for 5 days) on exploratory behavior of mice with high (HEF) and low effectiveness (LEF) in the cross maze compared with chemically pure Pyracetam substance (200 mg/kg).**

| **Substance (mg/kg) (n = 10 per group)** | **Number of explorations of maze compartments and number of surveillances, Kt** | | | |
|---|---|---|---|---|
| | **HEF (Number of compartment explorations // number of surveillances)** | **Kt** | **LEF** | **Kt** |
| Dist. water | 6.0 ± 0.5 // 1.8 ± 0.1 | 3.3 ± 0.3 | 8.7 ± 0.4 // 1.4 ± 0.2 | 6.2 ± 0.3 |
| Compound 1 - 100 | 4.3 ± 0.4 // 1.3 ± 0.2 | 3.3 ± 0.3 | 5.0 ± 0.5 // 1.6 ±0.2 * | 3.1 ± 0.35* |
| Compound 4 - 100 | 6.5 ± 0.7 // 1.6 ± 0.2 | 3.9 ± 0.45* | 7.0 ± 0.6 // 1.8 ± 0.2 | 3.9 ± 0.4* |
| Piracetam - 200 | 4.2 ± 0.7 // 1.9 ± 0.2 | 3.1 ± 0.45 | 5.3 ± 0.8 // 2.2 ± 0.2 * | 2.4 ± 0.5* |

| | **Population as a whole** | **Kt** | | |
|---|---|---|---|---|
| Dist. water | 6.9 ± 0.42 // 1.66 ± 0.1 | 4.3 ± 0.26 | | |
| Compound 1 - 100 | 4.5 ± 0.7 // 1.4 ± 0.2 | 3.2 ± 0.45 * | | |
| Compound 4 - 100 | 6.7 ± 0.7 // 1.7 ± 0.2 | 3.9 ± 0.45* | | |
| Piracetam - 200 | 4.6 ± 0.7 // 2.0 ± 0.2 | 1.3 ± 0.45* | | |

| | | | | |
|---|---|---|---|---|
| * - significant differences compared to the saline-treated group at p ≤ 0.05. | | | | |

**Table 3.2. Biologically active doses of Phenotropil by effect on the plasma level of cortisol in rats and evaluation of training stress factor (Tsf) activity, depending on initial the condition in different rat strains.**

| **Substance (mg/kg)** | **Tsf-activity measured by cortisol** | | | |
|---|---|---|---|---|
| **(n = 10 per group)** | **at 60 min, nmol/l** | **at 60 min, %** | **at 3 hours, %** | **km** |
| Control (Dist. water) - August | 39.6 ± 1.7 | 100 | 100 | 1.13 |
| Phenotropil 1.0 - 750 | 45.6 ± 4.1 | +14.1* | + 6.8 | 1.0 * |
| Control (Dist. water) - Wistar | 49.6 ± 3.4 | 100 | 100 | 0.9 |
| Phenotropil 1.0 - 750 | 50.9 ± 4.4 | + 2.0 | - 7.6 | 0.88 |

| | | | | |
|---|---|---|---|---|
| * - significant compared to the baseline control in strain groups at p ≤ 0.05. | | | | |

**Table 4. Effect of Phenotropil and olanzapine on apomorphine verticalization (a metod for modeling the positive symptoms of schizophrenia associated with hyperactivation of the dopamine system).**

| **Substance (mg/kg)** | | **Intensity of verticalization in points** |
|---|---|---|
| Apomorphine | 2 | 49 ± 6.4 |
| Phenotropil | 100 | 27 ± 8.7* |
| Phenotropil | 200 | 25 ±5.1* |
| Phenotropil | 300 | 19 ± 5.1 * |
| Olanzapine | 1.0 | 36 ± 8.7 |
| Olanzapine | 10 | 16.4 ± 8.4* |

| | | |
|---|---|---|
| * - significance of differences with control (apomorphine) at p ≤ 0.05. | | |

**Table 4.1. Effect of Phenotropil and olanzapine on hyperkinesis induced by 5-oxytryptophan in mice (a metod for modeling negative symptoms of schizophrenia associated with hyperactivation of the serotonergic system).**

| **Substance** | **(mg/kg)** | **Number of shakings** |
|---|---|---|
| 5-oxytryptophan | 300 | 11.3 ± 2.6 |
| Phenotropil | 100 | 1.4 ± 0.5* |
| Phenotropil | 200 | 1.5 ± 0.8* |
| Phenotropil | 300 | 1.2 ± 0.5* |
| Olanzapine | 1.0 | 0 * |

| | | |
|---|---|---|
| * - significance of differences with control (5-oxytryptophan) at p ≤ 0.05. | | |

**Table 4.2. Effect of Phenotropil and olanzapine on tremor caused by arecoline in mice (metod for modeling hyperactivation of central M-cholinergic system as a compoment in the pathogenesis of psychosis and identification of adverse reactions of treatment with neuroleptics).**

| **Substance** | **(mg/kg)** | **Duration of tremor (sec.)** |
|---|---|---|
| Arecoline | 25 | 418 ±89 |
| Phenotropil | 200 | 153 ±27 * |
| Phenotropil | 300 | 208 ±36* |
| Olanzapine | 1.0 | 178 ±28* |

| | | |
|---|---|---|
| * - significance of differences with control (arecoline) at p ≤ 0.05. | | |

**Table 4.3. Effect of Phenotropil on haloperidol-induced catalepsy (modeling symptoms of Parkinson's disease) in mice.**

| **Substances (mg/kg)** | **Catalepsy (sec)** | | |
|---|---|---|---|
| | **60 min** | **120 min** | **180 min** |
| Haloperidol 0.5 | 1.3 ± 0.8 | 27.5 ± 8.5 | 63.4 ± 12.8 |
| Haloperidol 0.5 + Phenotropil 50 | 0.0 ± 0.0 * | 8.5 ± 3.5 * | 4.8 ± 2.1 * |
| Haloperidol 0.5 + Phenotropil 100 | 0.0 ± 0.0 * | 2.0 ± 1.0 * | 0.0 ± 0.0 * |
| Haloperidol 0.5 + Phenotropil 300 | 6.6 ± 1.9 * | 10.6 ± 4.5 * | 21.4 ± 8.4 * |

| | | | |
|---|---|---|---|
| * - significant at p ≤ 0.05 compared with haloperidol. | | | |

**Table 5. Effect of Phenotropil on the horizontal motor activity of mice in the open field test.**

| **Substance** | **Distance covered during 1st min** | **Distance during 2nd min** | **Distance during 3rd min** | **Total distance at 3 min** |
|---|---|---|---|---|
| Control | 9±2.1 | 16.2±2.3 | 17.3±2.8 | 42.5±6.1 |
| Phenotropil 100 mg/kg | 23.7±5* | 22.5±3.1 | 44±10.7* | 90.2±8.7* |
| Phenotropil 200 mg/kg | 31.7±2.8* | 41.8±7.0* | 33.5±4.7* | 107±11.5* |
| Phenotropil 300 mg/kg | 29.7±12.3* | 27.2±10.2* | 28±6.4 | 84.8±28.0* |
| Olanzapine 1 mg/kg | 8.3±1.6 | 8.8±0.8* | 10.2±0.9* | 27.3±2.6* |

| | | | | |
|---|---|---|---|---|
| * - significance of differences from control at p ≤ 0.05. | | | | |

**Table 5.1. Effect of Phenotropil on the vertical motor activity of mice in the open field test.**

| **Substance** | **Distance covered during 1st min** | **Distance during 2nd min** | **Distance during 3rd min** | **Total distance at 3 min** |
|---|---|---|---|---|
| Control | 1.2±0.6 | 1.2±0.6 | 0.8±0.6 | 3.2±1.7 |
| Phenotropil 100 mg/kg | 2.3±1.1 | 3.8±1.7* | 4.7±1.6* | 10.8±4.2* |
| Phenotropil 200 mg/kg | 0±0 | 1.3±0.7 | 1.7±0.7 | 3.0±1.3 |
| Phenotropil 300 mg/kg | 0.3±0.2* | 2±1.2 | 2.7±1.1 | 5.0±1.8 |
| Olanzapine 1 mg/kg | 0.8±0.3 | 0.3±0.2* | 0.5±0.2* | 1.7±0.4* |

| | | | | |
|---|---|---|---|---|
| * - significance of differences from control at p ≤ 0.05. | | | | |

**Table 5.2. Effect of Phenotropil on explored holes in the open field test.**

| **Substance** | **Number of explorations during 1st min** | **Explorations during 2nd min** | **Explorations during 3rd min** | **Total number of explorations at 3 min** |
|---|---|---|---|---|
| Control | 0.8±0.3 | 0.3±0.2 | 1.0±0.6 | 2.2±0.7 |
| Phenotropil 100 mg/kg | 0.5±0.3 * | 1.5±0.4* | 1.3±0.7 | 3.3±0.9 * |
| Phenotropil 200 mg/kg | 0.2±0.2 * | 0.8±0.5 * | 0.5±0.5* | 1.5±0.9 * |
| Phenotropil 300 mg/kg | 0.3±0.2 * | 1.5±0.8* | 1.0±0.8 | 2.8±1.4 * |
| Olanzapine 1 mg/kg | 0.7±0.2 | 0.5±0.2 | 1.0±0.3 | 2.2±0.4 |

| | | | | |
|---|---|---|---|---|
| * - significance of differences from control at p ≤ 0.05. | | | | |

**Table 5.3. Effect of Phenotropil on number of washings in the open field test in mice.**

| **Substance** | **Number of washings during 1st min** | **Washings during 2nd min** | **Washings during 3rd min** | **Total number of washings at 3 min** |
|---|---|---|---|---|
| Control | 0.2±0.2 | 0.2±0.2 | 0±0 | 0.4±0.2 |
| Phenotropil 100 mg/kg | 0.2±0.2 | 0.1±0.2 | 0±0 | 0.3±0.2 |
| Phenotropil 200 mg/kg | 0±0 * | 0+0 * | 0±0 | 0±0 * |
| Phenotropil 300 mg/kg | 0.2±0.2 | 0±0 * | 0±0 | 0.2±0.2 * |
| Olanzapine 1 mg/kg | 0.5±0.2 * | 0.5±0.2 * | 0.2+0.2 * | 1.2±0.4 * |

| | | | | |
|---|---|---|---|---|
| * - significance of differences from control at p ≤ 0.05. | | | | |

**Table 5.4. Effect of Phenotropil on number of visits of animals to the center in the open field test in mice.**

| **Substance** | **Visits during 1st min** | **Visits during 2nd min** | **Visits during 3rd min** | **Total number at 3 min** |
|---|---|---|---|---|
| Control | 0.3±0.3 | 0.5±0.3 | 1±0.6 | 1.8±0.4 |
| Phenotropil 100 mg/kg | 0.7±0.5 | 2.2±0.8 | 1±0.5 | 3.9±0.6* |
| Phenotropil 200 mg/kg | 0.3±0.2 | 2.7±0.4* | 1.7±0.4 | 4.7±0.7* |
| Phenotropil 300 mg/kg | 1.2±0.4 | 1.3±0.5 | 1.7±0.5 | 4.2±0.8* |
| Olanzapine 1 mg/kg | 0±0 | 0.5±0.2 | 0.2±0.2 | 0.7±0.3* |

| | | | | |
|---|---|---|---|---|
| * - significance of differences from control at p ≤ 0.05 | | | | |

**Table 6. Effect of psychotropics on components of the transcallosal stimulation induced potential.**

| **Drug** | **Dose, mg/kg** | **P₁** | **N₁** | **P₁+N₁** | **P₂** |
|---|---|---|---|---|---|
| Normal saline solution | - | 0 | 0 | 0 | 0 |
| Piracetam | 300 - 500 | +++ | ++ | ++ | + |
| Pyritinol | 50 - 150 | + | + | + | + |
| *Phenotropil-Compound 1 and Compound 3 | 25 - 300 | **±** | **±** | **±** | **±** |
| Phenotropil-Compound 2 | 25 - 300 | ± | ± | ± | ± |

| | | | | | |
|---|---|---|---|---|---|
| +++ - amplitude increase by more than 50% + + - amplitude increase by 30 to 50% + - amplitude increase by less than 50%;-amplitude decrease by more than 10%; ± - biphasic effect, less than 50% but not less than 20%; ± - biphasic effect, less than 20% but not less than 10%; 0 - no effect. | | | | | |

**Table 6.1. Cerebrovascular effect of Phenotropil (100 mg/kg) after a single intravenous injection in anesthetized cats in % compared with the background values (n=6).**

| **Drug** | **SBP (1-2 min)** | **CCA (1-2 min)** | **VPR (1-4 min)** | **SBP (at 4 min)** | **CCA (at 4 min)** | **VPR (at 5 min)** |
|---|---|---|---|---|---|---|
| Phenotropil | - 24.0±6.4 | +26.0±6.0 | - | +14±4.0 | - | - 46.0±6.4 |

**Table 6.2. Effect of Phenotropil (100 mg/kg) on neurological deficiency in rats with hemorrhagic stroke (on McGrow scale).**

| **Neurological symptoms** | **Number of animals with various neurological symptoms in %** | | |
|---|---|---|---|
| | **At 1 day after surgery** | | |
| | **Groups of animals** | | |
| | **After sham surgery** | **With stroke** | **Phenotropil** |
| Lethargy, slowness of movement | 40 | 100 | 30 |
| Weakness of limbs | 30 | 90 | 30* |
| Circling movements | 0 | 40 | 0* |
| Paresis of 1-4 limbs | 0 | 30 | 20* |
| Paralysis of 1-4 limbs | 0 | 30 | 0* |
| Mortality | 20 | 30 | 0* |

| | | | |
|---|---|---|---|
| *- significance of differences from rats with stroke at p ≤ 0.05 (χ²) | | | |

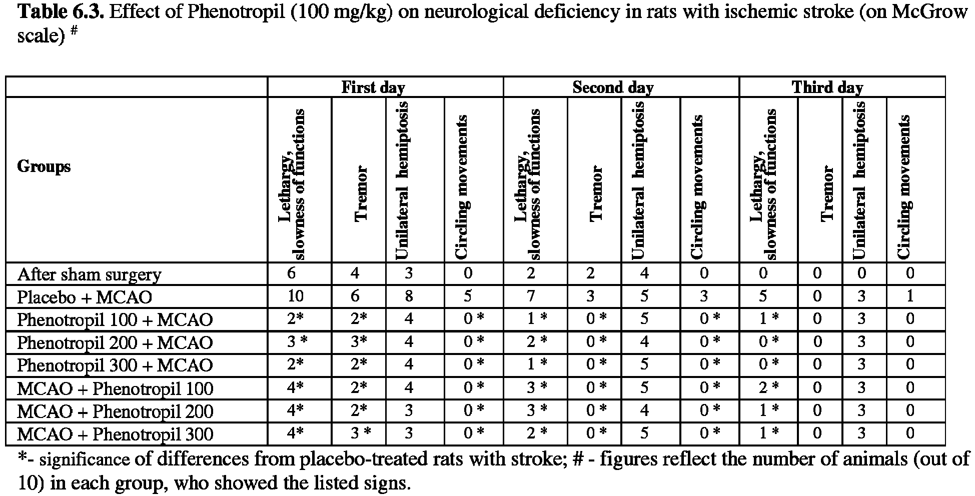

**Table 7. Effect of Phenotropil on retrograde amnesia in rats induced by electroshock and scopolamine.**

| **Group** | **Latent period of the first entry into the chamber at testing the preservation of CRPA in seconds. after 24 hours** |
|---|---|
| sodium chloride | 93.8 |
| sodium chloride + ECS | 7.4 |
| Phenotropil 25 mg/kg + ECS | 65.6* |
| sodium chloride | 110.2 |
| sodium chloride + ECS | 5.9 |
| Phenotropil 50 mg/kg + ECS | 74.4* |
| sodium chloride | 110.2 |
| sodium chloride + ECS | 9.6 |
| Phenotropil 100 mg/kg + ECS | 112.5* |
| sodium chloride | 121.9 |
| scopolamine 1 mg/kg | 49.9 |
| Phenotropil 25 mg/kg + scopolamine | 78.8* |
| Phenotropil 50 mg/kg + scopolamine | 96.0* |
| Phenotropil 100 mg/kg + scopolamine | 125.3* |

| | |
|---|---|
| * - p ≤ 0.05 compared with the experimental control group. | |

**Table 7.1. Antiamnestic effect of Phenotropil in the test of scopolamine-induced CRPA amnesia in rats 30-35 days of age.**

| **Groups** | **Training** | **At 24 hours** | |
|---|---|---|---|
| | **LP₁** | **LP₂** | **ΔLP** |
| Distilled water | 8.3 ± 2.1 | 105.1 ± 25.1 | 96.8 ± 18.2 |
| Scopolamine 1.35 mg/kg | 8.4 ± 1.9 | 14.4 ± 2.7* | 6.0 ± 0.9* |
| Phenotropil 100 mg/kg + scopolamine | 7.2 ± 2.8 | 25.2 ± 3.8** | 18.0 ± 2.9 ** |
| Phenotropil 200 mg/kg + scopolamine | 11.0 ± 3.0 | 45.2 ± 7.3** | 34.2 ± 9.0** |
| Phenotropil 300 mg/kg + scopolamine | 8.0 ± 3.0 | 82.4 ± 4.3 ** | 74.4 ± 3.7 ** |

| | | | |
|---|---|---|---|
| * - p < 0.05 compared with the control group. ** - p < 0.05 compared with scopolamine-treated group. | | | |

**Table 7.2. Effect of Phenotropil on training ability of immature under-trained rats (30 -35 days of age) in the CRPA test.**

| **Groups/mg/kg** | **Training** | **At 24 h** | |
|---|---|---|---|
| | **LP₁** | **LP₂** | **ΔLP** |
| Control - Dist. water | 10.3±1.1 | 15.1±2.3 | 4.8±1.7 |
| Phenotropil 50 mg/kg | 7.2±2.1 * | 18.4±4.6 * | 11.2±3.4 * |
| Phenotropil 100 mg/kg | 7.2±2.1 * | 36, 2±4.6* | 29.0±3.4* |
| Phenotropil 300 mg/kg | 6.8±1.2 | 45.0±7.5 * | 38.2±4.4 * |

| | | | |
|---|---|---|---|
| * - significantl compared with the control group, p < 0.05 | | | |

**Table 7.3. Effect of Phenotropil on scopolamine induced CRPA amnesia in adult male rats as a preventive and therapeutic agent at deprivation from scopolamine training.**

| **Drug** | **Dose (mg/kg)** | **Number of animals** | **LP Pre-training** | **LP of the training reproduction** | **Time spent in the dark chamber** | **Number of animals that did not enter into the dark chamber** |
|---|---|---|---|---|---|---|
| Control, naive | Dist. water | 20 | 15.9 ± 2.5 | - | - | - |
| Control - training without amnesia | Dist. water | 20 | 15.6 ± 2.1 | 127.3 ± 16.9 | 11.8 ± 4.2 | 18/20 90% |
| Control with amnesia-Scopolamine | 1.0 | 20 | 17.2 ± 2.7 | 48.2 ± 6.2 # | 118.5 ± 11.6 | 6/20 30%# |
| Phenotropil before training + Scopolamine | 100.0 | 20 | 17.0 ± 2.9 | 94.1 ± 8.7 * | 49.9 ± 6.9 | 16/20 80%* |
| Phenotropil during training and scopolamine | 100.0 | 20 | 18.4 ± 3.3 | 91.1 ± 10.1 * | 63.4 ± 12.5 | 14/20 70% * |

| | | | | | | |
|---|---|---|---|---|---|---|
| # - significant compared with the control without amnesia, p < 0.05. * - significant compared with the control with amnesia, p < 0.05. | | | | | | |

**Table 7.4. Effect of Phenotropil (100 mg/kg) on receptor binding in adult rats after a single dose**

| **Receptor** | **Control CRPA (saline solution)** | | **Scopolamine induced amnesia** | | **Phenotropil, 100 mg/kg during scopolamine induced amnesia** | |
|---|---|---|---|---|---|---|
| | **Kd, nM** | **Bmax, fmol/mg** | **Kd, nM** | **Bmax, fmol/mg** | **Kd, nM (%)** | **Bmax, fmol/mg (%)** |
| D1 striatum | 2.8±0.3 | 285.5±10.2 | 3.1±0.4 | 228.7±9.7^{#} | +21^{#}//+9.7* | -7.3^{#}//+16* |
| D2 striatum | 9.6±2.9 | 287.8±34.8 | 9.1±2.2 | 284.9±27.6 | +3.1^{#}//+8.8* | +28^{#}//+29* |
| D3 striatum | 21.7±6.2 | 34.9±4.9 | 39.1±6.5 | 56.5±6.6^{#} | +129.5^{#}//+27.4* | +101^{#}//+24* |
| 5-HT2 frontal cortex | 3.1±0.8 | 44.0±3.6 | 3.6±0.7 | 52.0±3.1^{#} | +19.4^{#}//+3.0* | +31^{#}//+11* |
| NMDA hippocampus | 15.2±3.1 | 2362±271 | 16.5±3.9 | 4565±617^{#} | +18.4^{#}//+9.0* | +69^{#}//-12* |
| nACh cortex | 43±14 | 262±68 | 52±19 | 512±134^{#} | + 39.5^{#}//+14.4* | +6^{#}//-46* |
| BDZ cortex | 5.86±0.6 | 2.52±0.09 | 5.16±0.67 | 2.09±0.09^{#} | -3.8^{#}//+9.3* | +4^{#}//+25* |

| | | | | | | |
|---|---|---|---|---|---|---|
| # - significant compared to saline-treated control, p<0.01 *- significant compared to the scopolamine treated group with CRPA amnesia (p <0.05, Fisher F-test). | | | | | | |

**Table 7.5. Effect of Phenohopil (100 mg/kg) on the characteristics of receptor binding ex vivo following subchronic administration to adult rats, (m+S.E.).**

| **Receptor** | **Control (saline solution)** | | **Phenotropil, 100 mg/kg/day** | |
|---|---|---|---|---|
| | **Kd, nM** | **Bmax, fmol/mg** | **Kd, nM** | **Bmax, fmol/mg (percentage relative to control)** |
| D1 | 1.14±0.26 | 778.8±45.3 | 1.42±0.28 | - 20 %* |
| D2 | 6.87±1.00 | 761.5±44.0 | 10.82±2.73 | + 24 %* |
| D3 | 14.70±3.44 | 32.1±4.2 | 17.84±2.71 | + 30 %* |
| 5-HT2 | 5.65±0.94 | 176.6±11.0 | 5.55±0.75 | - 18 %* |
| NMDA | 82.7±15.0 | 3350±300 | 112.0±18.4 | + 67 %* |
| nACh | 131.4±31.8 | 103.3+14.5 | 138.3±47.9 | + 58 %* |
| BDZ | 5.86±0.58 | 2.52±0.09 | 6.74±0.0 | + 29 % |

| | | | | |
|---|---|---|---|---|
| # - significant differences from control, p<0.01 *- significant antagonistic effect of Phenotropil vs. scopolamine (p <0.05, Fisher F-test). | | | | |

**Table 8. Effect of Phenotropil to Benzedrine-induced stimulation of orientation-locomotor activity in mice caused.**

| **Substance** | **Dose (mg/kg)** | **Number of stand-ups for 3 min.** | **Number of pulses for 3 min.** |
|---|---|---|---|
| Control | | 7.5 ± 1.4 | 24.8 ± 4.3 |
| Benzedrine | 3.0 | 14.2 ± 1.8* | 84.3 ± 7.9* |
| Benzedrine + Phenotropil | 50.0 | 15.9 ± 2.0* | 126.0 ± 12.4** |
| Benzedrine + Phenotropil | 100.0 | 24.2 ± 4.8** | 129.2 ± 14.2** |
| Benzedrine + Phenotropil | 300.0 | 12.1 ± 4.6** | 48.1 ± 9.4** |

| | | | |
|---|---|---|---|
| * - significance of differences compared to the control group, p <0.05. ** - significance of differences compared to the Benzedrine-treated group, p <0.05 | | | |

**Table 8.1. Evaluation of effect of Phenotropil on the duration of swimming of mice in water at 2°C with a single dose.**

| **Groups** | **Drug (mg/kg)** | **Duration of swimming (min)** |
|---|---|---|
| Control | Distilled water | 1.23 ± 0.1 |
| Phenotropil | 25 | 2.10 ± 0.17* (+70.7 %) |
| Phenotropil | 50 | 2.30 ± 0.22 * (+85 %) |
| Benzedrine | 2.5 | 1.60 ± 0.2 * (+30 %) |

| | | |
|---|---|---|
| * - significance of differences with the experimental control at P ≤ 0.05. | | |

**Table 8.1.1. Evaluation of effect of Phenotropil on the duration of swimming of mice in water at 25°C with a single dose.**

| **Groups** | **Drug (mg/kg)** | **Duration of swimming (min)** |
|---|---|---|
| Control | Distilled water | 119.90 ± 10.90 |
| Phenotropil | 25 | 161.90 ± 15.00* (+35 %) |
| Phenotropil | 50 | 209.83 ± 14.85* (+75 %) |
| Benzedrine | 2.5 | 160.70 ± 11.75* (+34 %) |

| | | |
|---|---|---|
| * - significance of differences with the experimental control at p ≤ 0.05. | | |

**Table 8.1.2. Evaluation of effect of Phenotropil on the duration of swimming of mice in water with 4-week administration.**

| **Groups and the water temperature** | **Drug (mg/kg)** | **Duration of swimming (min) at day 28** |
|---|---|---|
| **Control - 2°C** | Dist. water | 1.12 ± 0.07 |
| **Phenotropil - 2°C** | 50 | 2.34 ± 0.15* (+109 %) |
| **Control - 25°C** | Dist. water | 106.70 ± 7.13 |
| **Phenotropil - 25°C** | 50 | 222.03 ± 10.95* (+108 %) |

| | | |
|---|---|---|
| * - significance of differences with the experimental control at p ≤ 0.05. | | |

**Table 8.2. Effect of Phenotropil on behavior of mice in conflict situations (tranquilizing effect).**

| **Substance (n = 10)** | **Dose (mg/kg)** | **The time between the first and second intake of water (sec)** | **Number of water intakes for 3 min** |
|---|---|---|---|
| **Saline sol. (control)** | - | 12.4 ± 4.4 | 28.5 ± 9.3 |
| **Phenotropil** | 300 | 31.1 ± 3.4 * | 7.3 ± 3.9 * |

| | | | |
|---|---|---|---|
| * - significance of differences compared to the control group, p <0.05 | | | |

**Table 8.3. Intensity of the anticonvulsant effect of Phenotropil in convulsions induced by maximal electroshock and pharmacological convulsive agents in mice.**

| **Convulsant agent (n=10)** | **Dose**, **mg/kg** | **Mortality, %** |
|---|---|---|
| **Bicuculline** | 3.0 | 70 |
| **Bicuculline + Phenotropil** | 3.0 | |
| | 100.0 | 0* |
| | 300.0 | 0* |
| **Corazol** | 110.0 | 60 |
| **Corazol + Phenotropil** | 110.0 | |
| | 100.0 | 45* |
| | 300.0 | 35* |
| | 600.0 | 0* |
| **Thiosemicarbazide** | 18.0 | 90 |
| **Thiosemicarbazide + Phenotropil** | 18.0 | |
| | 100.0 | 60* |
| | 300.0 | 0* |
| | 600.0 | 0* |
| **MES** | - | 100 |
| **MES + Phenotropil** | 100.0 | 20* |
| | 300.0 | 0* |
| | 400.0 | 0* |
| **Picrotoxin** | 3.0 | 50 |
| **Picrotoxin + Phenotropil** | 300.0 | 0* |

| | | |
|---|---|---|
| *- significance relative to experimental control in groups, p < 0.05. | | |

**Table 8.4. Antihypoxic effect of Phenotropil on hypobaric hypoxia caused by decrease in atmospheric pressure compared with Fepiron and Piracetam in adult mice.**

| **Drug** | **Dose (mg/kg)** | **Duration of life (min)** |
|---|---|---|
| **(n=10)** | | |
| Control (Dist. water) | - | 2.3 ± 0.2 |
| Fepiron | 25 | 2.3 ± 0.2 |
| | 50 | 2.7 ± 0.2 |
| | 100 | 3.4 ± 0.2 * |
| Piracetam | 600 | 1.5 ± 0.6 # |
| | 900 | 4.0 ± 0.6 * |
| | 2000 | 4.2 ± 0.4 * |
| Phenotropil | 50 | 3.8 ± 0.5 * |
| | 100 | 5.7 ± 1.2 * |
| | 300 | 15.9 ± 0.4 * |

| | | |
|---|---|---|
| * - significance of differences compared to the control group, p < 0.05. # - negative effect, the significance of differences relative to the control group, p < 0.05. | | |

**Table 8.5. Effect of Phenotropil on pain sensitivity of mice by the "hot plate" method.**

| **Substance** | | **Latent period, min.** | | |
|---|---|---|---|---|
| **(n=10)** | **Dose (mg/kg)** | **30 min** | **60 min** | **120 min** |
| **Saline sol. (control)** | | 10.5 ± 0.9 | 10.8 ± 0.6 | 11.1 ± 0.8 |
| Phenotropil | 50.0 | 12.0 ± 1.1* | 11.7 ± 0.7 * | 12.3 ± 0.8 * |
| | 100.0 | 11.8 ± 0.7 | 13.5 ± 1.4 * | 14.6 ± 1.4 * |
| | 300.0 | 18.0 ± 1.0* | 18.2 ± 0.8* | 22.2 ± 1.0* |

| | | | | |
|---|---|---|---|---|
| * - significance compared to the control group, p < 0.05. | | | | |

**Table 9. Effect of Phenotropil on duration of life of 7-day-old rats on the hypobaric hypoxia model with the "ascension" to 11000 m (doses of 50 and 100 mg/kg) and to 12000 m (300 mg/kg).**

| **Substance** | **Dose, mg/kg** | **Duration of life of animals in minutes** |
|---|---|---|
| Control | Dist. water | 6.0 ± 0.7 |
| Phenotropil | 50 | 6.9 ± 0.7* |
| Control | Dist. water | 4.9 ± 0.4 |
| Phenotropil | 100 | 6.9 ± 0.7* |
| Control | Dist. water | 5.9 ± 0.7 |
| Phenotropil | 300 | 7.3 ± 0.7* |

| | | |
|---|---|---|
| *- significance of differences between control and experiment groups at p <0.05 (t-test) | | |

**Table 9.1. Effect of Phenotropil on duration of life of 7-day-old rats on a model of hypoxia with hypercapnia in the pressurized cell.**

| **Substance** | **Dose, mg/kg** | **Duration of life of animals in minutes** |
|---|---|---|
| Control | Saline sol. | 75.6±11.9 |
| Phenotropil | 50 | 83.3 ± 7.9 |
| Control | Saline sol. | 110.2 ± 5.3 |
| Phenotropil | 100 | 123.2 ± 2.9* |

| | | |
|---|---|---|
| *- significance of differences between control and experiment groups at p <0.05 (t-test) | | |

**Table 10. Study of antidepressant action of Phenotropil on duration of immobilization of rat pups in conditions of forced swimming by Porsolt.**

| **Group (mg/kg)** | **Duration of immobilization (sec)** | % |
|---|---|---|
| Control | 426.85 ± 32.13 | 100.0 |
| Phenotropil 100 | 86.35 ± 51.97 * | - 79.77 * |
| Phenotropil 200 | 118.72 ± 80.04 * | - 72.19 * |

| | | |
|---|---|---|
| *- difference with control groups, significant at P <0.05. | | |

**Table 10.1. The antidepressant activity of Phenotropil measured by the Nomura method of forced swimming of rat pups with freely rotating wheels.**

| **Group** (**mg**/**kg**) | **Number of revolutions** | **%** |
|---|---|---|
| Control | 110.3 ± 22.67 | 100.0 |
| Phenotropil 100 | 175.4 ± 22.98 * | + 59.0 * |
| Phenotropil 200 | 143.4 ± 33.14 * | + 30.0 * |

| | | |
|---|---|---|
| *- difference with the control group, significantly at p <0.05. | | |

**Table 10.2. Evaluation of antidepressant activity of Phenotropil by the forced swimming test in adult rats in a vessel with wheels by Nomura.**

| **Groups** | **Drug (mg/kg)** | **Number of revolutions of wheels for 10 minutes** |
|---|---|---|
| Control | Distilled water | 100 |
| Phenotropil | 10 | + 15.4 * |
| Phenotropil | 25 | + 67 * |
| Phenotropil | 50 | + 104.2 * |
| Phenotropil | 100 | + 142.4 * |

| | | |
|---|---|---|
| * - significance of differences with the experimental control at p ≤ 0.05. | | |

**Table 11. Effect of 45-day course of Phenotropil (100 mg/kg) on alcohol dependence in rats (A) caused a 4-month alcoholism, in the days of the free choice between alcohol and water.**

| **Groups** | **Alcohol (ml/day)** | **Water (ml/day)** |
|---|---|---|
| A - control test at the 45th day | 26.6 ± 7.6 | 19.6 ± 3.6 |
| Phenotropil at the 45th day of administration | 10.5 ± 4.2* | 24.0 ± 5.9 * |
| test of after-effect of Phenotropil | | |
| 1st day | 3.3 ± 1.1 | 26.7 ± 3.9 |
| 2nd day | 0.0 ** | 20.2 ± 3.3 |
| 3rd day | 0.0 ** | 21.2 ± 3.5 |

| | | |
|---|---|---|
| *- significant relative to the third day of control, p < 0.05. **- significant relative to the control on the respective day, p < 0.05. | | |

**Table 11.1. Effect of Phenotropil on alcohol consumption in abstinent alcoholics rats (A), after 7-month alcoholism and two days of abstinence with free choice between alcohol and water, starting from the third day.**

| **Groups** | | **Alcohol (ml/day)** |
|---|---|---|
| A - control, abstinence | day 3 | 11.6 ± 2.1 |
| | day 4 | 9.2 ± 2.1 (-20.4 %) * |
| | day 14 | 32.8 ± 5.3 (+ 183.0 %) * |
| A - Phenotropil 200 mg/kg | day 3 | 4.8 ± 0.8 (-58.6 %) * |
| | day 4 | 2.4 ± 0.7 (-74.0 %) ** |
| | day 14 | 0.0 ** |

| | | |
|---|---|---|
| *- significant relative to the third day of control, p < 0.05. **- significant relative to the control on the respective day, p < 0.05. | | |

**Table 12. Anti-inflammatory activity of Phenotropil (100 mg/kg) with intragastric administration and topical application.**

| **Groups of animals** | **Effect (%)** |
|---|---|
| Carrageenan + Water | 100 |
| Phenotropil + carrageenan | - |
| Vaseline + formalin | 100 |
| Phenotropil (2 hours before) + formalin | -79.9 % |
| Phenotropil (1 hour before) + formalin | - |

| | |
|---|---|
| *- significance of differences between control and experimental group with p ≤ 0.05 (t-test) | |

**Table 12.1. Effect of Phenotropil on mycobacteria (MTB) colony growth.**

| **Concentration of Phenotropil (ug/ml)** | **Mean number of MTB colonies** | **Concentration of Phenotropil (ug/ml)** | **Mean number of MTB colonies** |
|---|---|---|---|
| 0.031 | contiguous growth | 1.0 | 67 |
| 0.063 | contiguous growth | 1.25 | 74 |
| 0.125 | 107 | 2.0 | 79 |
| 0.250 | 97 | 5.0 | 90 |
| 0.310 | 83 | 10.0 | 123 |
| 0.500 | 70 | 50.0 | contiguous growth |
| 0.630 | 65 | 100 | contiguous growth |
| Not treated control - contiguous growth of MTB colonies | | | |

**Table 13. Optical density and plasma MDA content in Wistar, August and outbred rats.**

| **Groups of animals** | **Optical density of the samples (nm/minute)** | **MDA content (mcmol/l)** |
|---|---|---|
| Control - Wistar | 0.0066074 ± 0.0004045 * | 0.68 ± 0.0410 * |
| Control - August | 0.0038220 ± 0.0005050 * | 0.39 ± 0.0516 * |
| Control - outbred | 0.0052150 ± 0.00045475 | 0.54 ± 0.0467 |

| | | |
|---|---|---|
| *- significant relative to control, p < 0.01 | | |

**Table 13.1. Effect of single administration of Phenotropil on MDA content in Wistar rats after a single Ns-exposure.**

| **Wistar rats groups** | **Optical density of the samples (%)** | **MDA content (%)** |
|---|---|---|
| Control - saline solution | 100.0 | 100.0 |
| Ns + saline solution | - 12.2 * | - 11.8 * |
| Phenotropil 200 mg/kg | + 66.5 * | + 66.2 * |
| Ns + Phenotropil 200 mg/kg | - 6.0 * | - 6.0 * |

| | | |
|---|---|---|
| * - significance of differences with the baseline control at p ≤ 0.01. | | |

**Table 13.2. Effect of single administration of Phenotropil on MDA content in August rats after a single Ns-exposure**

| **August rats groups** | **Optical density of the samples (%)** | **MDA content (%)** |
|---|---|---|
| Control - saline solution | 100.0 | 100.0 |
| Ns + saline solution | + 186.2 * | + 187.0 * |
| Phenotropil 200 mg/kg | + 188.4 * | + 190.0 * |
| Ns + Phenotropil 200 mg/kg | + 220.3 * | + 223.0 * |

| | | |
|---|---|---|
| * - significance of differences with the baseline control at p ≤ 0.01. | | |

**Table 13.3. Effect of Phenotropil (200 mg/kg) measured by plasma MDA in rats in normal and destress conditions (three-time exposure).**

| **Groups** | **MDA (km)** | **(km %)** |
|---|---|---|
| **Oubread - control** | 1.0 | 100 |
| Wistar - control | 1.26 * | + 26 % * |
| August - control | 0.72 * | - 28 % * |
| **Oubread - Ns** | 1.6 | 100 |
| Wistar - Ns | 1.1* | - 31.3 % * |
| August - Ns | 2.1 * | +31.3 % * |
| **Oubread - Phenotropil** | 2.1 | 100 |
| Wistar - Phenotropil | 2.1 | 0 |
| August - Phenotropil | 2.1 | 0 |
| **Oubread - Ns + Phenotropil** | 1.8 | 100 |
| Wistar - Ns + Phenotropil | 1.2 * | - 33.3 % * |
| August - Ns + Phenotropil | 2.3 * | + 27.8 % * |

| | | |
|---|---|---|
| * - significance of differences with groups of outbred rats at p ≤ 0.01. | | |

**Table 13.4. Effect of Phenotropil (200 mg/kg) measured by plasma MDA in rats in destress conditions (seven days).**

| **Groups** | **MDA (km)** | **(km %)** |
|---|---|---|
| Oubread - control | 1.0 | 100 |
| Wistar - control | 1.26 * | + 26 * |
| August - control | 0.72 * | -28 * |
| Oubread - Ds - saline solution | 3.7 | + 270 ** |
| Wistar - Ds - saline solution | 3.2 | + 193.7 ** |
| August - Ds - saline solution | 0.5 | - 30.5 ** |
| Oubread - Ds + Phenotropil | 1.0 | - 73.0 *** |
| Wistar - Ds + Phenotropil | 1.1 * | - 65.6 *** |
| August - Ds + Phenotropil | 0.8 * | + 60.0 *** |

| | | |
|---|---|---|
| * - significance of differences with the outbred rats group at p ≤ 0.01. ** - significance of differences with pure strain control at p ≤ 0.01. *** Significance of differences the of pure strain group relative to the experiments 1 control (saline) at P≤0.05. | | |

**Table 13.5. Effect of Phenotropil on serum electrolytes in rats at 4-week administration (mEq/l).**

| **Group** | **K** | **Ca** | **Na** |
|---|---|---|---|
| Control - Dist. water | 3.41 ± 0.17 | 4.28 ± 0.07 | 123.4 ± 2.67 |
| Phenotropil 50 mg/kg | 3.15 ± 0.15 | 4.21 ± 0.23 | 127.3 ± 2.71 |
| Phenotropil 300 mg/kg | 3.44 ± 0.20 | 4.69 ± 0.24 | 125.7 ± 3.43 |

| | | | |
|---|---|---|---|
| * - significance of differences with the outbred rats group at p ≤ 0.01 | | | |

**Table 13.6. Effect of Phenotropil (200 mg/kg) on the expression of HSP32 measured by km in outbred rats after a single administration.**

| **Groups** | **Cortex HSP32 (km)** | **Hippocampus HSP32 (km)** | **Hypothalamus HSP32 (km)** | **Total (%)** |
|---|---|---|---|---|
| Control - saline solution | 1.0 | 0.7 | 0.6 | 100 |
| Control - Phenotropil | 1.5 * | 0.5 * | 1.2 * | + 38.5 * |
| Ns - saline solution | 1.7 * | 0.6 | 0.9 * | + 38.5 * |
| Ns - Phenotropil | 1.9 * | 1.0 * | 1.4 * | + 86.1 * |

| | | | | |
|---|---|---|---|---|
| * - significance of differences with the saline-treated group at p ≤ 0.05. | | | | |

**Table 13.7. Effect of Phenotropil (200 mg/kg) on expression of HSP32 measured by km in Wistar rats after a single administration.**

| **Groups** | **Cortex HSP32 (km)** | **Hippocampus HSP32 (km)** | **Hypothalamus HSP32 (km)** | **Total (%)** |
|---|---|---|---|---|
| Control - outbred | 1.0 | 0.7 | 0.6 | 100 |
| Control - Wistar | 0.9 | 0.3 * | 0.7 * | - 17.7 * |
| Control - Phenotropil | 1.7 * | 0.4 * | 1.8 * | + 68.8 * |
| Ns - saline solution | 1.1 | 0.2 * | 0.8 * | - 9.1 |
| Ns - Phenotropil | 1.9 * | 0.6 | 1.7 * | +81.8 * |

| | | | | |
|---|---|---|---|---|
| * - significance of differences with the saline-treated group at p ≤ 0.05. | | | | |

**Table 13.8. Effect of Phenotropil (200 mg/kg) on expression of HSP32 measured by km in August rats after a single administration.**

| **Groups** | **Cortex HSP32 (km)** | **Hippocampus HSP32 (km)** | **Hypothalamus HSP32 (km)** | **Total (%)** |
|---|---|---|---|---|
| Control - outbred | 1.0 | 0.7 | 0.6 | 100 |
| Control - August | 1.1 | 1.1 * | 0.5 | + 16.9 * |
| Control - Phenotropil | 1.4 * | 0.7 | 0.6 * | + 16.9 * |
| Ns - saline solution | 2.4 * | 1.1 * | 1.0 * | + 94.8 * |
| Ns - Phenotropil | 2.0 * | 1.5 * | 1.1 * | + 98.7 * |

| | | | | |
|---|---|---|---|---|
| * - significance of differences with the saline-treated group at p ≤ 0.05. | | | | |

**Table 14. Distribution of Phenotropil by internal organs of rats.**

| **Time after administration, h** | **Contents of Phenotropil per µg/g of organ** | | | |
|---|---|---|---|---|
| | brain | heart | liver | kidney |
| 0.25 | 1.6 ± 0.2 | 3.7 ± 0.6 | 25.6 ± 2.4 | 33.2 ± 3.4 |
| 0.50 | 2.0 ± 0.3 | 7.9 ± 0.9 | 30.1 ± 4.3 | 37.5 ± 4.0 |
| 1.00 | 6.4 ± 0.8 | 10.1 ± 1.0 | 75.8 ± 8.2 | 55.7 ± 5.8 |
| 1.50 | 4.0 ± 0.6 | 14.3 ± 1.3 | 67.7 ± 7.1 | 50.4 ± 6.0 |
| 2.00 | 1.2 ± 0.3 | 12.6 ± 1.2 | 59.6 ± 5.4 | 37.2 ± 4.1 |
| 4.00 | 0.4 ± 0.2 | 8.4 ± 1.0 | 30.2 ± 3.4 | 28.0 ± 3.2 |
| 6.00 | - | 4.4 ± 0.8 | 17.7 ± 2.1 | 16.0 ± 2.1 |
| 8.00 | - | - | 11.4 ± 1.6 | 10.1 ± 1.3 |

| | | | | |
|---|---|---|---|---|
| * - significance of differences with the saline-treated group at p ≤ 0.05. | | | | |

**Table 14.1. Effect of Phenotropil on HSP70 in the myocardium of Wistar rats under Ns-state conditions after single administration.**

| **Groups (mg/kg)** | | **HSP 70 ng/mg total protein** |
|---|---|---|
| Control, untreated | | 0.11 ± 0.007 |
| Ns - Control (Dist. water) | | 0.29 ±,018 * |
| Phenotropil | 25 | 0.18 ± 0.011 * |
| | 50 | 0.20 ± 0.014 * |
| | 100 | 0.19 ± 0.008 * |
| | 300 | 0.41 ± 0.016 * |
| Ns + Phenotropil | 25 | 0.40 ± 0.02 ** |
| | 50 | 0.41 ± 0.03 ** |
| | 100 | 0.47 ± 0.02 ** |
| | 300 | 0.55 ± 0.03 ** |

| | | |
|---|---|---|
| * - significant at p ≤ 0.001 relative to the untreated control group, ** - significant at p ≤ 0.001 relative to the control group of Sn-state | | |

**Table 15. Evaluation of modulatory-adaptogenic effect of Phenotropil and its enantiomers under the Ds-conditions.**

| **Groups** | | S/T | S/A | T/A |
|---|---|---|---|---|
| Control, untreated | | 1.1 ± 0.1 | 9.9 ± 1.6 | 7.7 ± 1.1 |
| Ds - saline solution | | 6.2 ± 0.3 | 7.3 ± 0.1 | 2.5 ± 1.3 |
| Ds - Seduxen | 2 mg/kg | 3.6 ± 0.1* | 7.6 ± 0.2 | 2.2 ± 1.4 |
| Ds - Piracetam | 200 mg/kg | 7.4 ± 0.7* | 12.1 ± 1.5* | 1.6 ± 0.2* |
| Ds - Piracetam | 400 mg/kg | 7.2 ± 0.4* | 10.8 ± 1.3* | 1.5 ± 0.1* |
| Ds - Piracetam | 600 mg/kg | 4.0 ± 0.1* | 10.2 ± 0.1* | 2.6 ± 0.1 |
| Ds - Phenibut | 100 mg/kg | 4.7 ± 0.1* | 8.0 ± 0.1 | 1.7 ± 0.1* |
| Ds - Phenibut | 50 mg/kg | 7.6 ± 0.2* | 10.2 ± 0.3* | 1.3 ± 0.6* |
| Ds - Phenibut (-) | 50 mg/kg | 4.8 ± 0.3* | 12.0 ± 0.3* | 2.5 ± 0.3 |
| Ds - Phenibut (+) | 50 mg/kg | 4.8 ± 0.1* | 13.0 ± 0.1* | 2.7 ± 0.1 |
| Ds - Fepiron | 200 mg/kg | 9.5 ± 0.9* | 9.4 ± 0.4* | 1.0 ± 0.1* |
| Ds - Fepiron | 100 mg/kg | 5.4 ± 0.3* | 9.0 ± 0.3* | 1.6 ± 0.3* |
| Ds - Fepiron | 50 mg/kg | 5.4 ± 0.3* | 11.3 ± 0.3* | 2.1 ± 0.2 |
| Ds - Fepiron (-) | 200 mg/kg | 4.9 ± 0.7* | 9.0 ± 0.5* | 1.9 ± 0.3 |
| Ds - Fepiron (+) | 200 mg/kg | 5.4 ± 0.1* | 11.3 ± 0.4* | 2.1 ± 0.6 |
| Ds - Phenotropil | 100 mg/kg | 1.2 ± 0.2* | 8.8 ± 1.2* | 5.5 ± 1.1* |
| Ds - Phenotropil | 50 mg/kg | 1.6 ± 0.1* | 8.2 ± 1.2* | 6.6 ± 1.1* |
| Ds - Phenotropil | 25 mg/kg | 2.2 ± 0.1* | 7.6 ± 1.2 | 5.5 ± 1.1* |
| Ds - Phenotropil (-) | 25 mg/kg | 2.7 ± 0.1* | 8.1 ± 1.5* | 4.3 ± 0.3* |
| Ds - Phenotropil (+) | 25 mg/kg | 3.2 ± 0.3* | 7.3 ± 0.4 | 4.4 ± 0.4* |

| | | | | |
|---|---|---|---|---|
| * - significance of differences relative to experimental control at p <0.05 (S - Spleen, T - thymus, A - adrenals) mg/100 g baseline body weight in the intersystem ratio. | | | | |

**Table 15.1. Evaluation anti-ulcerogenic activity of Phenotropil.**

| **Groups of animals** | **Number in%** |
|---|---|
| Control - 5.0 % glucose solution | 100 |
| Phenotropil 25 mg/kg + 5.0 % glucose solution | - 42 * |
| Phenotropil 50 mg/kg + 5.0 % glucose solution | - 63 * |
| Phenotropil 100 mg + 5.0 % glucose solution | - 80 * |

| | |
|---|---|
| * - significance of differences with the untreated control at p ≤ 0.05. | |

**Table 16. Effect of achiral and racemic pyrrolidones on receptor and psycho-emotional reactions of mice evoked by electrical pain stimulation on the electrical frounf.**

| **Groups** | | TA (%) | TFC (%) | TF (%) |
|---|---|---|---|---|
| Control - saline solution | | 100.0 | 100.0 | 100.0 |
| Phenotropil: | 25 | + 73.6 * | + 40.0 * | + 8.7 |
| | 50 | + 65.3 * | +76.0 * | + 4.0 |
| | 100 | + 57.0 * | + 82.0 * | + 7.6 |
| Control - saline solution | | 100.0 | 100.0 | 100.0 |
| Fepiron: | 25 | 0.0 | - 5.7 | + 48.0 * |
| | 50 | + 5.0 | + 11.4* * | + 84.0 * |
| | 100 | +12.5 * | + 2.8 | + 100.0 * |
| Control - saline solution | | 100.0 | 100.0 | 100.0 |
| Piracetam: | 100 | - 43.6 * | - 23.5 * | - 11.3 * |
| | 200 | -48.1 * | - 47.0 * | - 15.7 * |
| | 400 | - 20.0 * | + 5.0 | - 7.4 |

| | | | | |
|---|---|---|---|---|
| * - significance of differences relative to control at p ≤ 0.05. | | | | |

**Table 17. Effect of Phenotropil on the average life span of mice.**

| **Groups of animals** | **Average life span (number of days in %)** |
|---|---|
| Control untreated | 100 |
| Control experimental | - 2.84 |
| Phenotropil 50 mg/kg | + 12 * |
| Phenotropil 100mg/kg | + 20.6 * |
| Phenotropil 200 mg/kg | + 9.3 |
| Phenotropil 300 mg/kg | + 16.24 * |

| | |
|---|---|
| * - significance of differences relative to the experimental control at p ≤ 0.05. | |

**Table 18. Effect of Phenotropil the maximum life span of mice.**

| **Groups of animals** | **The maximum life span** (**number of days in %)** |
|---|---|
| Control untreated | 100 |
| Control - Dist. water | - 3.57 |
| Control 5.0 % glucose solution | - 2.59 |
| Phenotropil 25 mg/kg + 5.0 % glucose solution | + 20.1 * |
| Phenotropil 50 mg/kg + 5.0 % glucose solution | + 15.0* |
| Phenotropil 100 mg + 5.0 % glucose solution | + 20.8 * |

| | |
|---|---|
| *- significance of differences with the untreated control at p ≤ 0.05. | |

**Table 18.1. Effect of Phenotropil on spontaneous tumors in mice.**

| **Groups of animals** | **Number, in %** |
|---|---|
| Control untreated | 100 |
| Control (Dist. water) | 101 |
| Control (5.0 % glucose solution) | 98.3 |
| Phenotropil 25 mg/kg + 5.0 % glucose solution | 57.0* |
| Phenotropil 50 mg/kg + 5.0 % glucose solution | 74.5* |
| Phenotropil 100 mg + 5.0 % glucose solution | 42.2* |

| | |
|---|---|
| * - significance of differences with the untreated control at p ≤ 0.05. | |

**Table 18.2. Effect of Phenotropil on body weight in obese rats.**

| **Groups of animals** | Body weight (%) |
|---|---|
| Control - Dist. water | 100 |
| Phenotropil 25 mg/kg | - 14.0 |
| Phenotropil 50 mg/kg | - 25.0 * |
| Phenotropil 100 mg/kg | - 33.3 * |
| Phenotropil 200 mg/kg | - 35.0 * |
| Phenotropil 300 mg/kg | - 32.0 * |

| | |
|---|---|
| * - significance of differences with control at p ≤ 0.05. | |

**Table 19. Effect of Phenotropil on the fertility of mice.**

| **Groups of animals** | **Fertility (% of animals in the group)** |
|---|---|
| Control untreated | 0 |
| Experimental control | 10.0 |
| Phenotropil 100 mg/kg | 80.0 * |

| | |
|---|---|
| * - significance of differences with the experimental control group at p < 0.05. | |

**Table 20. Therapy effect of Phenotropil on cAMP level in human plasma in primary dysmenorrhoea during the first phase of the cycle.**

| **Groups** | **Dose (mg)** | **cAMP level (nmol/l)** |
|---|---|---|
| Control | - | 12.54 ± 0.72 |
| Background - dysmenorrhea | - | 20, 97 ± 0.93 * |
| Phenotropil - dysmenorrhea | 50 | 14.35 ± 0.87** **(-32%)** |
| | 100 | 12.77 ± 1.24** **(-39%)** |

| | | |
|---|---|---|
| * - significance of differences with control at p ≤ 0.05. ** - significance of differences with the background at p ≤ 0.05. | | |

**Table 20.1. Effect of Phenotropil on cAMP level in the blood plasma of mice one hour after a single injection.**

| **Substance (mg/kg)** | **cAMP level (pikomol/50 µl plasma)** |
|---|---|
| Control - saline solution | 3.77 ± 0.40 |
| Phenotropil 10 | 4.56 ± 0.67* (+ 21 %) * |
| Phenotropil 50 | 6.24 ± 0.81* (+ 65.5 %) * |
| Phenotropil 100 | 7.40 ± 0.62* (+ 96.3 %) * |

| | |
|---|---|
| * - significance of differences with control at p < 0.05. | |

**Table 21. Effect of Phenotropil on the cytokine profile after one month of course treatment in asthenoneurotic disorders in humans, at three months after treatment.**

| | **Before treatment** | **After treatment** | **Group of normal control** | **At 3 months after treatment** |
|---|---|---|---|---|
| IL-1 | 1.71 ± 0.04 | 1.65 ± 0.03 | 1.64 ± 0.04 | 1.63 ± 0.04 |
| IL-2 (×10) | 1.92 ± 0.06* | 2.09 ± 0.04** | 2.20 ± 0.03 | 2.21 ± 0.04** |
| IL-3 (×10) | 1.89 ± 0.07* | 2.13 ± 0.05** | 2.22 ± 0.03 | 2.23 ± 0.06** |
| IL-6 | 3.19 ± 0.07* | 3.55 ± 0.15** | 3.79 ± 0.04 | 3.80 ± 0.03** |
| α-TNF (×100) | 9.68 ± 0.52* | 8.56 ± 0.60** | 7.94 ± 0.43 | 7.92 ± 0.54** |
| α-INF | 4.66 ± 0.23* | 4.18 ± 0.06** | 4.00 ± 0.02 | 4.11 ± 0.05** |

| | | | | |
|---|---|---|---|---|
| * - significance of differences with the normal control group, p < 0.05. ** - significance compared to background tests before treatment, p < 0.05. | | | | |

**Table 22. Evaluation anti-kinetosis properties of Phenotropil.**

| **Groups** | **VVE (minutes)** | **VVR (points)** | **NEE (min)** | **km** |
|---|---|---|---|---|
| Placebo | 4.27 ± 0.7 | 7.2 ± 1.1 | 300 ± 60 | 0.44 |
| Phenotropil 25 - 300 mg | 7.70 ± 0.6 * | 2.3 ± 0.9 * | 22.5 ± 7.5 | 0.87 * |

| | | | | |
|---|---|---|---|---|
| * - significance of differences with the normal control group, p < 0.01. | | | | |

**Table 23. Effect of Phenotropil when used topically on the periodontal, enamel and dentin condition in human teeth.**

| **Groups of subjects** | **Efficacy on a 4-point scale** |
|---|---|
| Control - dist. water | 0 |
| Phenotropil 1% | 2* |
| Phenotropil 2 % | 3* |
| Phenotropil 3 % | 3* |

| | |
|---|---|
| * - significance of differences with control at p ≤ 0.05. | |

**Table 23.1. Effect of Phenotropil on the content of MDA in human saliva after topical course treatment.**

| **Groups of subjects** | **MDA content (nmol/l)** |
|---|---|
| Control - dist. water | 0.043 ± 0.0261 |
| Phenotropil 100 me per 100 ml water | 0.026 ± 0.0415 * |

| | |
|---|---|
| * - significance of differences with control at p ≤ 0.05. | |

**Table 24. Effect of an aqueous solution of Phenotropil on human skin aging after topical course treatment.**

| **Groups** | **Efficacy in %** |
|---|---|
| Baseline control | 100.0 |
| Phenotropil 0.1 % | 212.0* |

| | |
|---|---|
| * - significance of the differences with the baseline control at p ≤ 0.05. | |

**Table 24.1. Effect of Phenotropil (cream) on human skin aging after topical course treatment.**

| **Groups** | **Efficacy in %** |
|---|---|
| Baseline control | 100.0 |
| Baseline control - placebo | 80.0 * |
| Phenotropil 50 mg per 100 g of the base | 140.0 * |
| Phenotropil 100 mg per 100 g of the base | 180.0 * |
| Phenotropil 200 mg per 100 g of the base | 187.0 * |
| Phenotropil 300 mg per 100 g of the base | 200.0 * |

| | |
|---|---|
| * - significance of the differences with the baseline control at p ≤ 0.05. | |

**Table 24.2. Effect of Phenotropil, prepared on the basis of the "Lux" cream, on human skin aging, after topical course treatment.**

| **Groups** | **Efficacy in %** |
|---|---|
| Baseline control | 100.0 |
| Control "Lux" | 110.0 |
| Phenotropil 50 mg + "Lux" 100 g | 167.0 * |
| Phenotropil 100 mg + "Lux" 100 g | 210.0 * |
| Phenotropil 200 mg + "Lux" 100 g | 240.0 * |
| Phenotropil 300 mg + "Lux" 100 g | 280.0 * |

| | |
|---|---|
| * - significance of the differences with the baseline control at p ≤ 0.05. | |

**Table 24.3. Effect of Phenotropil, prepared on the basis of the "Gerantol" cream, on human skin aging, after topical course treatment**

| **Groups** | **Efficacy in % relative to baseline control** |
|---|---|
| Baseline control | 100.0 |
| Control "Gerantol" | 118.0 * |
| Phenotropil 50 mg + "Gerantol" 100 g | 132.0 * |
| Phenotropil 100 mg + "Gerantol" 100 g | 205.0 * |
| Phenotropil 200 mg + "Gerantol" 100 g | 251.0 * |
| Phenotropil 300 mg + "Gerantol" 100 g | 277.0 * |

| | |
|---|---|
| * - significance of the differences with the baseline control at p ≤ 0.05 | |

**Table 24.4. Effect of Phenotropil prepared on the basis of the "Vecher" cream, on human skin aging, after topical course treatment.**

| **Groups of subjects** | **Efficacy expressed in %** |
|---|---|
| Baseline control | 100 |
| Control - "Vecher" | 110 |
| Phenotropil 100 mg + "Vecher" 100 g | 317* |
| Phenotropil 200 mg + "Vecher" 100 g | 280* |
| Phenotropil 300 mg + "Vecher" 100 g | 249* |

| | |
|---|---|
| * - significance of the differences with the baseline control at p ≤ 0.05 | |

**Table 24.5. Effect of Phenotropil in diluted effective dose per 100 g of the base, after topical course treatment, on the age-related changes of blood vessels and joints.**

| **Groups of subjects** | **Efficacy expressed in %** |
|---|---|
| Baseline control | 100 |
| Cream base | 108 |
| Phenotropil 100 mg + 100 g of cream base | 211* |
| Phenotropil 200 mg + 100 of cream base | 219* |
| Phenotropil 300 mg + 100 g of cream base | 174* |

| | |
|---|---|
| * - significance of the differences with the baseline control at p ≤ 0.05. | |

**Table 25. Effect of Phenotropil on human skin in different types of seborrhea.**

| **Groups of subjects** | **Efficacy expressed in points on a 4-point scale** |
|---|---|
| Baseline control | 0 |
| DF - topically Phenotropil 0.1 % aqueous solution | 2* |
| OF - topically Phenotropil 0.1 % aqueous solution | 2* |
| MF - topically Phenotropil 0.1 % aqueous solution + Phenotropil 100 mg orally | 3* |
| MF - oral Phenotropil 100 mg | 1* |

| | |
|---|---|
| * - significance of differences with control at p ≤ 0.05. | |

**Table 25.1. Effect of Phenotropil on acne rush during hormonal alteration**

| **Groups of subjects** | **Efficacy expressed in %** |
|---|---|
| Baseline control | 100 |
| Oily skin - topically Phenotropil 0.1 % aqueous solution | - 79 * |
| Dry skin - topically Phenotropil 0.1 % aqueous solution | - 84 * |

| | |
|---|---|
| * - significance of differences with control at p ≤ 0.05. | |

**Table 26. Effect of Phenotropil on the body weight of adult humans.**

| **Groups of subjects** | **Body weight (%)** |
|---|---|
| Background (not treated) | 100 |
| Control - placebo | + 8.0 |
| Phenotropil 100 mg/day | -12.0 * |
| Phenotropil 200 mg/day | -15.0 * |
| Phenotropil 300 mg/day | - 17.8 * |

| | |
|---|---|
| * - significance of differences with the background values at p <0.05. | |

**Table 26.1. Effect of Phenotropil on daily diuresis in adult humans.**

| **Groups of subjects** | **Daily urine volume (ml/day, %)** |
|---|---|
| Background (not treated) | 100 |
| Control - placebo | 98 |
| Phenotropil 25 mg/day | 130 * |
| Phenotropil 50 mg/day | 137 * |
| Phenotropil 100 mg/day | 137 * |

| | |
|---|---|
| * - significance of differences with placebo-treated control at p <0.05. | |

**Table 27. Effect of Phenotropil in migraine attacks in humans when administered intranasally.**

| **Groups of subjects** | **Efficacy** |
|---|---|
| Phenotropil 0.5 mg x 3 times per day | 5/10 - 100 % efficacy |
| Phenotropil 0.5 mg x 3 times per day | 3/10 - 30 % partial effect |
| Phenotropil 0.5 mg x 3 times per day | 2/10 - 20 % no effect |

### References:

1. Inventor's certificate SU N° 797219, A61 K21/40 "N-carbamoylmethyl-4-phenyl-2-pyrrolidone having antihypertensive activity" with priority date 08.05.1979 (published 25.07.1995).
2. Patent RU # 2050851 C1, priority date 28.08.1990.
3. Patent RU # 2232578 C1, priority date 10.04.2003.
4. Patent RU # 2240783 C1, priority date 17.07.2003.
5. Patent RU # 2329804 C2, priority date 28.03.2006.
6. Patent RU # 2391976 C2, priority date 18.10.2007.
7. Patent RU # 2327458 C1, priority date 19.02.2007.
8. Guide on experimental (preclinical) study of new pharmacological substances, edited by R.U. Habrieva, Moscow, Medicine, 2005.
9. Akhapkina V.I., Clinical and experimental justification of the new nootropic drug Phenotropil in emergency medicine// Proc. X Conference on Space Biology and Aerospace Medicine. Moscow, 1994, p.362-363.
10. Akhapkina V.I., Pharmacological correction of functional state in the extreme conditions of human activity // Abstracts of the XI Conference on Space Biology and Aerospace Medicine, Volume I. Moscow, Russia, 1998, p.63-64.
11. Akhapkina V.I., Goncharov I.B., Some results and perspectives of pharmacological support of manned spaceflight // Abstracts of the XI Conference on Space Biology and Aerospace Medicine, Volume I. Moscow, Russia, 1998, pp.64- 66.
12. Akhapkina V.I., Portugalov S.N. Results of clinical studies of the effect of the new nootropic drug Phenotropil on physical performance // Proc. of XII Conference on Space Biology and Aerospace Medicine. Moscow, Russia, 2002, p.35.
13. Akhapkina V.I., Berlyand AS Pharmacokinetic studies of Phenotropil // Proceedings of the XII Conference on Space Biology and Aerospace Medicine. Moscow, Russia, 2002, p.34.
14. Akhapkina V.I. Experimental and Clinical Pharmacology of Phenotropil // Abstracts of the XI Russian National Congress "Man and medicine". Moscow, Russia, 2004, p.70.
15. Akhapkina V.I., Fedin A.I., Avedisova A.S., Akhapkin R.V. Effectiveness of Phenotropil in the treatment of asthenic syndrome and chronic fatigue syndrome // Atmosphere. Nervous Diseases, Nº 3, Moscow, Russia, 2004, pp.28-31.
16. Akhapkina V.I., Voronina T.A. Spectrum of pharmacological effects of Phenotropil // Farmateka, Moscow, Russia, Nº 13, 2005, p. 19-25.
17. Akhapkina V.I. Adaptogenic effect of nootropics // Russian Medical Journal, N° 3, 2005, p.40-43.
18. Akhapkina V.I. Identification and evaluation of neuromodulator activity of Phenotropil // Proceedings of IX Russian Congress of Neurology, Yaroslavl, Russia, 2006, p.551.
19. Akhapkina V.I. Mechanisms for implementation of neuromodulator activity of Phenotropil // Proc. XIV Russian National Congress "Man and Medicine", Moscow, Russia, 2007, p.795.
20. Akhapkina V.I. On the neuromodulator activity of Phenotropil // Proc. XV Russian National Congress "Man and Medicine", Moscow, Russia, 2008, p.31-32.
21. Akhapkina V.I., Akhapkin R.V. Identification and evaluation of neuromodulator activity. Proc. XVII Russian National Congress "Man and medicine." Moscow, Russia, 2010, p.572-573.
22. Voronina T.A. and others, The specificity of action of piracetam, encephabol and cleregil on transcallosal evoked potential // Bul. Experimental Biology and Medicine. Moscow, Russia, 1986, v. 1011, Nº 3. pp. 320-322.
23. Voronina T.A. Experimental psychopharmacology of nootropics // In collection of papers Pharmacology of nootropics (experimental and clinical studies), Moscow, 1989, p.8-19.
24. Voronina T.A., Seredenin S.B. Nootropic drugs, achievements and new challenges // Experimental and Clinical Pharmacology, 1998, v. 61, Nº 4, p.3-9.
25. Ivanets N.N., Vinnikova M.A., Mokhnachev S.O. et al, Therapeutic efficacy and safety of Phenotropil in patients with alcohol dependence // Issues of Narcology. Moscow, Russia, 2008, Nº 4, p.16-32.
26. Kovalev G.I., Akhapkina V.I., Abaimov D.A., Firstova Y.Y. Phenotropil as a receptor modulator of synaptic neurotransmission // Atmosphere, Nervous Diseases, Nº 4. Moscow, Russia, 2007, p. 22-26.
27. Mashkovskii M.D., Pharmaceuticals, Part 2, 1988, p.168- 177.
28. Meletova O.K. Study of neurotrophic activity of pyrazolo[C]pyridine and related compounds. St. Petersburg, 2007, 144 p
29. Oxford Dictionary of Psychology, ed. A. Reber, 2002 // Neuromodulators.
30. Perekalin V.V., Novikov B.M, Zobacheva M.M. et al. Description of invention to the author's certificate SU N° 797219, A61 K21/40 "N-carbamoylmethyl-4-phenyl-2-pyrrolidone having antihypertensive activity" with priority date 08.05.1979, published on 25.07.1995.
31. Register of Pharmaceuticals of Russia (RPR), Encyclopedia of drugs, 1993-2010, including Anatomical Therapeutic Chemical Classification System.
32. Firstova Y.Y., Salimov R.M., Kovalev G.I. Effect of Phenotropil on neurochemical characteristics and behavior of mice with high and low effectiveness of exploratory behavior in a maze // Psychopharmacol. Biol. Narcology, Moscow, Russia, 2007, v.7, p.2-1982.
33. Firstova Y.Y. Study of ways of modulating synaptic plasticity in the neurochemical mechanism of action of neuroprotective drugs // Abstract and Thesis, Moscow, Russia, 2008.
34. Shipov A.G., Kramarova E.P., Negrebetsky V.V., Akhapkina V.I., Pogozhikh S.A., Baukov Y.A. // Methods of synthesis, molecular and crystal structure of Phenotropil // Bulletin of the Russian State Medical University, number 1 (48). Moscow, 2006, pp. 58-61, signed for printing 15.01.2006.
35. Shtulman D.R., Levin O.S. Neurology, Handbook of practical doctor // Moscow, MEDpress-Inform, 2005, 943 p. 419-423.
36. Eccles J.C., Fatt P., Koketsu K. Cholinergic and inhibitory synapses in a pathway from motor-axon collaterals to motoneurones. // J Physiol (Lond), 1954, Vol. 126, p. 524-562.
37. Schwartz J.H, Kandel E.R: Modulation of synaptic transmission: second-messenger systems // Essentials of Neural Science and Behavior. Edited by Kandel E.R, Schwartz J.H, Jessell T.M. East Norwalk, Conn, Appleton & Lange, 1995, p. 243-267.
38. Florey E. Neurotransmitters and modulators in the animal kingdom. // Federation. Proc., 1967, Vol. 26, p. 1164-1178.
39. Neurology 43, 301, 1993; Arch Gerontol Geriatr 16, 149, 1993; Stroke 1997; 28: 2347-52; CNS Drugs 1998; 9 Suppl 1: 41-9.
40. WHO Drug Information, Vol. 23, No. 2, 2009 // rac-2-[(4R)-2-oxo-4-phenylpirolidin-1-yl] acetamid - fonturacetamum, fonturacetam, fonturacetam.
41. WHO Drug Information Vol. 24, No. 1, 2010 // rac-2-[(4R)-2-oxo-4-phenylpirolidin-1-yl]acetamid -fonturacetamum, fonturacetam, fonturacetam.

## Claims

1. (RS)-2-(2-oxo-4-phenylpyrrolidin-1-yl)acetamide having modulatory activity with commensurate effect.

2. Compound as in claim 1, having psychomodulatory activity.

3. Compound as in claim 1, having psioperandmodulatory activity.

4. Compound as in claim 1, having neuromodulatory activity.

5. Compound as in claim 1, having operandmodulatory activity.

6. Compound as in claim 1, having incretomodulatory activity.

7. Compound as in claim 1, having immunomodulatory activity.

8. Compound as in claim 1, having cytomodulatory activity.

9. Compound as in claim 1, having promoutmodulatory activity.

10. Compound as in claim 1, having unimodulatory activity.

11. Compound as in claim 1, having training stress factor activity.

12. Compound as in claim 1, having adaptogenic activity.

13. Compound as in claim 1, having neuroleptic activity.

14. Compound as in claim 1, having anticonvulsant activity.

15. Compound as in claim 1, having antiparkinsonian activity.

16. Compound as in claim 1, having psychostimulant activity.

17. Compound as in claim 1, having anxiolytic activity.

18. Compound as in claim 1, having antidepressant activity.

19. Compound as in claim 1, having nootropic activity.

20. Compound as in claim 1, having mnemotropic activity.

21. Compound as in claim 1, having anti-craving activity.

22. Compound as in claim 1, having neuroprotective, neurotrophic and neurometabolic activity.

23. Compound as in claim 1, having metabotropic and antiapoptotic activity.

24. Compound as in claim 1, having analgesic activity.

25. Compound as in claim 1, having anti-ischemic and anti-infarction activity.

26. Compound as in claim 1, having cerebrovascular and cerebroprotective activity.

27. Compound as in claim 1, having antioxidant and prooxidant activity.

28. Compound as in claim 1, having antihypoxic activity.

29. Compound as in claim 1, having normotonic activity.

30. Compound as in claim 1, having anti-seasick activity.

31. Compound as in claim 1, having antiinflammatory activity.

32. Compound as in claim 1, having antitoxic activity.

33. Compound as in claim 1, having antiulcerogenic activity.

34. Compound as in claim 1, having anticarcinogenic activity.

35. Compound as in claim 1, having antiviral activity.

36. Compound as in claim 1, having antiedematous activity.

37. Compound as in claim 1, having diuretic activity.

38. Compound as in claim 1, having regenerative and reparative activity.

39. Compound as in claim 1, having rejuvenescent activity.

40. Compound as in claim 1, having slendering activity.

41. Pharmaceutical substance of the racemic compound
(RS)-2-(2-oxo-4-phenylpyrrolidin-1-yl)acetamide, containing:
2-((2-oxo-4-phenylpyrrolidin-1-yl)acetamide - no less than 99.0% and no more than 100.5% on dry basis, individual related impurities individually or in total - no more than 0.2%; residual amounts of organic solvents individually or in total - no more than 3000 ppm.

42. Pharmaceutical substance as in claim 41, differing in that the loss on drying is no more than 0.1%.

43. Pharmaceutical substance as in claim 41, differing in that the loss on drying may be no more than 0.5%.

44. Pharmaceutical substance as in claim 41, differing in that the melting temperature measured by the capillary method is in the range from 130 to 133°C.

45. Pharmaceutical substance of the racemic compound
(RS)-2-(2-oxo-4-phenylpyrrolidin-1-yl)acetamide, differing in that the content of individual related impurities individually or in total may not exceed 0.25%.

46. Pharmaceutical substance as in claim 45, differing in that the content of residual amounts of organic solvents individually or in total may not exceed 5000 ppm.

47. Pharmaceutical substance (RS)-2-(2-oxo-4-phenylpyrrolidin-1-yl)acetamide, differing in that the assay content of its constituent components may be:
2-((2-oxo-4-phenylpyrrolidin-1-yl)acetamide - not less than 98.0% and not more than 100.5% on dry basis; the individual related impurities in total - not less than 0.5%; residual amounts of organic solvents individually or in total - not less than 5000 ppm.

48. Pharmaceutical substance as in claim 47, differing in that the melting temperature measured by the capillary method is in the range from 128 to 133°C.

49. Method for producing the pharmaceutical substance (RS)-2-(2-oxo-4-phenylpyrrolidin-1-yl)acetamide, involving the synthesis of the desired technical raw materials from the racemic mixture of 4(RS)-phenylpyrrolidin-2-one, differing in that the desired technical raw materials obtained from the synthesis are subjected to purification, crystallization and stabilization of the compound by processing it with demineralized (distilled) water, and the isothermal crystallization from propanol followed by drying to a constant weight.

50. Use of the pharmaceutical substance (RS)-2-(2-oxo-4-phenylpyrrolidin-1-yl)acetamide in the manufacture of any expedient pharmaceutical or para-pharmaceutical composition that has an activity acceptable for its intended purpose as in any one of claims 1 to 40, differing in that before the start of standard industrial production of the composition, the substance powder is sieved through a sieve and dried to a constant weight, **characterized by** the following indicators: 2-(2-oxo-4-phenylpyrrolidin-1-yl)acetamide - not less than 99.0% and not more than 100.5% on dry basis; individual related impurities (precursors and products of synthesis) individually or in total - not more than 0.2%; residual amounts of organic solvents individually or in total - not more than 0.3% (not more than 3000 ppm); sulphate ash - not more than 0.1%, heavy metals - not more than 0.001%, loss on drying - not more than 0.1%, the melting point 130 to 133°C.

51. Titrimetric assay method of 2-(2-oxo-4-phenylpyrrolidin-1-yl)acetamide which consists in following:
0.2g (accurately weighed) of substance of 2-(2-oxo-4-phenylpyrrolidin-1-yl)acetamide was placed in a Kjeldahl flask; 20 ml of 0.1 M hydrochloric acid solution was added, the Kjeldahl flask was attached to the apparatus for determination of nitrogen and the distillation was started; after having obtained the steady distillation, 40 ml of 30 % sodium hydroxide was slowly added to the flask (making sure that the solution in the flask was stirred by the steam flow), 200 mL of distillate were collected in the receptacle with 20 ml of 4 % boric acid solution and 0.1 ml of mixed indicator solution; the distilled fraction was titrated with 0.1M hydrochloric acid to achieve the red-violet color, and the control experiment was carried out (1 ml of 0.1 M solution of HCl corresponds to 0.021826 g or 21.826/21.83 mg of C₁₂H₁₄N₂O₂).

52. Pharmaceutical composition for internal use having an activity acceptable for its intended purpose as in claims 1 to 40, containing per 100% of mass, mass-volume, volume:
| | |
|---|---|
| (RS)-2-(2-oxo-4-phenylpyrrolidin-1-yl)acetamide | 0.01-75 |
| Intentional additives, including excipients and carriers | 99.99-25 |

53. Pharmaceutical composition for topical use having an activity acceptable for its intended purpose as in claims 1 to 40, containing per 100% of mass, mass-volume, volume:
| | |
|---|---|
| (RS)-2-(2-oxo-4-phenylpyrrolidin-1-yl)acetamide | 0.001-90 |
| Intentional additives, including excipients and carriers | 99.999-10 |

54. Parapharmaceutical composition for internal use having an activity acceptable for its intended purpose as in claims 1 to 40, containing per 100% of mass, mass-volume, volume:
| | |
|---|---|
| (RS)-2-(2-oxo-4-phenylpyrrolidin-1-yl)acetamide | 0.01-75 |
| Intentional additives, including excipients and carriers | 99.99-25 |

55. Parapharmaceutical composition for topical use having an activity acceptable for its intended purpose as in claims 1 to 40, containing per 100% of mass, mass-volume, volume:
| | |
|---|---|
| (RS)-2-(2-oxo-4-phenylpyrrolidin-1-yl)acetamide | 0.001-90 |
| Intentional additives, including excipients and carriers | 99.999-10 |

## Amended claims

### Amended claims under Art. 19.1 PCT

**1. A compound** (RS)-2-(2-oxo-4-phenylpyrrolidin-1-yl)acetamide having modulatory activity with commensurate effect.

**2.** Compound as in claim 1, having psychomodulatory activity.

**3.** Compound as in claim 1, having psioperandmodulatory activity.

**4.** Compound as in claim 1, having neuromodulatory activity.

**5.** Compound as in claim 1, having operandmodulatory activity.

**6.** Compound as in claim 1, having incretomodulatory activity.

**7.** Compound as in claim 1, having immunomodulatory activity.

**8.** Compound as in claim 1, having cytomodulatory activity.

**9.** Compound as in claim 1, having promoutmodulatory activity.

**10.** Compound as in claim 1, having unimodulatory activity.

**11.** Compound as in claim 1, having training stress factor activity.

**12.** Compound as in claim 1, having adaptogenic activity.

**13.** Compound as in claim 1, having neuroleptic activity.

**14.** Compound as in claim 1, having anticonvulsant activity.

**15.** Compound as in claim 1, having antiparkinsonian activity.

**16.** Compound as in claim 1, having psychostimulant activity.

**17.** Compound as in claim 1, having anxiolytic activity.

**18.** Compound as in claim 1, having antidepressant activity.

**19.** Compound as in claim 1, having nootropic activity.

**20.** Compound as in claim 1, having mnemotropic activity.

**21.** Compound as in claim 1, having anti-craving activity.

**22.** Compound as in claim 1, having neuroprotective, neurotrophic and neurometabolic activity.

**23.** Compound as in claim 1, having metabotropic and antiapoptotic activity.

**24.** Compound as in claim 1, having analgesic activity.

**25.** Compound as in claim 1, having anti-ischemic and anti-infarction activity.

**26.** Compound as in claim 1, having cerebrovascular and cerebroprotective activity.

**27.** Compound as in claim 1, having antioxidant and prooxidant activity.

**28.** Compound as in claim 1, having antihypoxic activity.

**29.** Compound as in claim 1, having normotonic activity.

**30.** Compound as in claim 1, having anti-seasick activity.

**31.** Compound as in claim 1, having antiinflammatory activity.

**32.** Compound as in claim 1, having antitoxic activity.

**33.** Compound as in claim 1, having antiulcerogenic activity.

**34.** Compound as in claim 1, having anticarcinogenic activity.

**35.** Compound as in claim 1, having antiviral activity.

**36.** Compound as in claim 1, having antiedematous activity.

**37.** Compound as in claim 1, having diuretic activity.

**38.** Compound as in claim 1, having regenerative and reparative activity.

**39.** Compound as in claim 1, having rejuvenescent activity.

**40.** Compound as in claim 1, having slendering activity.

**41.** Pharmaceutical substance of the racemic compound
(RS)-2-(2-oxo-4-phenylpyrrolidin-1-yl)acetamide, containing:
2-((2-oxo-4-phenylpyrrolidin-1-yl)acetamide - no less than 99.0% and no more than 100.5% on dry basis, individual related impurities individually or in total - no more than 0.2%; residual amounts of organic solvents individually or in total - no more than 3000 ppm.

**42.** Pharmaceutical substance as in claim 41, differing in that the loss on drying is no more than 0.1%.

**43.** Pharmaceutical substance as in claim 41, differing in that the loss on drying may be no more than 0.5%.

**44.** Pharmaceutical substance as in claim 41, differing in that the melting temperature measured by the capillary method is in the range from 130 to 133°C.

**45.** Pharmaceutical substance of the racemic compound
(RS)-2-(2-oxo-4-phenylpyrrolidin-1-yl)acetamide, differing in that the content of individual related impurities individually or in total may not exceed 0.25%.

**46.** Pharmaceutical substance as in claim 45, differing in that the content of residual amounts of organic solvents individually or in total may not exceed 5000 ppm.

**47.** Pharmaceutical substance (RS)-2-(2-oxo-4-phenylpyrrolidin-1-yl)acetamide, differing in that the assay content of its constituent components may be:
2-((2-oxo-4-phenylpyrrolidin-1-yl)acetamide - not less than 98.0% and not more than 100.5% on dry basis; the individual related impurities in total - not less than 0.5%; residual amounts of organic solvents individually or in total - not less than 5000 ppm.

**48.** Pharmaceutical substance as in claim 47, differing in that the melting temperature measured by the capillary method is in the range from 128 to 133°C.

**49.** Method for producing the pharmaceutical substance (RS)-2-(2-oxo-4-phenylpyrrolidin-1-yl)acetamide, involving the synthesis of the desired technical raw materials from the racemic mixture of 4(RS)-phenylpyrrolidin-2-one, differing in that the desired technical raw materials obtained from the synthesis are subjected to purification, crystallization and stabilization of the compound by processing it with demineralized (distilled) water, and the isothermal crystallization from propanol followed by drying to a constant weight.

**50.** Use of the pharmaceutical substance (RS)-2-(2-oxo-4-phenylpyrrolidin-1-yl)acetamide in the manufacture of any expedient pharmaceutical or para-pharmaceutical composition that has an activity acceptable for its intended purpose as in any one of claims 1 to 40, differing in that before the start of standard industrial production of the composition, the substance powder is sieved through a sieve and dried to a constant weight, **characterized by** the following indicators: 2-(2-oxo-4-phenylpyrrolidin-1-yl)acetamide - not less than 99.0% and not more than 100.5% on dry basis; individual related impurities (precursors and products of synthesis) individually or in total - not more than 0.2%; residual amounts of organic solvents individually or in total - not more than 0.3% (not more than 3000 ppm); sulphate ash - not more than 0.1%, heavy metals - not more than 0.001%, loss on drying - not more than 0.1%, the melting point 130 to 133°C.

**51.** Titrimetric assay method of 2-(2-oxo-4-phenylpyrrolidin-1-yl)acetamide which consists in following:
0.2g (accurately weighed) of substance of 2-(2-oxo-4-phenylpyrrolidin-1-yl)acetamide was placed in a Kjeldahl flask; 20 ml of 0.1 M hydrochloric acid solution was added, the Kjeldahl flask was attached to the apparatus for determination of nitrogen and the distillation was started; after having obtained the steady distillation, 40 ml of 30 % sodium hydroxide was slowly added to the flask (making sure that the solution in the flask was stirred by the steam flow), 200 mL of distillate were collected in the receptacle with 20 ml of 4 % boric acid solution and 0.1 ml of mixed indicator solution; the distilled fraction was titrated with 0.1M hydrochloric acid to achieve the red-violet color, and the control experiment was carried out (1 ml of 0.1 M solution of HCl corresponds to 0.021826 g or 21.826/21.83 mg of C₁₂H₁₄N₂O₂).

**52.** Pharmaceutical composition for internal use having an activity acceptable for its intended purpose as in claims 1 to 40, containing per 100% of mass, mass-volume, volume:
| | |
|---|---|
| (RS)-2-(2-oxo-4-phenylpyrrolidin-1-yl)acetamide | 0.01-75 |
| Intentional additives, including excipients and carriers | 99.99-25 |

**53.** Pharmaceutical composition for topical use having an activity acceptable for its intended purpose as in claims 1 to 40, containing per 100% of mass, mass-volume, volume:
| | |
|---|---|
| (RS)-2-(2-oxo-4-phenylpyrrolidin-1-yl)acetamide | 0.001-90 |
| Intentional additives, including excipients and carriers | 99.999-10 |

**54.** Parapharmaceutical composition for internal use having an activity acceptable for its intended purpose as in claims 1 to 40, containing per 100% of mass, mass-volume, volume:
| | |
|---|---|
| (RS)-2-(2-oxo-4-phenylpyrrolidin-1-yl)acetamide | 0.01-75 |
| Intentional additives, including excipients and carriers | 99.99-25 |

**55.** Parapharmaceutical composition for topical use having an activity acceptable for its intended purpose as in claims 1 to 40, containing per 100% of mass, mass-volume, volume:
| | |
|---|---|
| (RS)-2-(2-oxo-4-phenylpyrrolidin-1-yl)acetamide | 0.001-90 |
| Intentional additives, including excipients and carriers | 99.999-10 |

Statement under Art. 19.1 PCT
In response to the written communication of the International Search Authority of 07.02.2013 the Applicant submits the amended claims and informs of the following.

The amended claims are based on the initial claims enclosed to the application materials filed with WIPO.

Claim 1 has been amended, claims 2-55 have been left unchanged.

Grounds for the amendments: the corrections have been made to line 1 of Claim 1 - the feature "compound" has been added. Claim 1 now describes "A compound (RS)-2-(2-oxo-4-phenylpyrrolidin-1-yl)acetamide having modulatory activity with commensurate effect." The amendment has been made to provide for correspondence of Claims 1-40 to the criterion of novelty. Since the Article of Shipov A.G. et al. "Metody sinteza, molekuliarnaia in kristallicheskaia struktura fenotropila", Vestnik RGMU, Zhurnal Rossiiskogo gosudarstvennogo meditsinskogo Universiteta, M., 2006, No. 1(48), p. 56-61 (D1) discloses the method of producing and structure of 2-(2-oxo-4-phenylpyrrolidin-1-yl)acetamide it does not anticipate novelty of the amended Claim 1 disclosing a compound (RS)-2-(2-oxo-4-phenylpyrrolidin-1-yl)acetamide. This amendment is based on the initial Claims and description since all claims dependent on Claim 1 (2-40) disclosed "Compound as in claim 1".

In response to the written communication of the International Search Authority of 07.02.2013 the Applicant submits the amended claims and informs of the following.

**Amendments to the Claims**

The amended claims are based on the initial Claims enclosed to the application materials filed with WIPO.

Claim 1 has been amended, claims 2-55 have been left unchanged.

Grounds for the amendments: the corrections have been made to line 1 of Claim 1 - the feature "compound" has been added. Claim 1 now describes **"A compound** (RS)-2-(2-oxo-4-phenylpyrrolidin-1-yl)acetamide having modulatory activity with commensurate effect." The amendment has been made to provide for correspondence of Claims 1-40 to the criterion of novelty. Since the Article of Shipov A.G. et al. "Metody sinteza, molekuliarnaia in kristallicheskaia struktura fenotropila", Vestnik RGMU, Zhurnal Rossiiskogo gosudarstvennogo meditsinskogo Universiteta, M., 2006, No. 1(48), p. 56-61 (D1) discloses the *method of producing and structure* of 2-(2-oxo-4-phenylpyrrolidin-1-yl)acetamide it does not anticipate novelty of the amended Claim 1 disclosing a **compound** (RS)-2-(2-oxo-4-phenylpyrrolidin-1-yl)acetamide. This amendment is based on the initial Claims and description since all claims dependent on Claim 1 (2-40) disclosed "Compound as in claim 1".
